# EUROPÄISCHE PATENTSCHRIFT

(11) **EP 3 180 411 B1**
(45) Veröffentlichungstag und Bekanntmachung des Hinweises auf die Patenterteilung: **29.08.2018**
(21) Anmeldenummer: 15737989.2
(22) Anmeldetag: 15.07.2015
(51) Int. Cl.: C09K 11/06, C07D 333/00, C07D 307/00, H01L 51/00, C07D 209/88, C07D 405/14, C07D 487/14, C07D 405/04, C07D 409/04, C07D 409/14, C07D 471/14, C07D 487/04, C07D 491/048, C09K 11/02, H01L 51/50, C07D 495/04, C07F 5/04

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 13.08.2014 EP 14002819
(43) Veröffentlichungstag der Anmeldung: 21.06.2017
(73) Patentinhaber: Merck Patent GmbH, 64293 Darmstadt (DE)
(72) Erfinder: PARHAM, Amir Hossain, 60486 Frankfurt am Main (DE); EBERLE, Thomas, 76829 Landau (DE); JATSCH, Anja, 60489 Frankfurt am Main (DE); GROSSMANN, Tobias, 64297 Darmstadt (DE); KROEBER, Jonas Valentin, 60311 Frankfurt am Main (DE); ANEMIAN, Rémi Manouk, Seoul 657-169 (KR)
(86) Internationale Anmeldenummer: PCT/EP2015/001456
(87) Internationale Veröffentlichungsnummer: WO 2016/023608

(56) Entgegenhaltungen:
- EP-A1- 2 757 608
- WO-A1-2008/032171
- WO-A1-2014/088284
- WO-A1-2014/088285
- DE-A1-102009 053 382
- KR-A- 20140 015 202
- KR-A- 20140 046 541
- US-A1- 2013 256 645
- US-A1- 2014 001 456
- MORELLATO LAURENCE ET AL: "Synthesis of novel 9-aryl and heteroarylpurine derivatives via copper mediated coupling reaction", TETRAHEDRON LETTERS, Bd. 55, Nr. 9, 28. Januar 2014 (2014-01-28), Seiten 1625-1627, XP028615089, ISSN: 0040-4039, DOI: 10.1016/J.TETLET.2014.01.091

## Beschreibung

Die vorliegende Erfindung betrifft elektronenarme Heteroaromaten, welche mit Dibenzofuran- bzw. Dibenzothiophenderivaten und mit Carbazolen bzw. Aminen substituiert sind, insbesondere für die Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Carbazolderivate (z. B. gemäß WO 2014/015931), Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Aus der WO 2011/046182 sind Carbazol-Arylen-Triazin-Derivate bekannt, welche am Triazin mit einer Fluorenylgruppe substituiert sind. Aus der WO 2013/077352 sind Triazinderivate bekannt, worin die Triazingruppe über eine divalente Arylengruppe an eine Dibenzofurangruppe gebunden ist. Diese Verbindungen sind als Lochblockiermaterialien beschrieben. Eine Verwendung dieser Materialien als Host für phosphoreszierende Emitter ist nicht offenbart. Aus der KR 2014-0046541 sind Carbazol-Triazin-Dibenzofuran- bzw. Carbazol-Triazin-Dibenzothiophen-Verbindungen bekannt, wobei das Dibenzofuran bzw. Dibenzothiophen über dessen 4-Position an das Triazin gebunden ist.

Generell besteht bei Materialien für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen.

Überraschend wurde gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung gemäß der folgenden Formel (1), 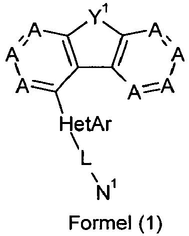 wobei für die verwendeten Symbole gilt:
- A: ist bei jedem Auftreten gleich oder verschieden CR¹ oder N, wobei maximal zwei Gruppen A pro Cyclus für N stehen;
- Y¹: ist O oder S;
- L: ist bei jedem Auftreten gleich oder verschieden eine Einfachbindung oder ein aromatisches oder heteroaromatisches Ringsystem mit 5 bis 30 aromatischen Ringatomen, das durch einen oder mehrere Reste R¹ substituiert sein kann;
- HetAr: ist eine Gruppe der folgenden Formel (2) oder (3), 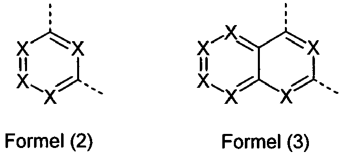 wobei die gestrichelten Bindungen die Verknüpfung dieser Gruppe mit dem Dibenzofuran- bzw. Dibenzothiophenderivat und mit L darstellen;
- X: ist bei jedem Auftreten gleich oder verschieden CR² oder N mit der Maßgabe, dass mindestens ein Symbol X für N steht;
- N¹: ist eine Gruppe der folgenden Formel (4), (5) oder (6), 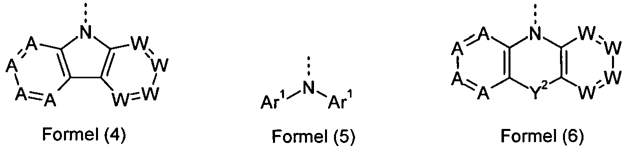 wobei die gestrichelte Bindung die Verknüpfung dieser Gruppe mit L darstellt und A die oben genannten Bedeutungen aufweist;
- Ar¹: ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann;
- W: ist bei jedem Auftreten gleich oder verschieden CR¹ oder N, wobei maximal zwei Gruppen W für N stehen, oder zwei benachbarte Gruppen W stehen zusammen für eine Gruppe der folgenden Formel (7) oder (8), wobei die Gruppe der Formel (4) bzw. Formel (6) maximal eine Gruppe der Formel (7) oder (8) aufweist, und die verbleibenden Gruppen W stehen gleich oder verschieden bei jedem Auftreten für CR¹ oder N, 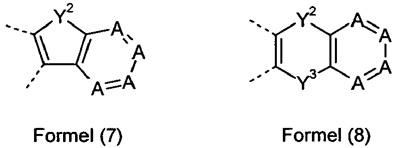 wobei die gestrichelten Bindungen die Verknüpfung dieser Gruppe andeuten und A die oben genannten Bedeutungen aufweist;
- Y², Y³: ist gleich oder verschieden bei jedem Auftreten O, NR⁴, S, C(R⁴)₂, Si(R⁴)₂, BR⁴ oder C=O, wobei der Rest R⁴, der an N gebunden ist, ungleich H ist;
- R¹, R², R³, R⁴: ist bei jedem Auftreten gleich oder verschieden ausgewählt aus der Gruppe bestehend aus H, D, F, Cl, Br, I, CN, NO₂, N(Ar²)₂, N(R⁵)₂, C(=O)Ar², C(=O)R⁵, P(=O)(Ar²)_{2,} P(Ar²)₂, B(Ar²)₂, Si(Ar²)₃, Si(R⁵)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, Si(R⁵)₂, C=O, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei benachbarte Substituenten R¹ bzw. zwei benachbarte Substituenten R³ bzw. zwei benachbarte Substituenten R⁴ miteinander ein aliphatisches, aromatisches oder heteroaromatisches Ringsystem bilden, das mit einem oder mehreren Resten R⁵ substituiert sein kann;
- Ar²: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R⁵ substituiert sein kann; dabei können zwei Reste Ar², welche an dasselbe N-Atom, P-Atom oder B-Atom binden, auch durch eine Einfachbindung oder eine Brücke, ausgewählt aus N(R⁵), C(R⁵)₂, O oder S, miteinander verbrückt sein;
- R⁵: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein aliphatisches Ringsystem bilden;
mit der Maßgabe, dass Y¹ für O steht, wenn die Gruppe N¹ für eine Gruppe der Formel (4) steht, die keine Gruppe der Formel (7) oder (8) enthält.

Benachbarte Substituenten im Sinne der vorliegenden Erfindung sind Substituenten, die an Kohlenstoffatome gebunden sind, die direkt miteinander verknüpft sind, oder die an dasselbe Kohlenstoffatom gebunden sind.

Wenn L für eine Einfachbindung steht, sind die Gruppen HetAr und N¹ direkt aneinander gebunden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

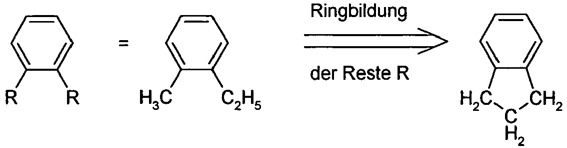

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Ein aromatisches Ringsystem im Sinne dieser Erfindung enthält 6 bis 40 C-Atome im Ringsystem. Unter einem aromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, also Oligoarylene bzw. Oligoheteroarylene, wie zum Beispiel Biphenyl, Terphenyl oder Quaterphenyl als aromatische Ringsysteme im Sinne dieser Anmeldung bezeichnet.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy oder 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 40 aromatischen Ringatomen werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer bevorzugten Ausführungsform der Erfindung steht in Formel (1) maximal eine Gruppe A pro Cyclus für N und die anderen Gruppen A stehen für CR¹. Besonders bevorzugt steht A für CR¹, so dass es sich bei der Verbindung der Formel (1) um eine Verbindung gemäß der folgenden Formel (1a) handelt, 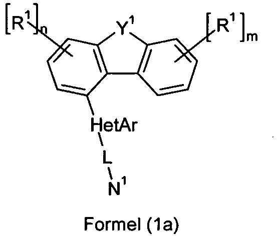 wobei die verwendeten Symbole die oben genannten Bedeutungen aufweisen, n für 0, 1, 2 oder 3 und m für 0, 1, 2, 3 oder 4 steht.

In einer bevorzugten Ausführungsform der Erfindung ist der Index n in Formel (1a) 0 oder 1 und der Index m in Formel (1a) 0, 1 oder 2. Besonders bevorzugt sind die Indizes n und m gleich oder verschieden bei jedem Auftreten 0 oder 1 und ganz besonders bevorzugt gleich 0.

Bevorzugte Ausführungsformen der Formel (1a) sind daher die Verbindungen der folgenden Formeln (1b) bis (1h), 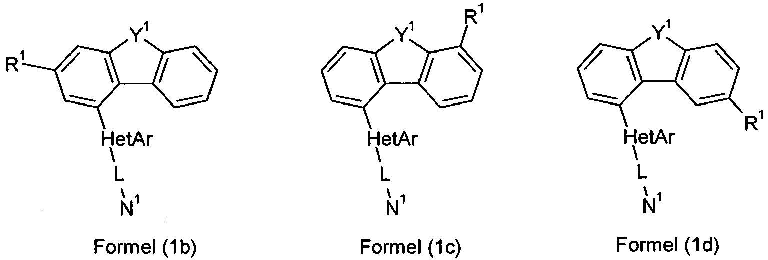 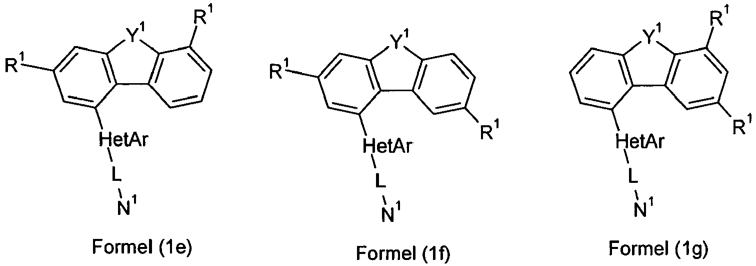 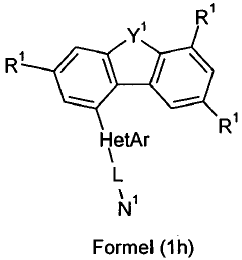 wobei die verwendeten Symbole die oben genannten Bedeutungen aufweisen.

Eine besonders bevorzugte Ausführungsform der Formel (1a) ist die Verbindung der folgenden Formel (1i), 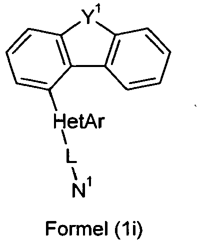 wobei die verwendeten Symbole die oben genannten Bedeutungen aufweisen.

Im Folgenden werden bevorzugte Ausführungsformen der Gruppe HetAr beschrieben.

Bevorzugte Ausführungsformen der Gruppen der Formeln (2) und (3) sind die Gruppen der folgenden Formeln (2-1) bis (2-7) und (3-1), 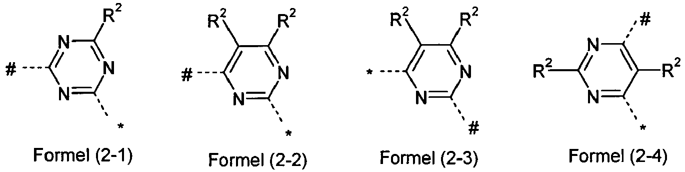 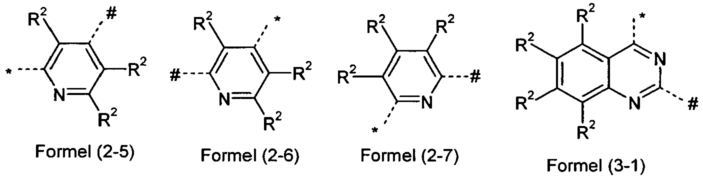 wobei die gestrichelte Bindung und * die Verknüpfung dieser Gruppen mit dem Dibenzofuran- bzw. Dibenzothiophenderivat in Formel (1) darstellt, die gestrichelte Bindung und # die Verknüpfung dieser Gruppen mit L bzw. für L gleich Einfachbindung mit N¹ darstellt und R² die oben genannten Bedeutungen aufweist.

Bevorzugt sind die Gruppen der Formeln (2-1) bis (2-4) und (3-a), und besonders bevorzugt ist die Gruppe der Formel (2-1).

Bevorzugte Ausführungsformen der oben genannten Gruppen sind die Gruppen der folgenden Formeln (2-1a) bis (3-1a), 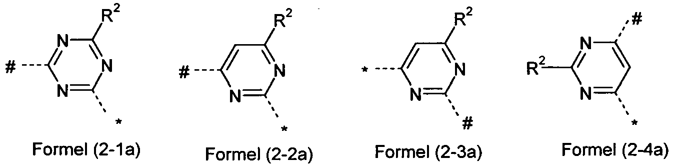 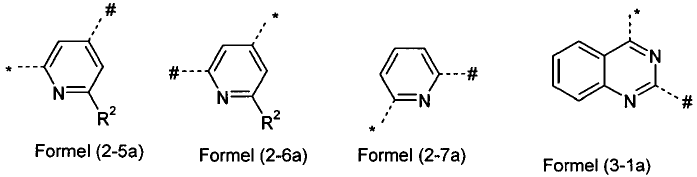 wobei die gestrichelte Bindung und * die Verknüpfung dieser Gruppen mit dem Dibenzofuran- bzw. Dibenzothiophenderivat in Formel (1) darstellt, die gestrichelte Bindung und # die Verknüpfung dieser Gruppen mit L bzw. für L gleich Einfachbindung mit N¹ darstellt und R² einen Substituenten gemäß der oben genannten Definition ungleich Wasserstoff darstellt.

Der Substituent R² an der Gruppe HetAr ist bevorzugt H oder ein aromatisches oder heteroaromatisches Ringsystem mit 6 bis 24 aromatischen Ringatomen, das durch einen oder mehrere Reste R⁵ substituiert sein kann. Dabei ist R² in den Gruppen der Formeln (2-1a) bis (2-6a) ungleich Wasserstoff. Das aromatische oder heteroaromatische Ringsystem hat bevorzugt 6 bis 18 aromatische Ringatome. Besonders bevorzugt handelt es sich um ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R⁵ substituiert sein kann, bevorzugt aber unsubstituiert ist. Beispiele für geeignete Gruppen R² sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R⁵ substituiert sein können, bevorzugt aber unsubstituiert sind.

Beispiele für geeignete Gruppen R² sind die im Folgenden aufgeführten Strukturen R²-1 bis R²-24, 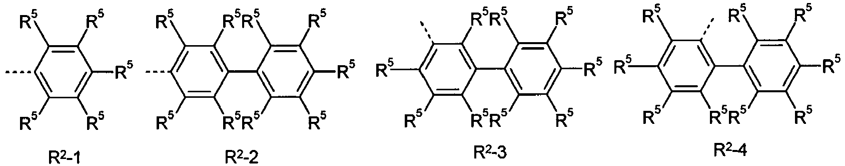 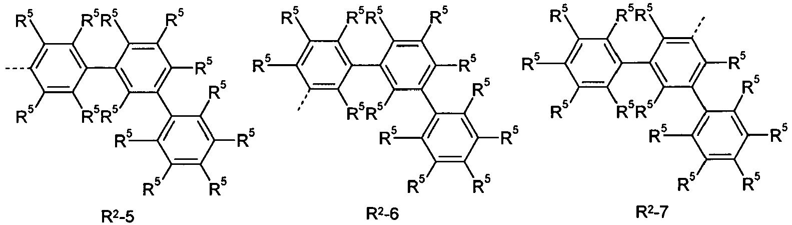 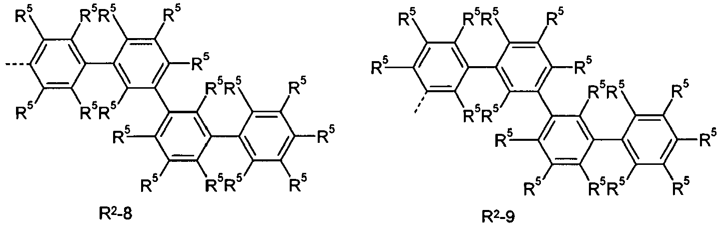 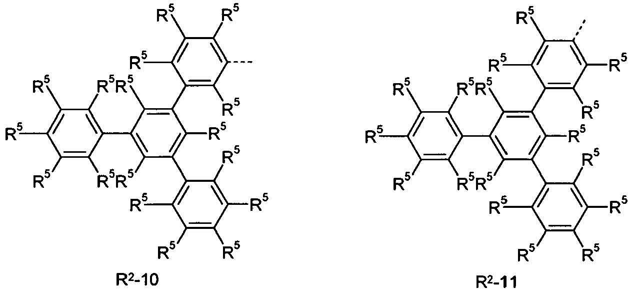 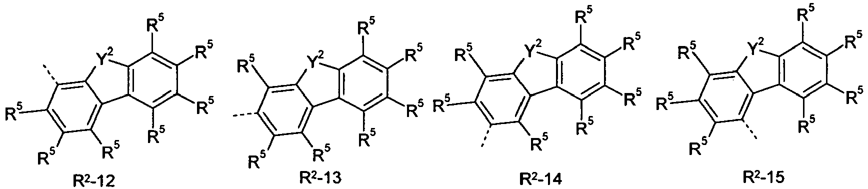 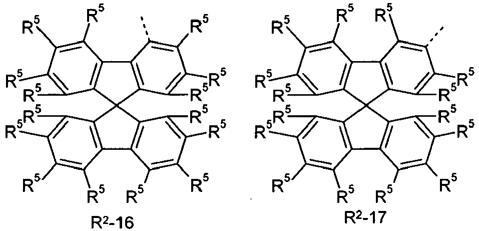 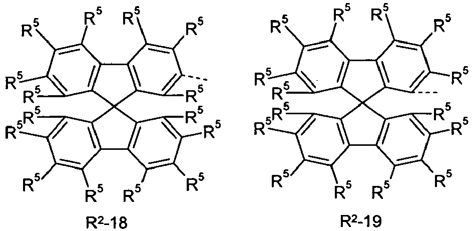 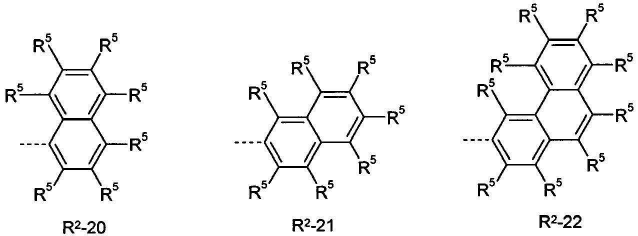 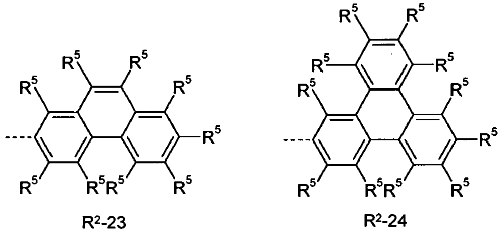 wobei Y² und R⁵ die oben genannten Bedeutungen aufweisen und die gestrichelte Bindung die Bindung an die Heteroarylgruppe darstellt.

Im Folgenden werden bevorzugte Ausführungsformen der Gruppe N¹ ausgeführt.

In den Gruppen der Formeln (4) und (6) ist es bevorzugt, wenn maximal eine Gruppe A für N steht und die anderen Gruppen A für CR¹ stehen. Besonders bevorzugt stehen alle Gruppen A in Formeln (4) und (6) für CR¹. Besonders bevorzugte Gruppen der Formel (4) sind somit die Gruppen der folgenden Formeln (4-1) und (4-2), und besonders bevorzugte Gruppen der Formel (6) sind die Gruppen der folgenden Formel (6-1), 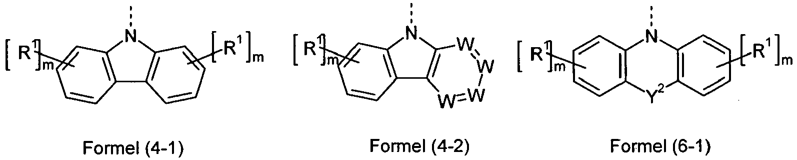 wobei R¹ und m die oben genannten Bedeutungen aufweisen und weiterhin gilt:
zwei benachbarte Gruppen W stehen zusammen für eine Gruppe der folgenden Formel (7a) oder (8a) und die anderen beiden Gruppen W stehen für CR¹ und bevorzugt für CH, 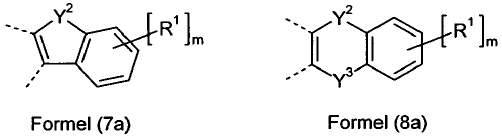 wobei Y², Y³, R¹ und m die oben genannten Bedeutungen aufweisen.

In einer bevorzugten Ausführungsform der Erfindung steht der Index m in den Formeln (4-1), (4-2) und (6-1) für 0, 1, 2 oder 3, besonders bevorzugt für 0, 1 oder 2 und ganz besonders bevorzugt für 0 oder 1.

Bevorzugte Ausführungsformen der Gruppe der Formel (4-1) sind die Gruppen der folgenden Formeln (4-1a) bis (4-1f), 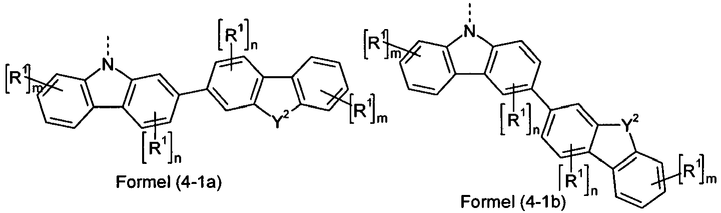 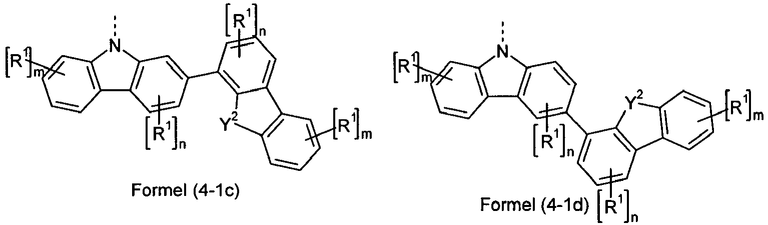 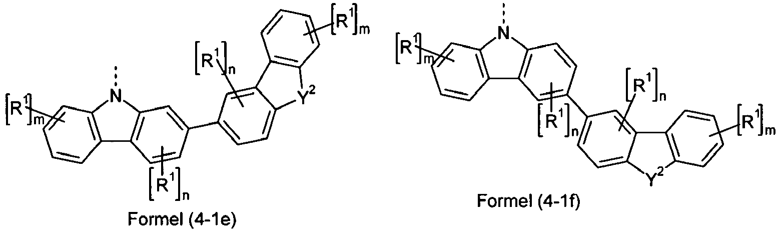 wobei Y² die oben genannten Bedeutungen aufweist und bevorzugt für NR⁴, O oder S steht und m und n die oben genannten Bedeutungen aufweisen.

Bevorzugte Ausführungsformen der Gruppe der Formel (4-2) sind die Gruppen der folgenden Formeln (4-2a) bis (4-2f), 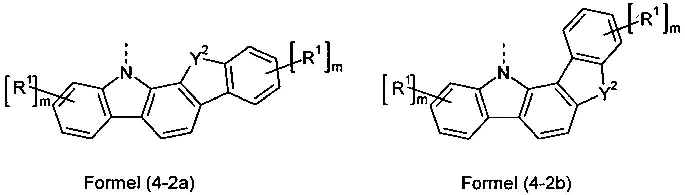 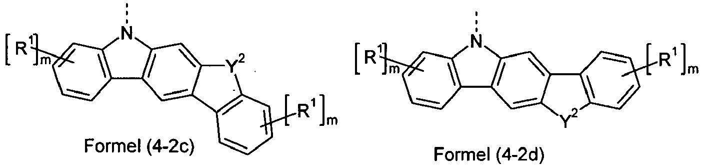 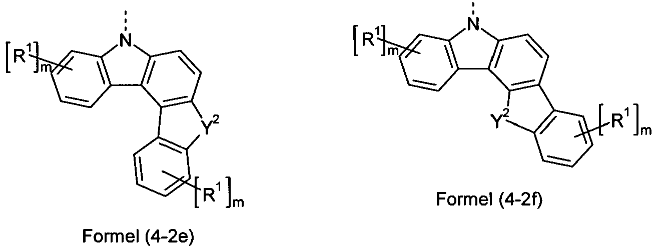 wobei die verwendeten Symbole und Indizes die oben genannten Bedeutungen aufweisen.

In einer weiteren bevorzugten Ausführungsform der Erfindung stehen Y² und Y³ gleich oder verschieden bei jedem Auftreten für O, C(R⁴)₂ oder NR⁴, wobei der an den Stickstoff gebundene Rest R⁴ ungleich H ist. In den Formeln (4-2a) bis (4-2f) steht Y² besonders bevorzugt für C(R⁴)₂ oder NR⁴, wobei der an den Stickstoff gebundene Rest R⁴ ungleich H ist, und ganz besonders bevorzugt für C(R⁴)₂.

Wenn Y² bzw. Y³ für NR⁴ steht, ist es bevorzugt, wenn dieser Rest R⁴ bei jedem Auftreten gleich oder verschieden für ein aromatisches oder heteroaromatisches Ringsystem mit 5 bis 24 aromatischen Ringatomen steht, das jeweils mit einem oder mehreren Resten R⁵ substituiert sein kann, besonders bevorzugt für ein aromatisches oder heteroaromatisches Ringsystem mit 6 bis 18 aromatischen Ringatomen, das mit einem oder mehreren Resten R⁵ substituiert sein kann. Beispiele für geeignete Substituenten R⁴ sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3- oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R⁵ ungleich H oder D substituiert ist. Dabei können diese Gruppen jeweils durch einen oder mehrere Reste R⁵ substituiert sein, sind bevorzugt aber unsubstituiert. Dabei sind geeignete Strukturen R⁴ die gleichen Strukturen, wie sie vorne für R²-1 bis R²-24 abgebildet sind.

Wenn Y² für C(R⁴)₂ steht, ist es bevorzugt, wenn diese Reste R⁴ bei jedem Auftreten gleich oder verschieden für eine geradkettige Alkylgruppe mit 1 bis 10 C-Atomen oder eine verzweigte oder cyclische Alkylgruppe mit 3 bis 10 C-Atomen oder eine Alkenylgruppe mit 2 bis 10 C-Atomen stehen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, oder für ein aromatisches oder heteroaromatisches Ringsystem mit 5 bis 24 aromatischen Ringatomen, das jeweils mit einem oder mehreren Resten R⁵ substituiert sein kann; dabei können optional die beiden Substituenten R⁴ ein monocyclisches oder polycyclisches, aliphatisches, aromatisches oder heteroaromatisches Ringsystem bilden, das mit einem oder mehreren Resten R⁵ substituiert sein kann. Durch Ringbildung der beiden Substituenten R⁴ wird ein Spirosystem aufgespannt, beispielsweise ein Spirobifluoren bzw. Derivat eines Spirobifluorens, wenn die Gruppen R⁴ für Phenylgruppen stehen.

In einer bevorzugten Ausführungsform der Erfindung steht in der Gruppe der Formel (5) die Gruppe Ar¹ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R³ substituiert sein kann, bevorzugt aber unsubstituiert ist. Beispiele für geeignete Gruppen Ar¹ sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R³ substituiert sein können, bevorzugt aber unsubstituiert sind.

Besonders bevorzugte Gruppen Ar¹ sind die Gruppen der folgenden Formeln (Ar¹-1) bis (Ar¹-21), 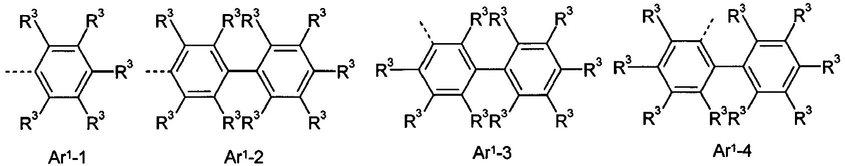 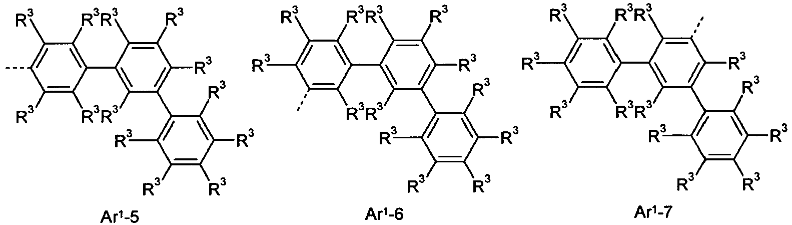 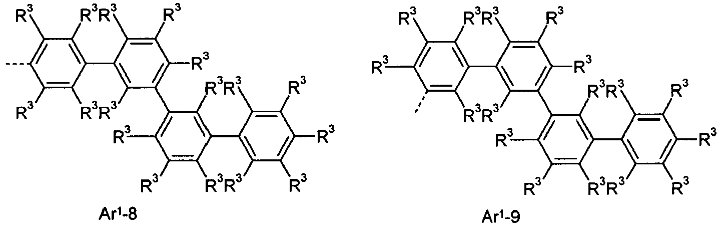 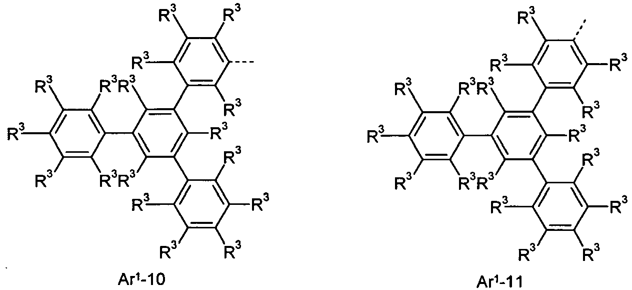 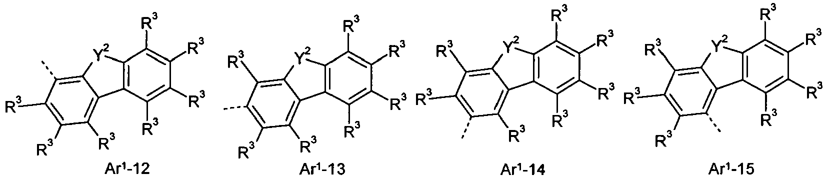 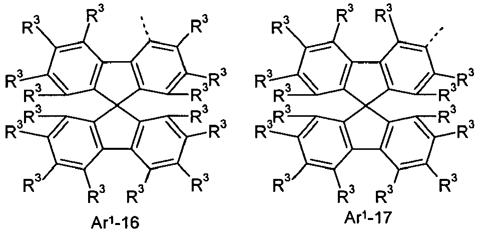 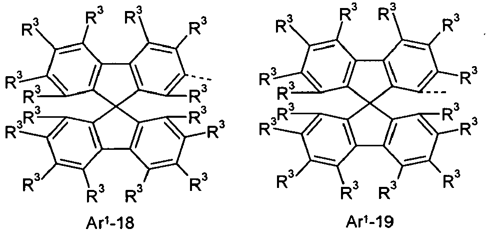 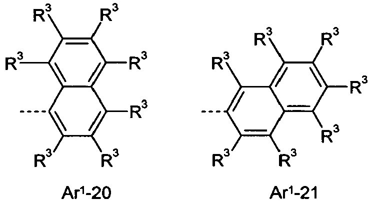 wobei Y² und R³ die oben genannten Bedeutungen aufweisen und die gestrichelte Bindung die Bindung an den Stickstoff in Formel (5) darstellt.

In einer bevorzugten Ausführungsform der Erfindung steht R³ gleich oder verschieden bei jedem Auftreten für H, eine Alkylgruppe mit 1 bis 4 C-Atomen oder ein aromatisches oder heteroaromatisches Ringsystem mit 5 bis 14 aromatischen Ringatomen. Besonders bevorzugt steht R³ gleich oder verschieden bei jedem Auftreten für H oder eine Alkylgruppe mit 1 bis 4 C-Atomen.

In einer weiteren bevorzugten Ausführungsform der Erfindung steht L gleich oder verschieden bei jedem Auftreten für eine Einfachbindung oder ein aromatisches oder heteroaromatisches Ringsystem mit 5 bis 24 aromatischen Ringatomen, das durch einen oder mehrere Reste R¹ substituiert sein kann. Besonders bevorzugt steht L gleich oder verschieden bei jedem Auftreten für eine Einfachbindung oder ein aromatisches Ringsystem mit 6 bis 12 aromatischen Ringatomen oder ein heteroaromatisches Ringsystem mit 6 bis 13 aromatischen Ringatomen, das jeweils durch einen oder mehrere Reste R¹ substituiert sein kann, bevorzugt aber unsubstituiert ist. Ganz besonders bevorzugt steht L für eine Einfachbindung, wenn N¹ eine Gruppe der Formel (4) oder (6) ist, und steht für ein aromatisches Ringsystem mit 6 bis 12 aromatischen Ringatomen oder ein heteroaromatisches Ringsystem mit 6 bis 13 aromatischen Ringatomen, das jeweils durch einen oder mehrere Reste R¹ substituiert sein kann, bevorzugt aber unsubstituiert ist, wenn N¹ eine Gruppe der Formel (5) ist. Beispiele für geeignete aromatische oder heteroaromatische Ringsysteme L sind ausgewählt aus der Gruppe bestehend aus Phenylen, Biphenyl, Fluoren, Pyridin, Pyrimidin, Triazin, Dibenzofuran, Dibenzothiophen und Carbazol, die jeweils durch einen oder mehrere Reste R¹ substituiert sein können, bevorzugt aber unsubstituiert sind.

Wenn die erfindungsgemäßen Verbindungen Substituenten R¹ aufweisen, dann sind diese bevorzugt gewählt aus der Gruppe bestehend aus H, D, F, CN, N(Ar²)₂, C(=O)Ar², P(=O)(Ar²)_{2,} einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁵ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁵ substituiert sein kann.

Besonders bevorzugt sind die Substituenten R¹ ausgewählt aus der Gruppe bestehend aus H, D, F, CN, N(Ar²)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R⁵ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R⁵ substituiert sein kann, bevorzugt aber unsubstituiert ist; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁵ substituiert sein kann, bevorzugt aber unsubstituiert ist.

Ganz besonders bevorzugt sind die Substituenten R¹ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R⁵ substituiert sein kann, bevorzugt aber unsubstituiert ist. Beispiele für geeignete Substituenten R¹ sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R⁵ substituiert sein können, bevorzugt aber unsubstituiert sind. Dabei sind geeignete Strukturen R¹ die gleichen Strukturen, wie sie vorne für R²-1 bis R²-24 abgebildet sind.

In einer weiteren bevorzugten Ausführungsform der Erfindung ist R⁵ bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Die oben genannten Bevorzugungen können einzeln oder gemeinsam auftreten. Es ist bevorzugt, wenn die oben genannten Bevorzugungen gemeinsam auftreten.

Bevorzugt sind somit Verbindungen gemäß der oben genannten Formel (1a), für die gilt:
- Y¹: ist O oder S;
- L: ist bei jedem Auftreten gleich oder verschieden eine Einfachbindung oder ein aromatisches oder heteroaromatisches Ringsystem mit 5 bis 24 aromatischen Ringatomen, das durch einen oder mehrere Reste R¹ substituiert sein kann;
- HetAr: ist eine Gruppe gemäß einer der oben aufgeführten Formeln (2-1) bis (2-7) oder (3-1);
- N¹: ist eine Gruppe der folgenden Formel (4-1), (4-2), (5) oder (6-1), 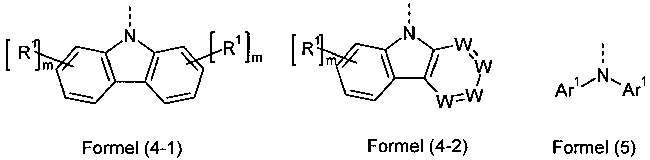 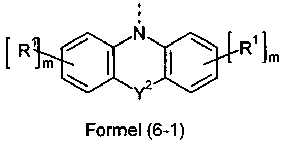 zwei benachbarte Gruppen W stehen zusammen für eine Gruppe der folgenden Formel (7a) oder (8a) und die anderen beiden Gruppen W stehen für CR¹ und bevorzugt für CH, 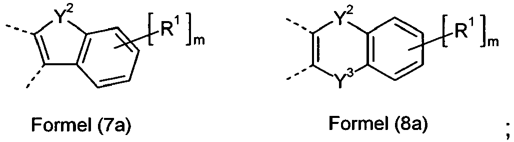
- Ar¹: ist bei jedem Auftreten gleich oder verschieden ein aromatisches oder heteroaromatisches Ringsystem mit 6 bis 24 aromatischen Ringatomen, bevorzugt mit 6 bis 18 aromatischen Ringatomen, welches durch einen oder mehrere Reste R³ substituiert sein kann;
- Y², Y³: ist gleich oder verschieden bei jedem Auftreten O, S, NR⁴ oder C(R⁴)₂, wobei der Rest R⁴, der an N gebunden ist, ungleich H ist;
- R¹: ist bei jedem Auftreten gleich oder verschieden gewählt aus der Gruppe bestehend aus H, D, F, CN, N(Ar²)₂, C(=O)Ar², P(=O)(Ar²)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ring-atomen, das jeweils mit einem oder mehreren Resten R⁵ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁵ substituiert sein kann;
- R²: ist bei jedem Auftreten gleich oder verschieden H oder ein aromatisches oder heteroaromatisches Ringsystem mit 6 bis 24 aromatischen Ringatomen, insbesondere mit 6 bis 18 aromatischen Ring-atomen, das jeweils durch einen oder mehrere Reste R⁵ substituiert sein kann;
- R³: ist bei jedem Auftreten gleich oder verschieden H, eine Alkylgruppe mit 1 bis 4 C-Atomen oder ein aromatisches oder heteroaromatisches Ringsystem mit 5 bis 14 aromatischen Ringatomen;
- R⁴: ist für Y² bzw. Y³ = NR⁴ ein aromatisches oder heteroaromatisches Ringsystem mit 5 bis 24 aromatischen Ringatomen, bevorzugt mit 6 bis 18 aromatischen Ringatomen, das jeweils mit einem oder mehreren Resten R⁵ substituiert sein kann; und ist für Y² bzw. Y³ = C(R⁴)₂ bei jedem Auftreten gleich oder verschieden eine geradkettige Alkylgruppe mit 1 bis 10 C-Atomen oder eine verzweigte oder cyclische Alkylgruppe mit 3 bis 10 C-Atomen oder eine Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, oder ein aromatisches oder heteroaromatisches Ringsystem mit 5 bis 24 aromatischen Ringatomen, das jeweils mit einem oder mehreren Resten R⁵ substituiert sein kann; dabei können optional die beiden Substituenten R⁴ ein monocyclisches oder polycyclisches, aliphatisches, aromatisches oder heteroaromatisches Ringsystem bilden, das mit einem oder mehreren Resten R⁵ substituiert sein kann;
- Ar²: hat die oben aufgeführten Bedeutungen;
- R⁵: ist bei jedem Auftreten gleich oder verschieden ausgewählt aus der Gruppe bestehend aus H, D, F, CN, einem aliphatischen Kohlenwasserstoffrest mit 1 bis 10 C-Atomen oder einem aromatischen oder heteroaromatischen Ringsystem mit 5 bis 30 aromatischen Ringatomen, das durch ein oder mehrere Alkylgruppen mit jeweils 1 bis 4 Kohlenstoffatomen substituiert sein kann;
- n: ist bei jedem Auftreten gleich oder verschieden 0, 1, 2 oder 3, bevorzugt 0, 1 oder 2;
- m: ist gleich oder verschieden bei jedem Auftreten 0, 1, 2, 3 oder 4, bevorzugt 0, 1, 2 oder 3.

Besonders bevorzugt sind die Verbindungen der oben genannten Formeln (1b) bis (1i), für die gilt:
- Y¹: ist O oder S;
- L: ist bei jedem Auftreten gleich oder verschieden eine Einfachbindung oder ein aromatisches Ringsystem mit 6 bis 12 aromatischen Ring-atomen oder ein heteroaromatisches Ringsystem mit 6 bis 13 aromatischen Ringatomen, das jeweils durch einen oder mehrere Reste R¹ substituiert sein kann, bevorzugt aber unsubstituiert ist;
- HetAr: ist eine Gruppe gemäß einer der oben aufgeführten Formeln (2-1a) bis (2-7a) oder (3-1a);
- N¹: ist eine Gruppe der oben aufgeführten Formeln (4-1), (4-2a) bis (4-2f), (5) oder (6-1);
- Ar¹: ist bei jedem Auftreten gleich oder verschieden ausgewählt aus der Gruppe bestehend aus Phenyl, Biphenyl, Terphenyl, Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R substituiert sein können, bevorzugt aber unsubstituiert sind, und ist insbesondere ausgewählt aus den oben aufgeführten Gruppen der Formeln (Ar¹-1) bis (Ar¹-21);
- Y², Y³: ist gleich oder verschieden bei jedem Auftreten O, NR⁴ oder C(R⁴)₂, wobei der Rest R⁴, der an N gebunden ist, ungleich H ist;
- R¹: ist bei jedem Auftreten gleich oder verschieden ausgewählt aus der Gruppe bestehend aus H, D, F, CN, N(Ar²)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R⁵ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ring-atomen, besonders bevorzugt mit 6 bis 13 aromatischen Ring-atomen, das jeweils mit einem oder mehreren nicht-aromatischen Resten R⁵ substituiert sein kann, bevorzugt aber unsubstituiert ist; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁵ substituiert sein kann, bevorzugt aber unsubstituiert ist; insbesondere bevorzugt ist R¹ gleich oder verschieden bei jedem Auftreten ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nichtaromatischen Resten R⁵ substituiert sein kann, bevorzugt aber unsubstituiert ist;
- R²: ist gleich oder verschieden bei jedem Auftreten ausgewählt aus der Gruppe bestehend aus Phenyl, Biphenyl, Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R⁵ substituiert sein können, und ist insbesondere ausgewählt aus den Gruppen der oben aufgeführten Strukturen R²-1 bis R²-24;
- R³: ist H oder eine Alkylgruppe mit 1 bis 4 C-Atomen;
- R⁴: ist für Y² bzw. Y³ = NR⁴ ausgewählt aus der Gruppe bestehend aus Phenyl, Biphenyl, Terphenyl, Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3- oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R⁵ ungleich H oder D substituiert ist, wobei diese Gruppen jeweils durch einen oder mehrere Reste R⁵ substituiert sein können; besonders bevorzugt sind die vorn abgebildeten Strukturen R²-1 bis R²-24; und ist für Y² bzw. Y³ = C(R⁴)₂ bei jedem Auftreten gleich oder verschieden eine geradkettige Alkylgruppe mit 1 bis 5 C-Atomen oder eine verzweigte oder cyclische Alkylgruppe mit 3 bis 8 C-Atomen oder eine Alkenylgruppe mit 2 bis 8 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, oder ein aromatisches oder heteroaromatisches Ringsystem mit 6 bis 18 aromatischen Ringatomen, das jeweils mit einem oder mehreren Resten R⁵ substituiert sein kann; dabei können optional die beiden Substituenten R⁴ ein monocyclisches oder polycyclisches, aliphatisches, aromatisches oder heteroaromatisches Ringsystem bilden, das mit einem oder mehreren Resten R⁵ substituiert sein kann;
- Ar²: hat die oben aufgeführten Bedeutungen;
- R⁵: ist bei jedem Auftreten gleich oder verschieden ausgewählt aus der Gruppe bestehend aus H, D, F, CN, einem aliphatischen Kohlenwasserstoffrest mit 1, 2, 3 oder 4 C-Atomen oder einem aromatischen oder heteroaromatischen Ringsystem mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist;
- n: ist bei jedem Auftreten gleich oder verschieden 0 oder 1;
- m: ist gleich oder verschieden bei jedem Auftreten 0, 1 oder 2, bevorzugt 0 oder 1.

Beispiele für geeignete erfindungsgemäße Verbindungen sind die nachstehend gezeigten Strukturen.

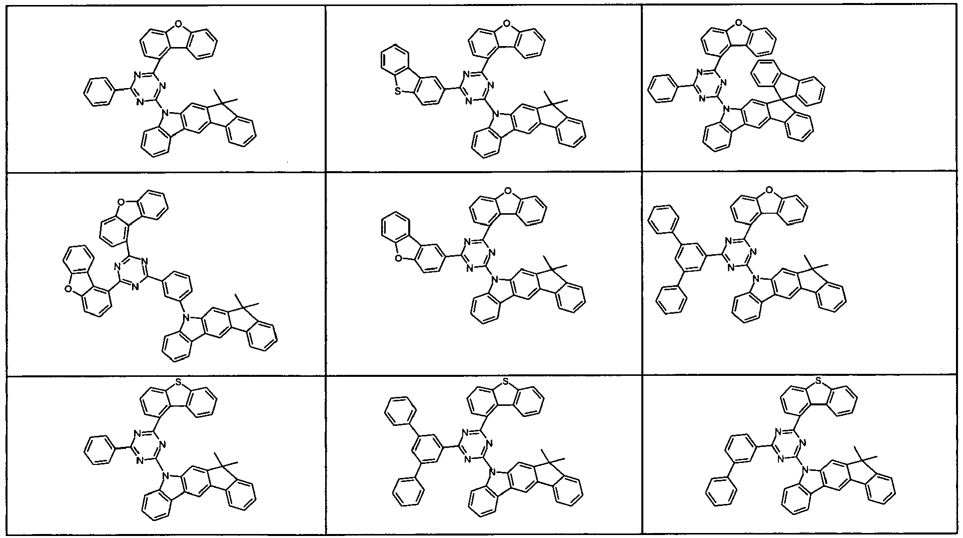

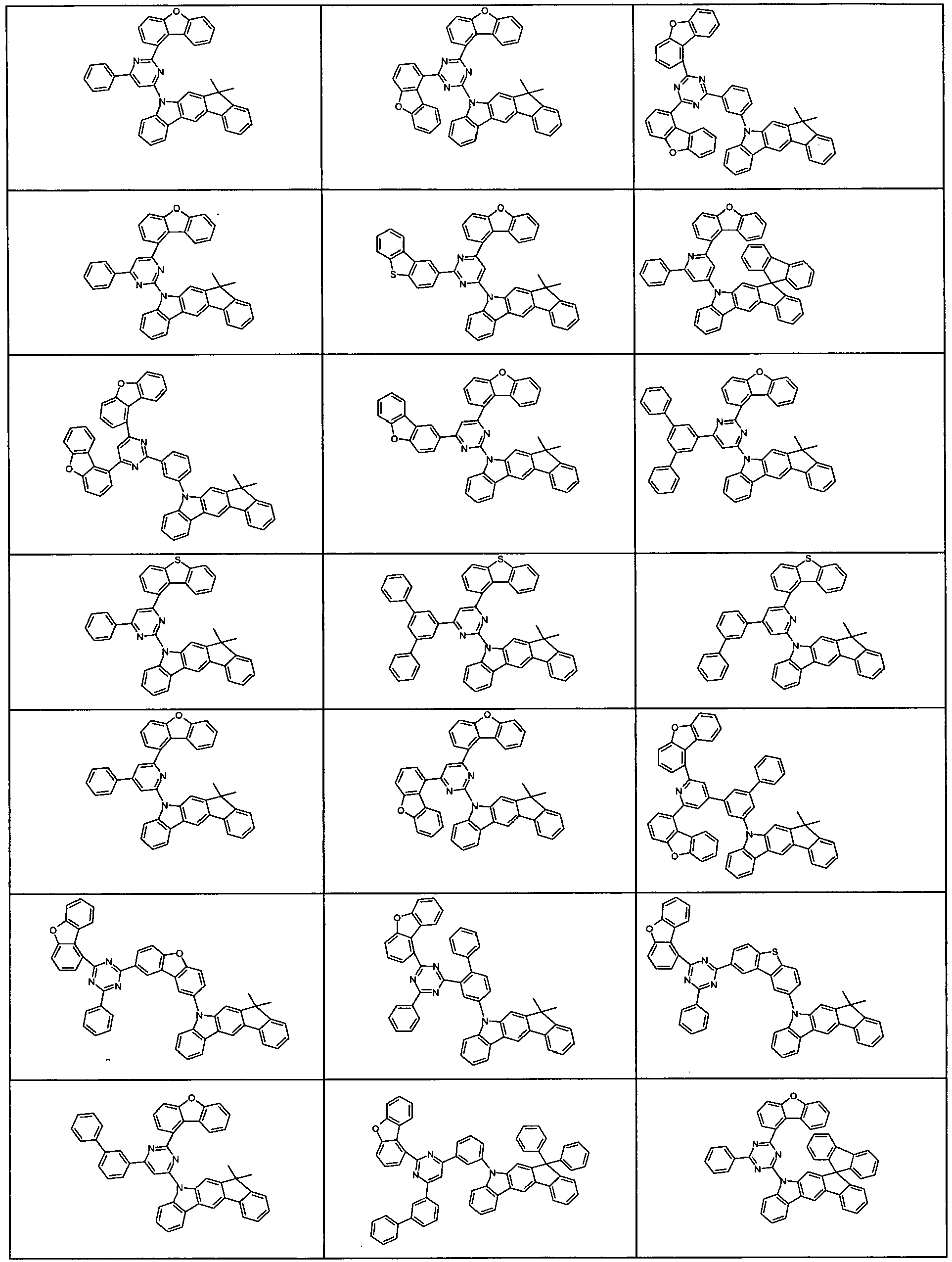

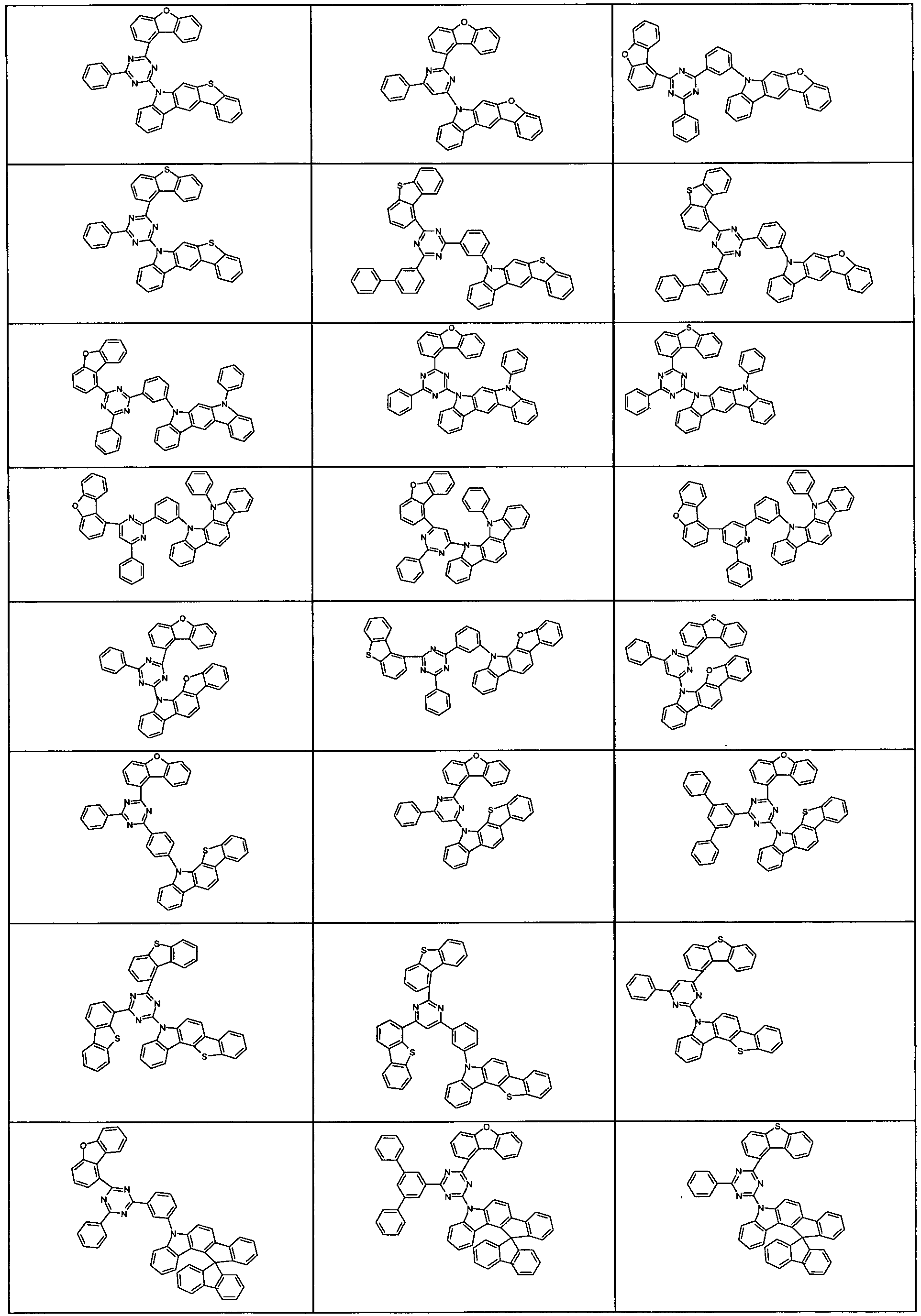

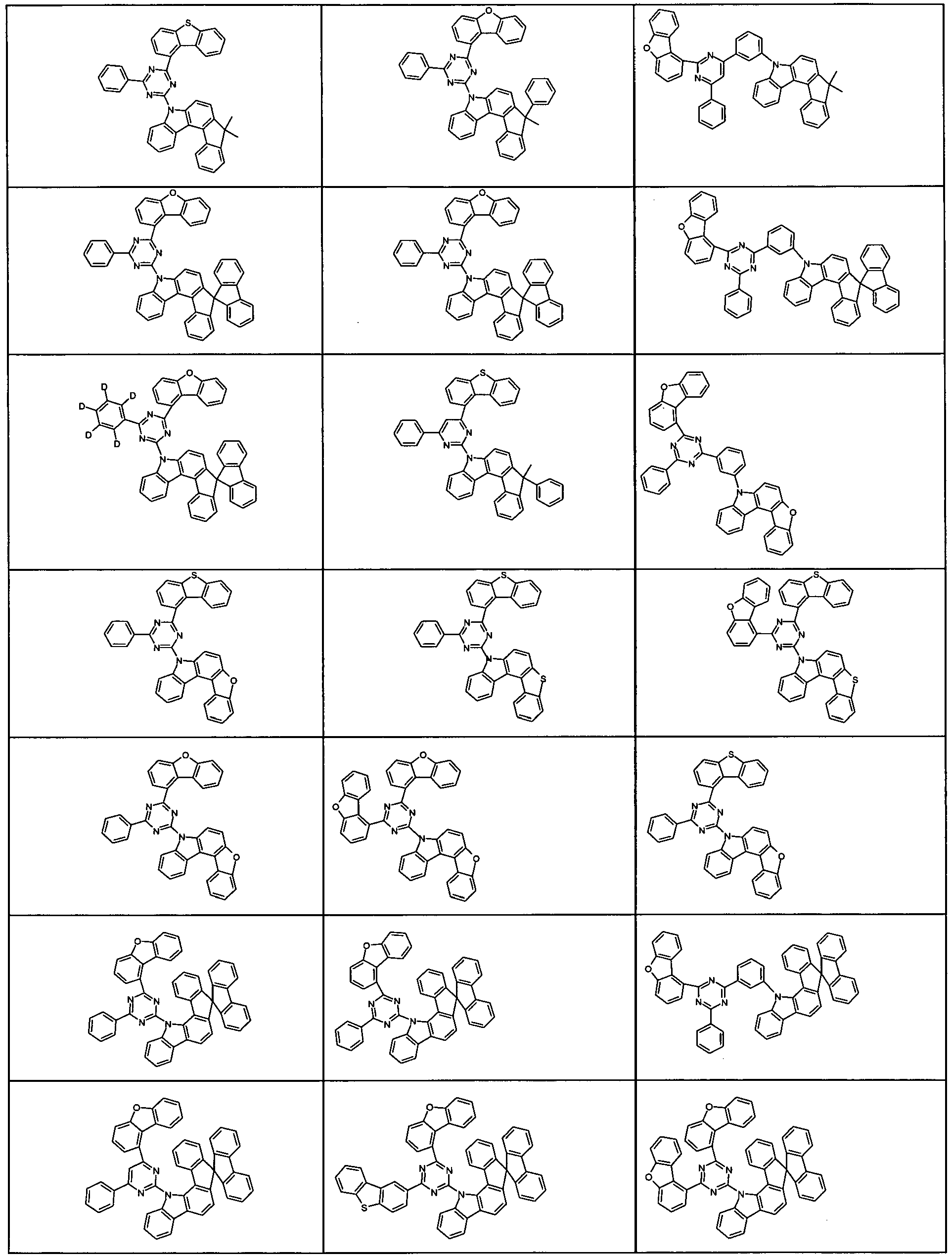

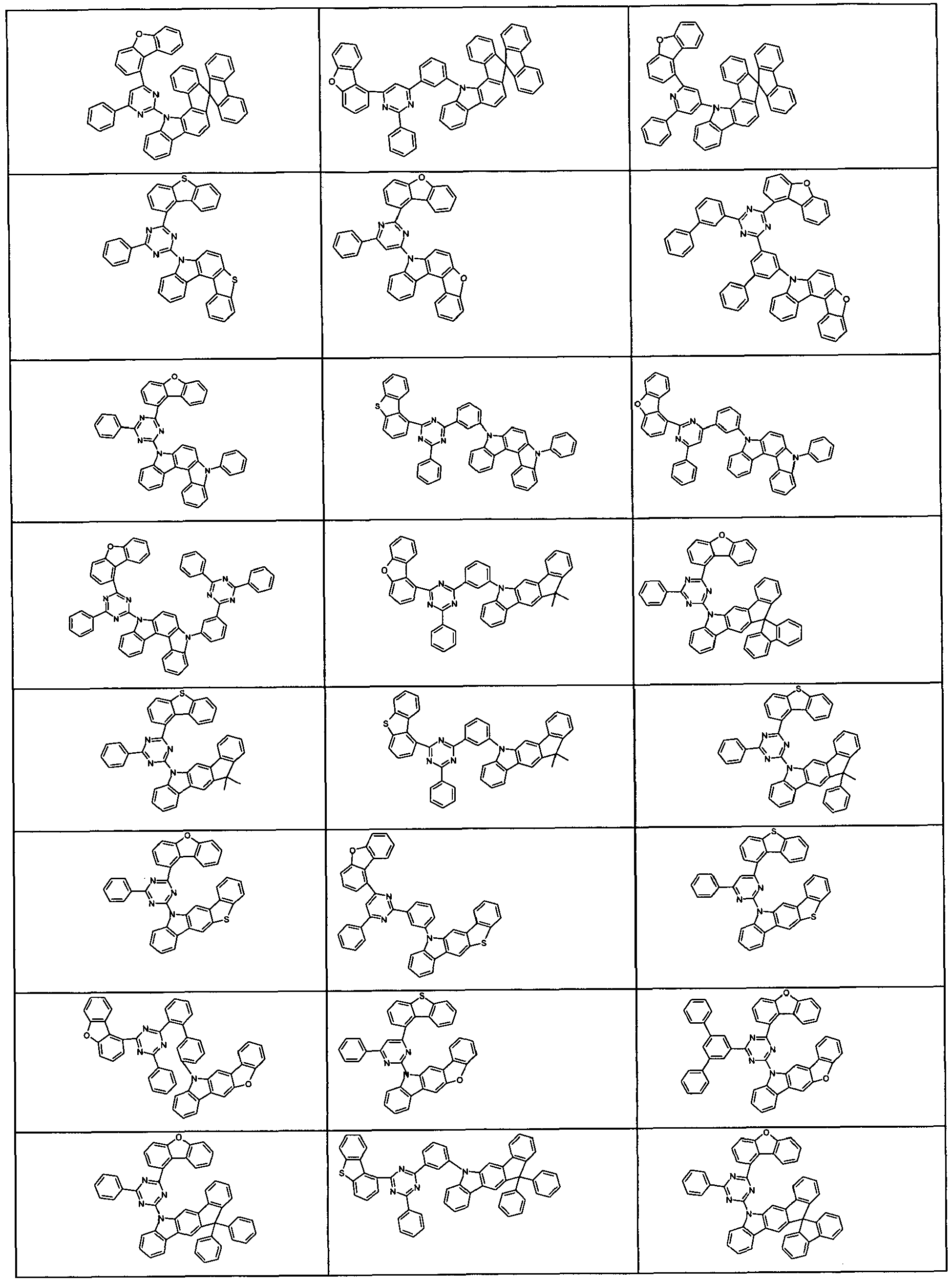

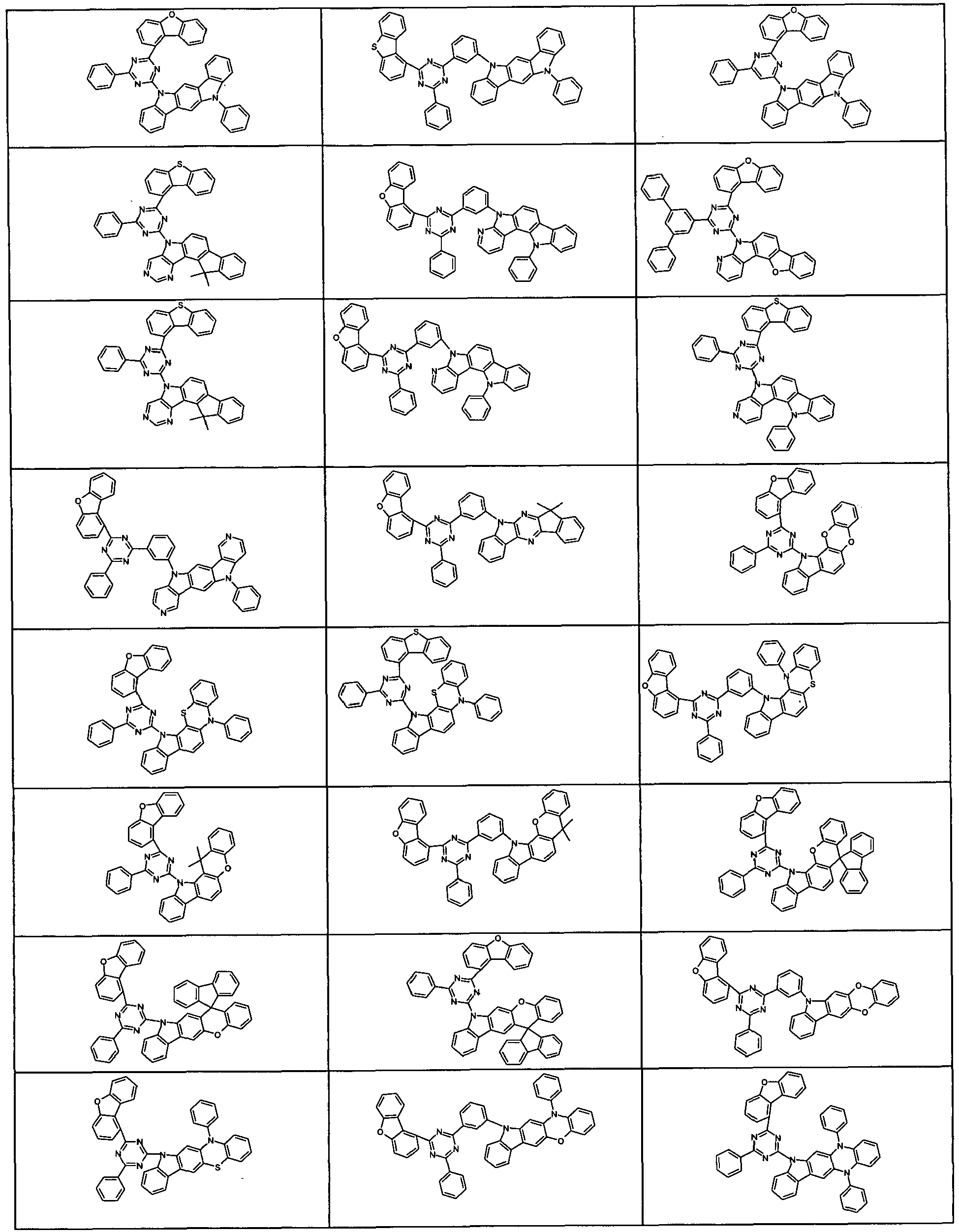

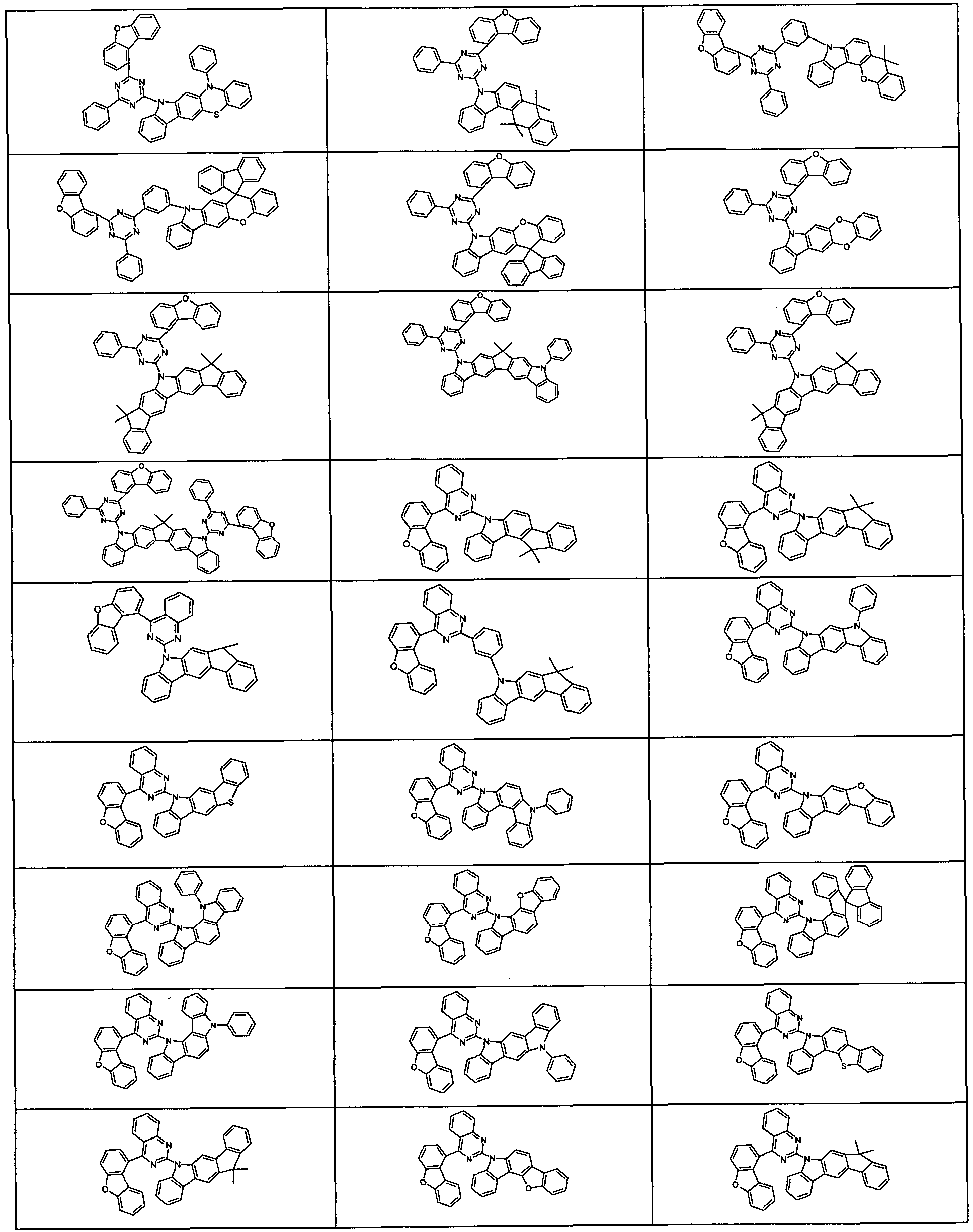

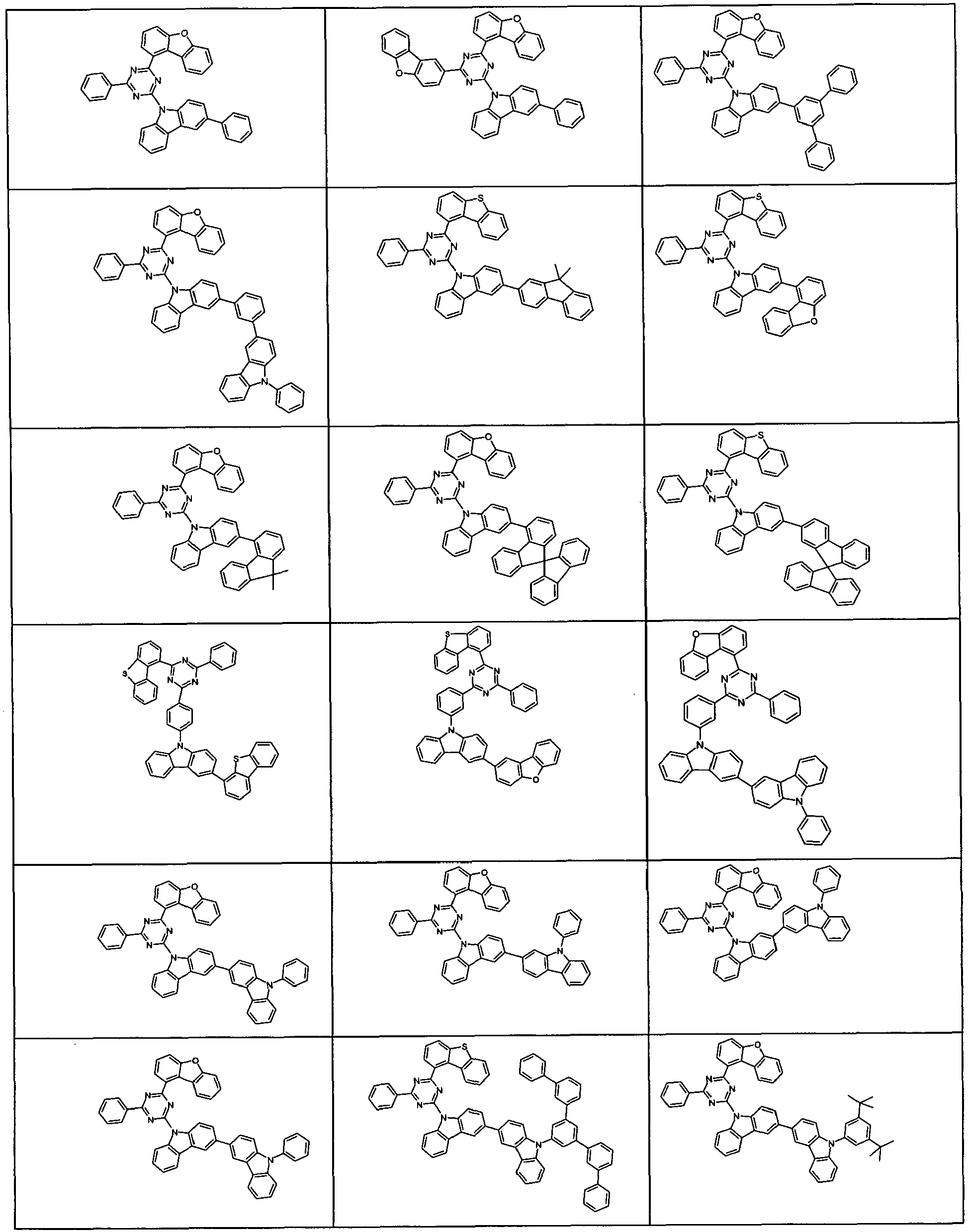

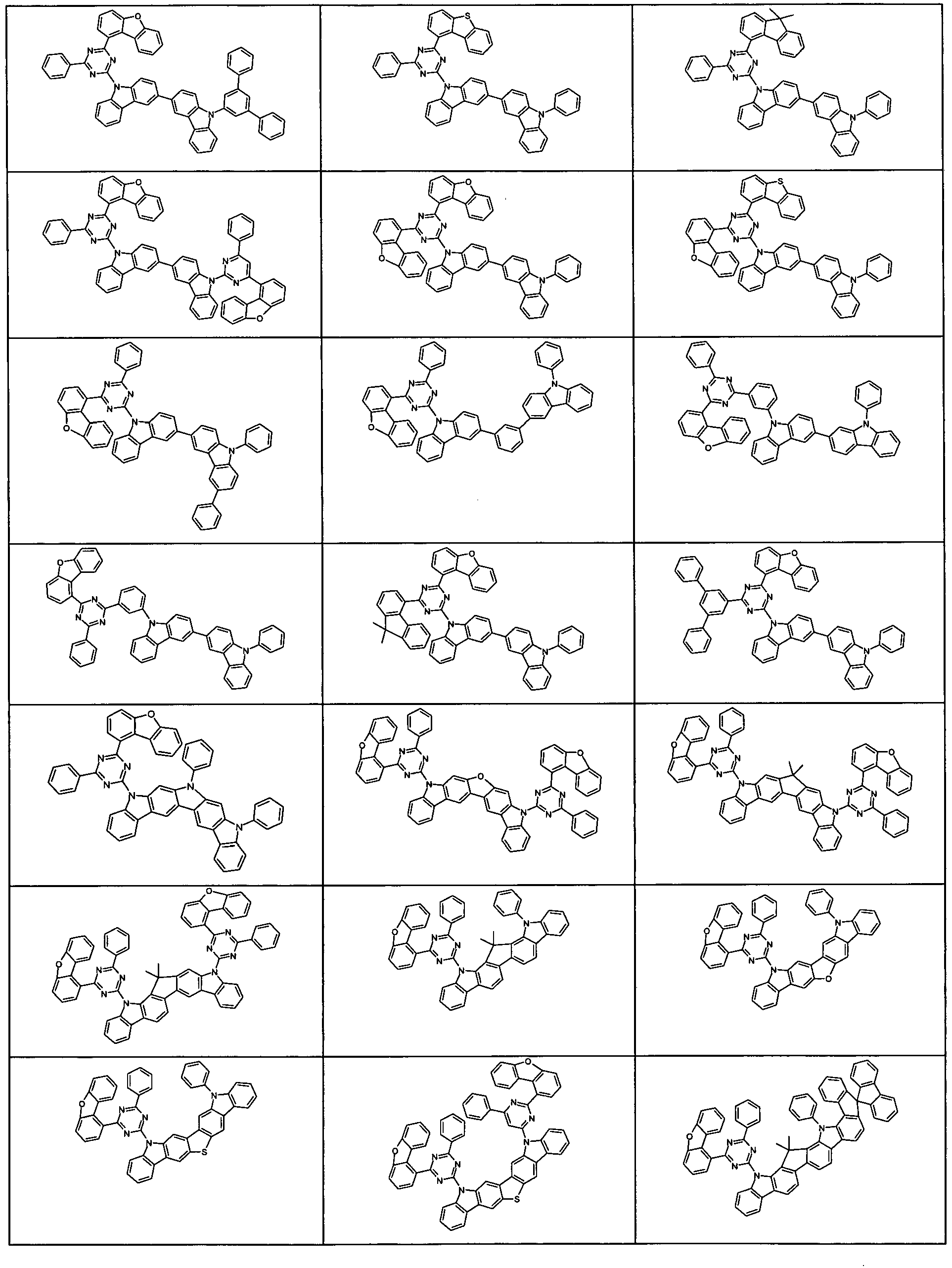

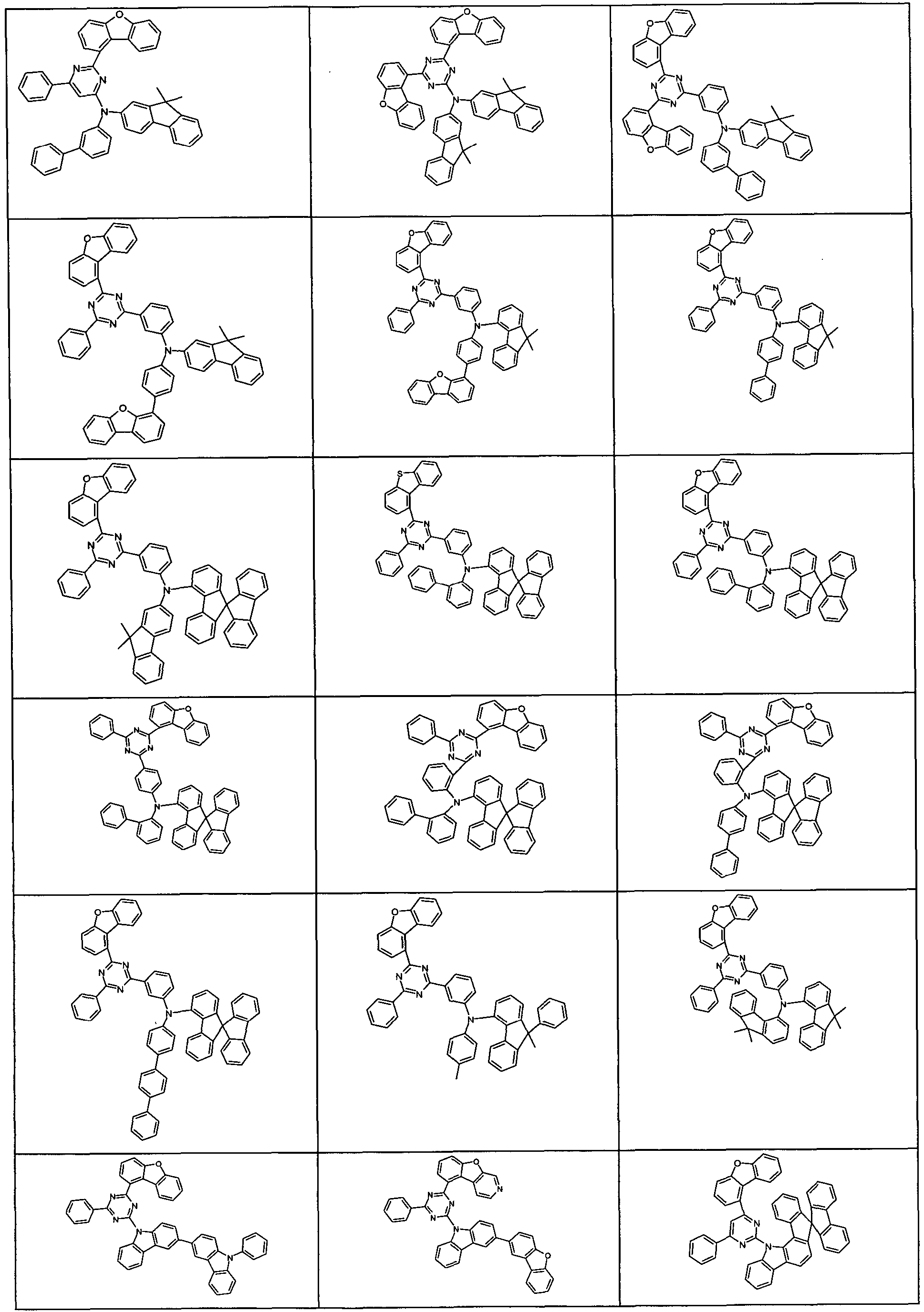

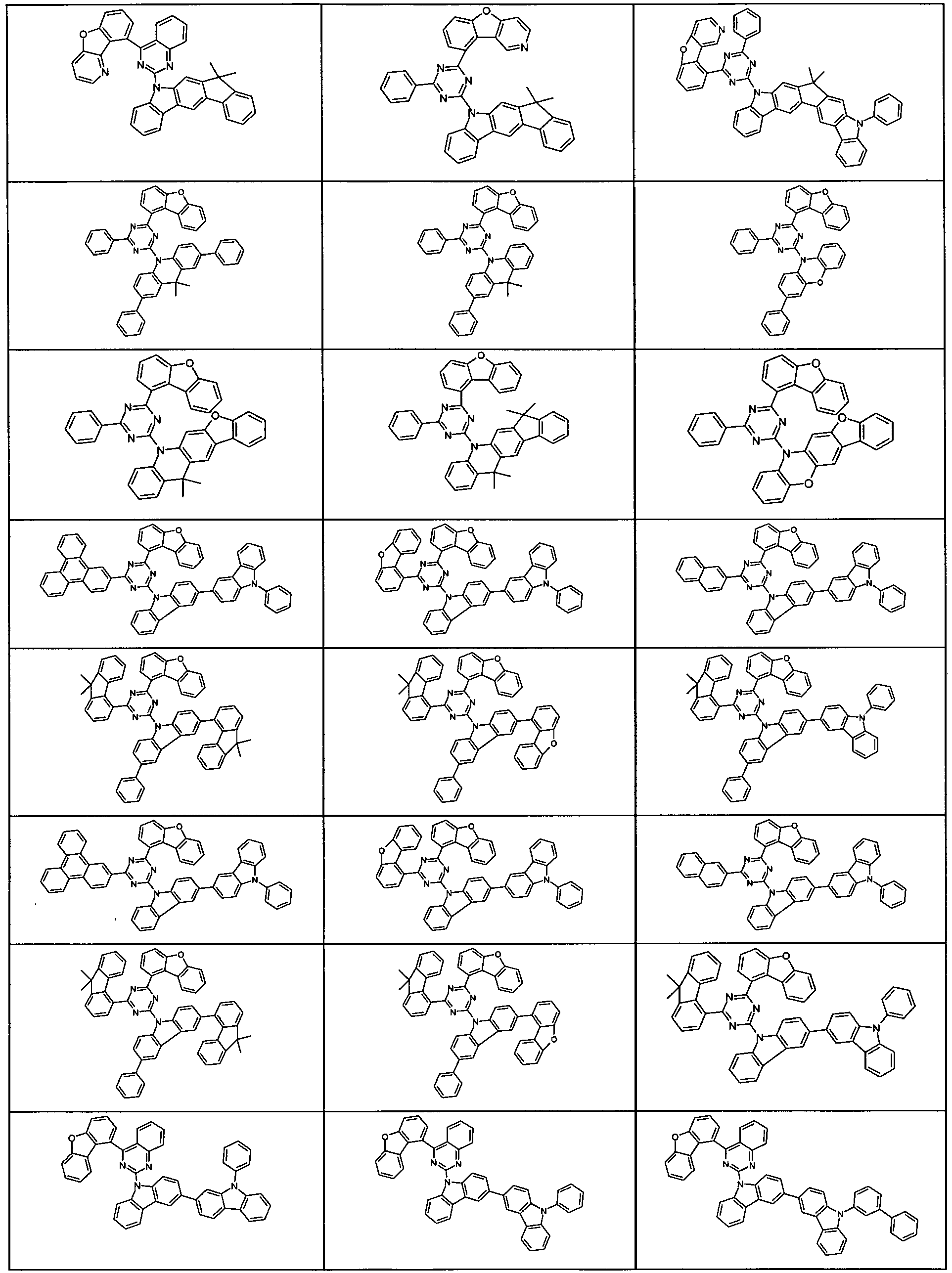

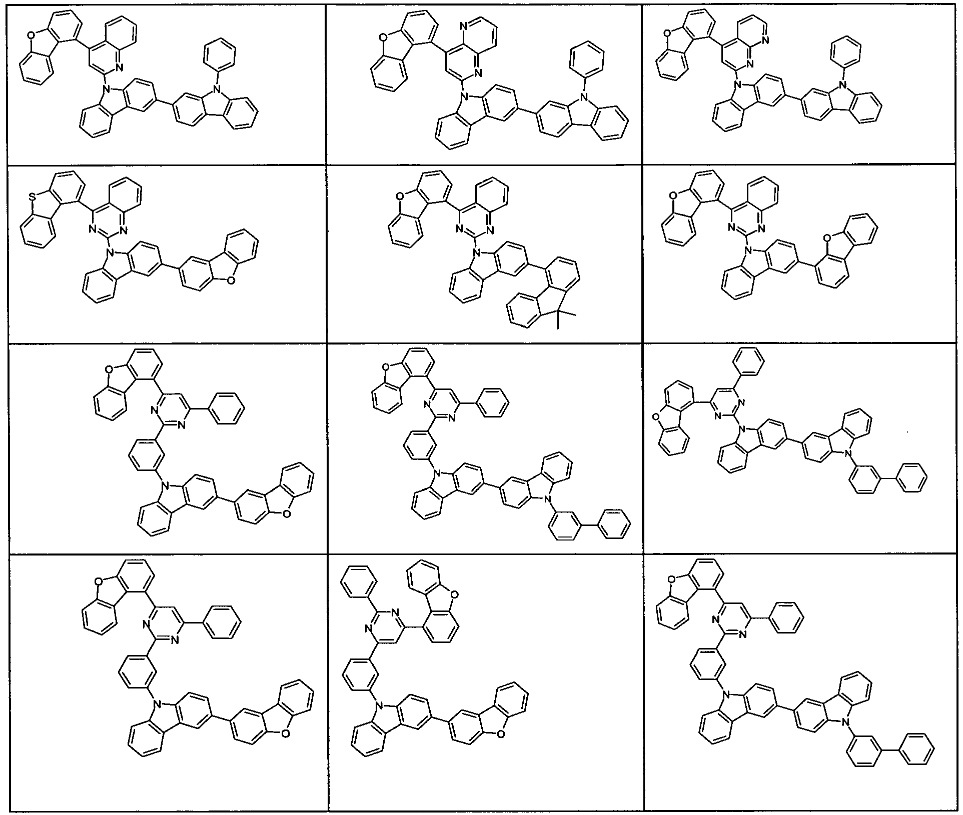

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt.

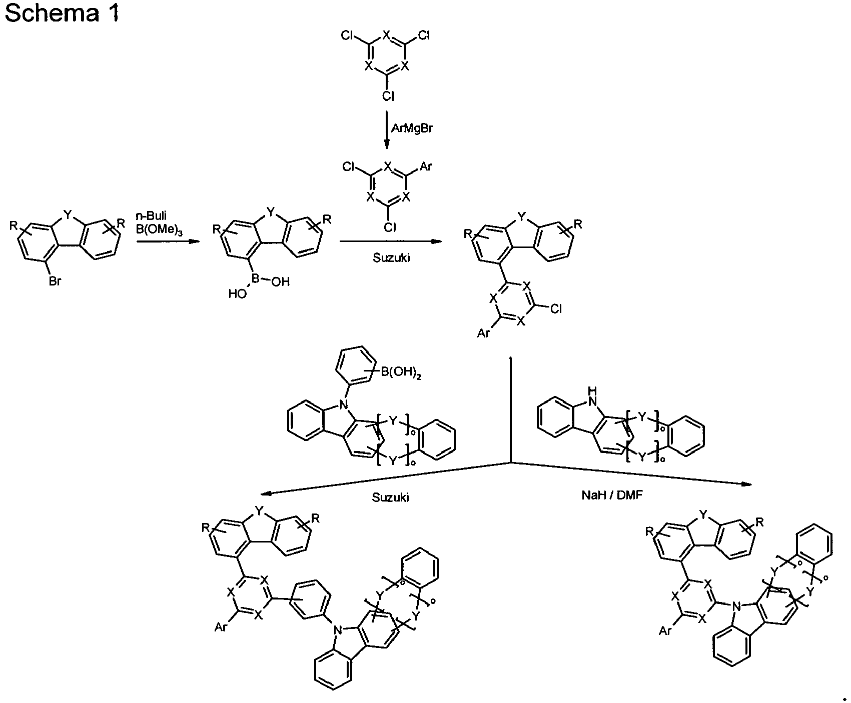

Die Synthese geht aus von gegebenenfalls substituiertem 1-Halogen-Dibenzofuran bzw. -Dibenzothiophen, welches zur entsprechenden Boronsäure bzw. einem Boronsäurederivat umgesetzt werden kann. Im nächsten Schritt kann die Gruppe HetAr, welche noch eine weitere Abgangsgruppe trägt, durch Suzuki-Kupplung eingeführt werden. Im letzten Schritt kann diese weitere Abgangsgruppe an der Gruppe HetAr umgesetzt werden, entweder in einer Suzuki-Kupplung zur Einführung einer Gruppe -L-N¹, wobei L für ein aromatisches oder heteroaromatisches Ringsystem steht, oder in einer C-N-Kupplungsreaktion, beispielsweise einer Hartwig-Buchwald-Kupplung, oder einer nukleophilen aromatischen Substitutionsreaktion zur Einführung der Gruppe N¹.

Das oben beschriebene allgemeine Verfahren zur Synthese der erfindungsgemäßen Verbindungen ist exemplarisch. Der Fachmann kann alternative Synthesewege im Rahmen seines allgemeinen Fachwissens entwickeln.

Ein weiterer Gegenstand der vorliegenden Erfindung ist daher ein Verfahren zur Synthese der erfindungsgemäßen Verbindungen, ausgehend von 1-Halogen-Dibenzofuran bzw. 1-Halogen-Dibenzothiophen, wobei das Halogen bevorzugt Brom ist, gekennzeichnet durch die folgenden Schritte:
(1) optional Umsetzung der Halogengruppe zu einer Boronsäure bzw. einem Boronsäurederivat;
(2) Einführung der Gruppe HetAr durch eine Kupplungsreaktion, insbesondere eine Suzuki-Kupplung;
(3) Einführung der Gruppe N¹ durch eine Kupplungsreaktion, insbesondere eine Hartwig-Buchwald-Kupplung oder eine nukleophile aromatische Substitution, oder der Gruppe -L-N¹ durch eine Kupplungsreaktion, insbesondere eine Suzuki-Kupplung.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Lactame, Carbazolderivate und Indenocarbazolderivate.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094962, WO 2014/094961 oder WO 2014/094960 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer in einer Lochblockier- oder Elektronentransportschicht einzusetzen. Dies gilt insbesondere für erfindungsgemäße Verbindungen, die keine Carbazolstruktur aufweisen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen.

Weiterhin bevorzugt ist eine organische Elektrolumineszenzvorrichtung, dadurch gekennzeichnet, dass eine oder mehrere Schichten mit einem Sublimationsverfahren aufgebracht werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation aufgebracht werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar. Weiterhin weisen die Verbindungen eine hohe Glasübergangstemperatur und eine hohe thermische Stabilität auf.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### a) 6-Brom-2-fluor-2'-methoxy-biphenyl

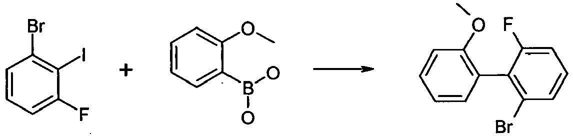

200 g (664 mmol) 1-Brom-3-fluor-2-iod-benzol, 101 g (664 mmol) 2-Methoxyphenylboronsäure und 137.5 g (997 mmol) Natriumtetraborat werden in 1000 mL THF und 600 ml Wasser gelöst und entgast. Es wird mit 9.3 g (13.3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

Analog dazu werden die folgenden Verbindungen hergestellt:

| | **Edukt 1** | **Edukt 2** | **Produkt** | **Ausbeute** |
|---|---|---|---|---|
| a1 | 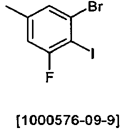 | 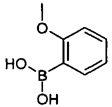 | 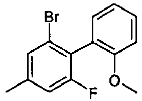 | 77% |
| a2 | 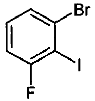 | 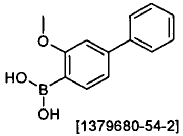 | 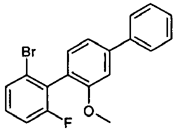 | 74% |
| a3 | 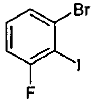 | 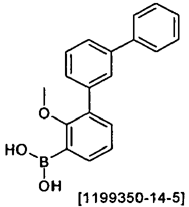 | 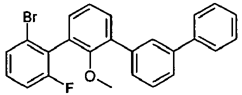 | 76% |
| a4 | 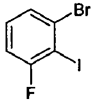 | 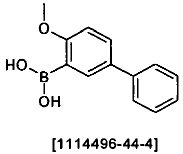 | 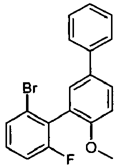 | 71% |

### b) 6'-Brom-2'-fluor-biphenyl-2-ol

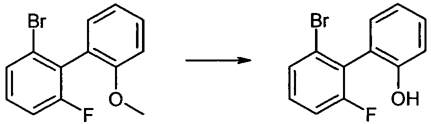

112 g (418 mmol) 6-Brom-2-fluor-2'-methoxy-biphenyl werden in 2 L Dichlormethan gelöst und auf 5 °C gekühlt. Zu dieser Lösung werden innerhalb von 90 min. 41.01 ml (431 mmol) Bortribromid zugetropft und über Nacht weiter gerührt. Das Gemisch wird im Anschluss langsam mit Wasser versetzt, die organische Phase dreimal mit Wasser gewaschen, über Na₂SO₄ getrocknet, einrotiert und chromatographisch gereinigt. Ausbeute: 104 g (397 mmol), 98 % der Theorie.

Analog dazu werden die folgenden Verbindungen hergestellt:

| | **Edukt 1** | **Produkt** | **Ausbeute** |
|---|---|---|---|
| b1 | 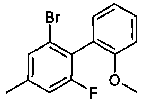 | 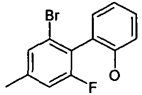 | 92% |
| b2 | 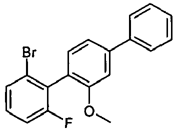 | 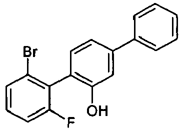 | 90% |
| b3 | 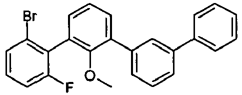 | 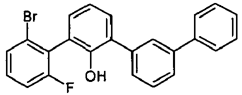 | 93% |
| b4 | 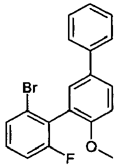 | 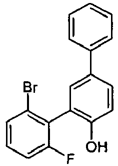 | 94% |

### c) 1-Brom-dibenzofuran

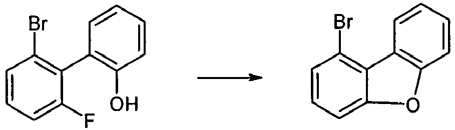

111 g (416 mmol) 6'-Brom-2'-fluor-biphenyl-2-ol werden in 2 L DMF (max. 0.003% H₂O)SeccoSolv® gelöst und auf 5 °C gekühlt. Zu dieser Lösung wird portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird im Anschluss nach dem Abkühlen langsam mit 500 ml Ethanol versetzt, komplett einrotiert und dann chromatographisch gereinigt. Ausbeute: 90g (367 mmol), 88.5 % der Theorie.

Analog dazu werden die folgenden Verbindungen hergestellt:

| | **Edukt 1** | **Produkt** | **Ausbeute** |
|---|---|---|---|
| c1 | 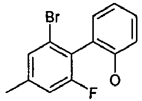 | 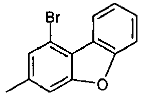 | 81% |
| c2 | 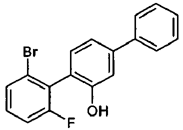 | 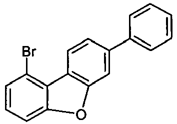 | 78% |
| c3 | 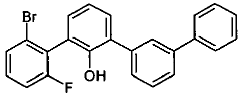 | 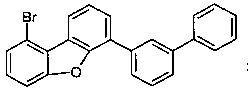 | 73% |
| c4 | 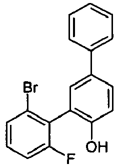 | 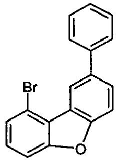 | 79% |

### d) Dibenzofuran-1-boronsäure

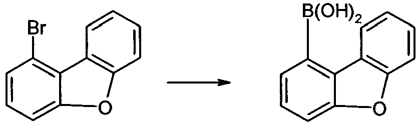

180 g (728 mmol) 1-Brom-dibenzofuran werden in 1500 mL trockenem THF gelöst und auf -78 °C gekühlt. Bei dieser Temperatur wird mit 305 mL (764 mmol / 2.5 M in Hexan) n-Butyllithium innerhalb von ca. 5 min. versetzt und anschließend für 2.5 h bei -78 °C nachgerührt. Dann wird bei dieser Temperatur mit 151 g (1456 mmol) Borsäure-trimethylester möglichst zügig versetzt, und die Reaktionsmischung wird langsam auf Raumtemperatur kommen gelassen (ca. 18 h). Die Reaktionslösung wird mit Wasser gewaschen und der ausgefallene Feststoff und die organische Phase mit Toluol azeotrop getrocknet. Das Rohprodukt wird aus Toluol/ Methylenchlorid bei ca. 40 °C ausgerührt und abgesaugt. Ausbeute: 146 g (690 mmol), 95 % der Theorie.

Analog dazu werden die folgenden Verbindungen hergestellt:

| | **Edukt 1** | **Produkt** | **Ausbeute** |
|---|---|---|---|
| d1 | 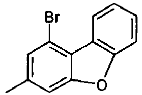 | 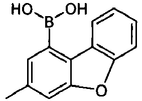 | 81% |
| d2 | 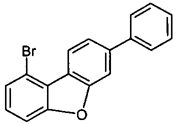 | 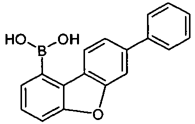 | 78% |
| d3 | 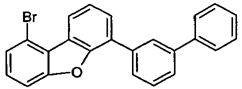 | 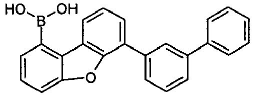 | 73% |
| d4 | 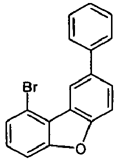 | 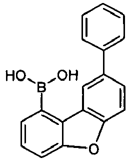 | 79% |
| d5 | 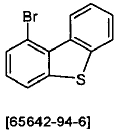 | 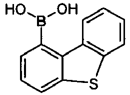 | 73% |

### e) 2-Chlor-4-dibenzofuran-1-yl-6-phenyl-[1,3,5]triazin

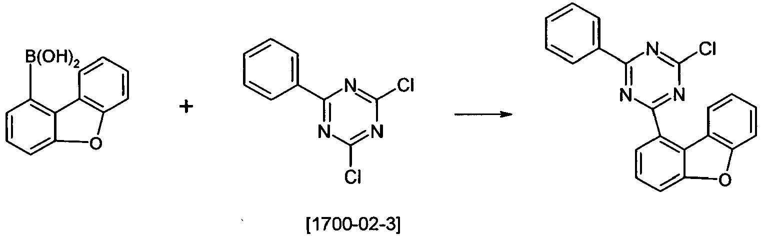

37.8 g (153.0 mmol) Dibenzofuran-1-boronsäure, 34.6 g (153.0 mmol) 2,4-Dichlor-6-phenyl-1,3,5-triazin und 17 g (168.0 mmol) Natriumcarbonat werden in 120 mL Toluol, 300 mL Dioxan und 300 mL Wasser suspendiert. Zu dieser Suspension werden 1.7 g (1.5 mmol) Tetrakis(triphenylphosphin)palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan /Heptan umkristallisiert. Die Ausbeute beträgt 46 g (131 mmol), entsprechend 86 % der Theorie.

Analog dazu werden die folgenden Verbindungen hergestellt:

| | **Edukt 1** | **Edukt 2** | **Produkt** | **Ausbeute** |
|---|---|---|---|---|
| e1 | 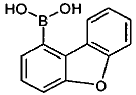 | 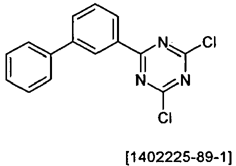 | 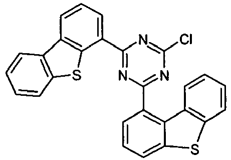 | 74% |
| e2 | 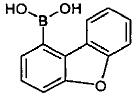 | 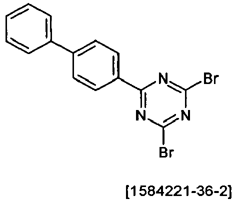 | 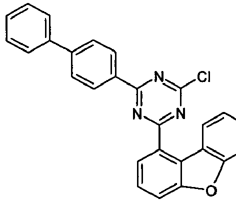 | 79% |
| e3 | 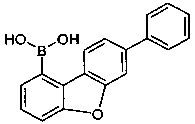 | 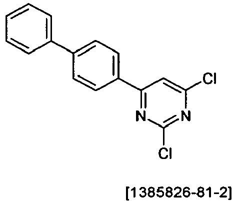 | 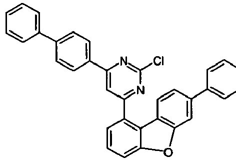 | 78% |
| e4 | 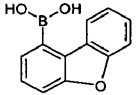 | 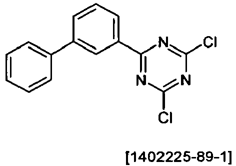 | 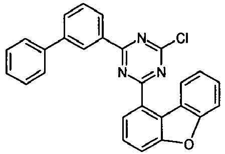 | 79% |
| e5 | 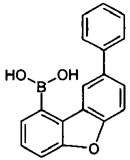 | 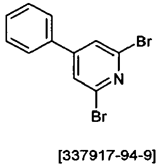 | 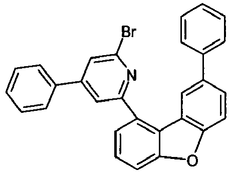 | 68% |
| e6 | 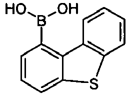 | 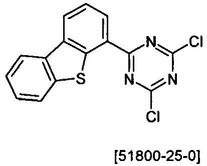 | 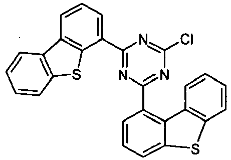 | 80% |
| e7 | 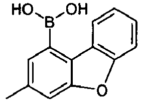 | 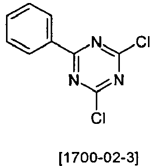 | 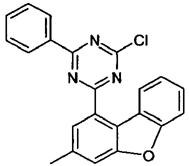 | 85% |
| e8 | 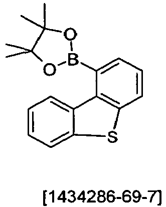 | 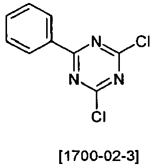 | 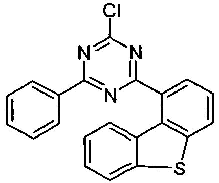 | 84% |
| e9 | 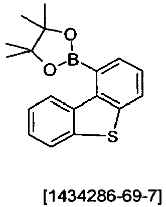 | 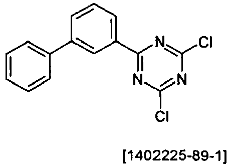 | 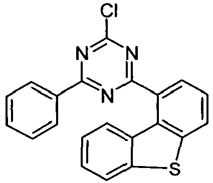 | 80% |
| e10 | 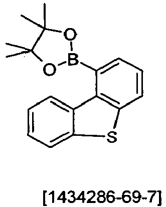 | 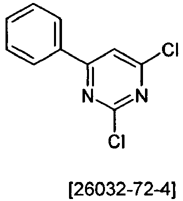 | 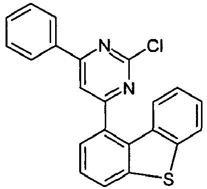 | 83% |
| e11 | 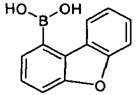 | 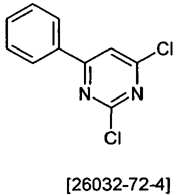 | 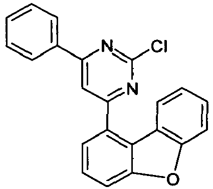 | 80% |

### f) 10-(4-Dibenzofuran-1-yl-6-phenyl-[1,3,5]triazin-2-yl)-12,12-dimethyl-10,12-dihydro-10-aza-indeno[2,1-b]fluoren

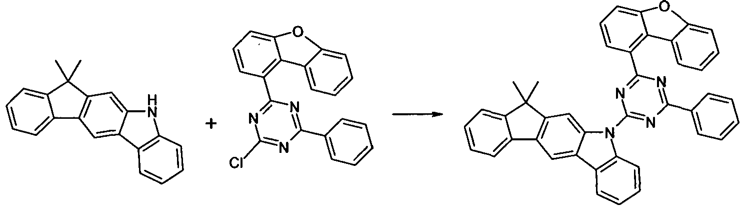

8 g (28,2 mmol) 12,12-Dimethyl-10,12-dihydro-10-aza-indeno[2,1-b]fluoren werden in 225 ml Dimethylformamid unter Schutzgasatmosphäre gelöst und mit 1.5 g NaH, 60%ig in Mineralöl, (37.5 mmol) versetzt. Nach 1 h bei Raumtemperatur wird eine Lösung von 11.3 g (31.75 mmol) 2-Chlor-4-dibenzofuran-1-yl-6-phenyl-[1,3,5]triazin in 75 mL Dimethylformamid zugetropft. Das Reaktionsgemisch wird 12 h bei Raumtemperatur gerührt, dann auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert, und aus Chlorbenzol umkristallisiert (HPLC-Reinheit >99.9%) und im Vakuum sublimiert. Die Ausbeute beträgt 15.2 g (25 mmol), entsprechend 80 % der Theorie.

Analog dazu werden die folgenden Verbindungen hergestellt:

| | **Edukt 1** | **Edukt 2** | **Produkt** | **Ausbeute** |
|---|---|---|---|---|
| f1 | 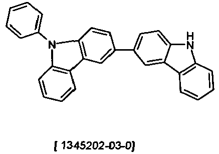 | 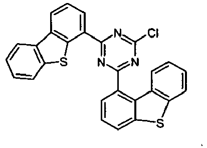 | 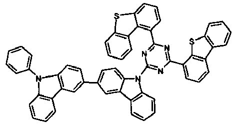 | 69% |
| f2 | 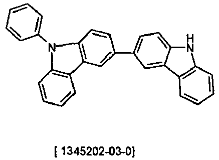 | 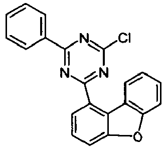 | 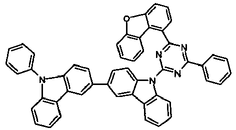 | 73% |
| f3 | 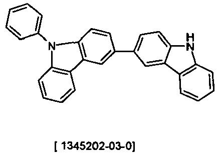 | 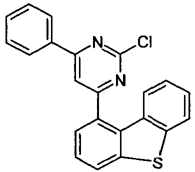 | 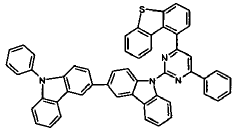 | 79% |
| f4 | 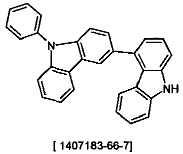 | 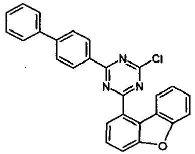 | 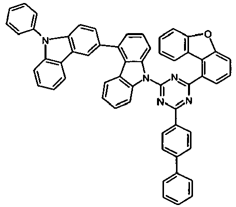 | 78% |
| f5 | 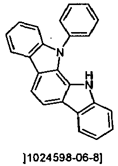 | 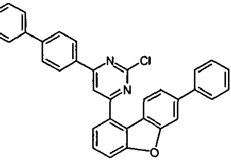 | 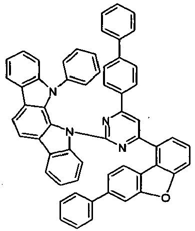 | 75% |
| f6 | 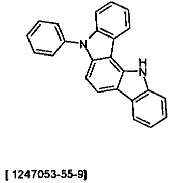 | 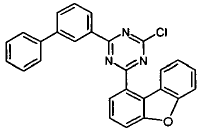 | 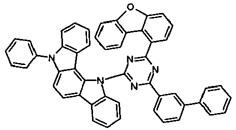 | 68% |
| f7 | 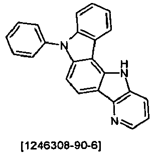 | 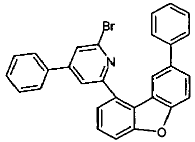 | 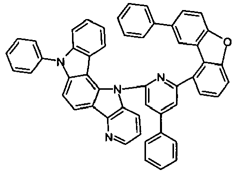 | 80% |
| f8 | 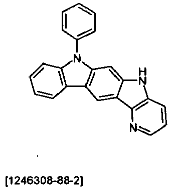 | 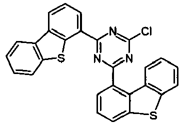 | 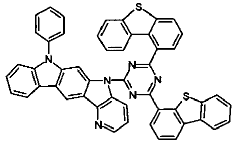 | 88% |
| f9 | 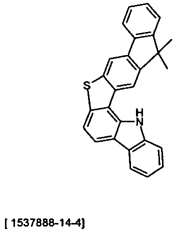 | 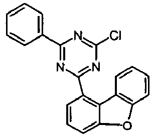 | 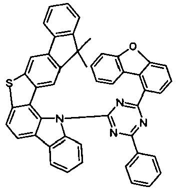 | 74% |
| f10 | 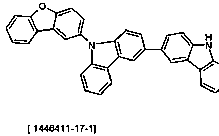 | 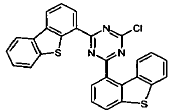 | 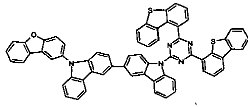 | 80% |
| f11 | 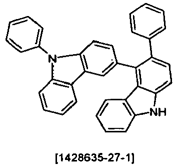 | 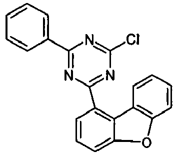 | 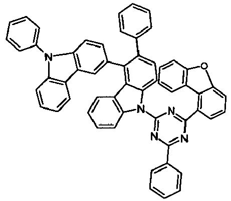 | 80% |
| f12 | 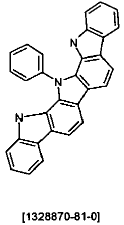 | 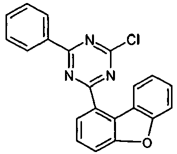 | 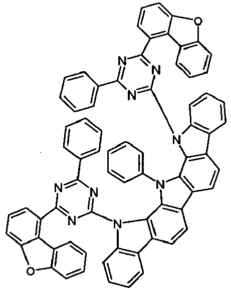 | 78% |
| f13 | 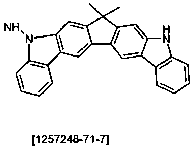 | 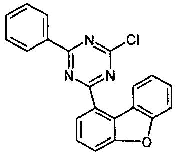 | 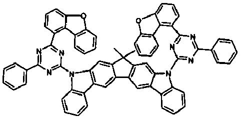 | 83% |
| f14 | 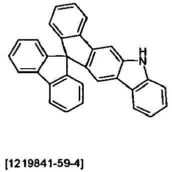 | 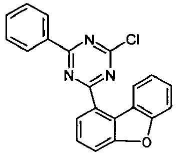 | 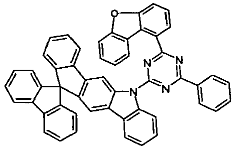 | 85% |
| f15 | 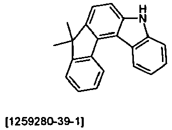 | 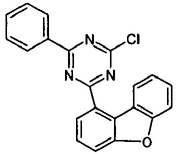 | 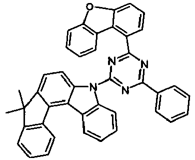 | 75% |
| f16 | 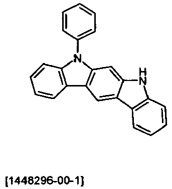 | 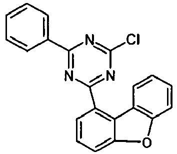 | 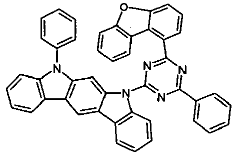 | 83% |
| f17 | 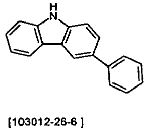 | 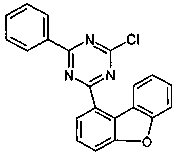 | 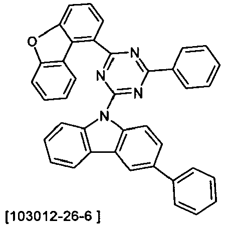 | 85% |
| f18 | 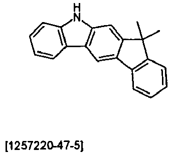 | 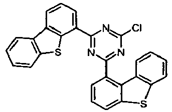 | 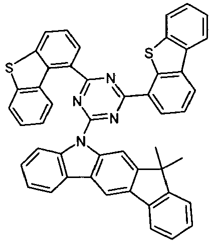 | 79% |
| f19 | 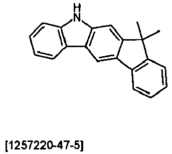 | 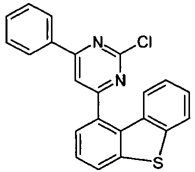 | 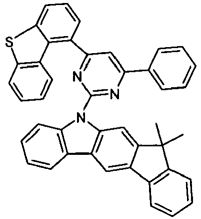 | 76% |
| f20 | 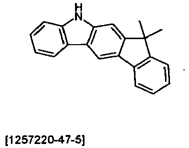 | 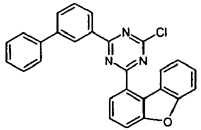 | 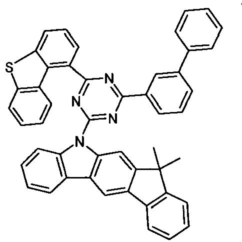 | 75% |
| f21 | 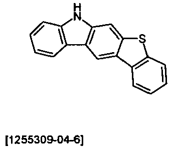 | 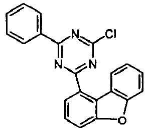 | 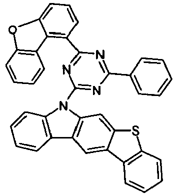 | 65% |
| f22 | 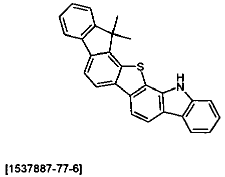 | 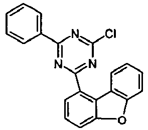 | 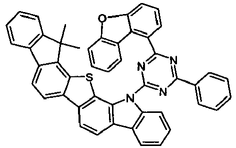 | 80% |
| f23 | 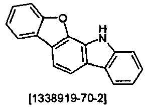 | 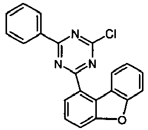 | 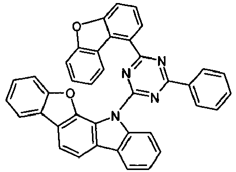 | 84% |
| f24 | 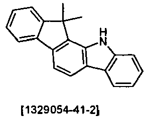 | 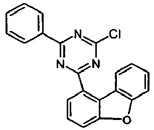 | 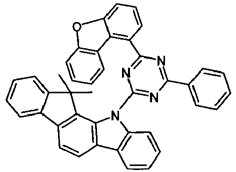 | 78% |
| f25 | 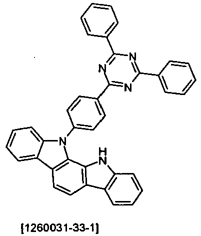 | 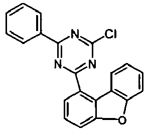 | 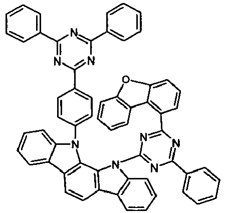 | 81% |
| f26 | 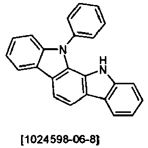 | 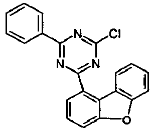 | 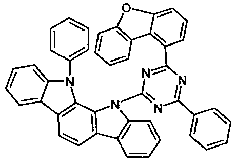 | 83% |
| f27 | 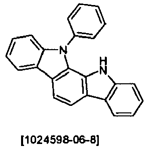 | 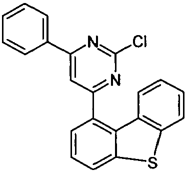 | 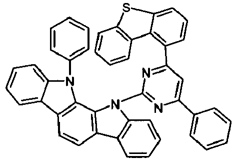 | 78% |
| f28 | 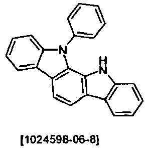 | 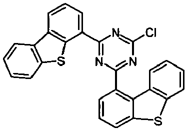 | 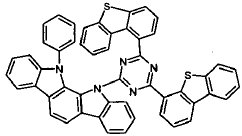 | 85% |
| f29 | 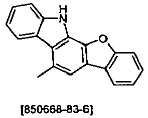 | 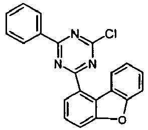 | 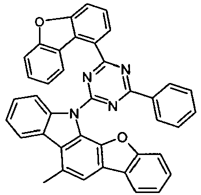 | 85% |
| f30 | 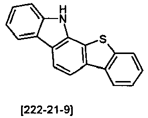 | 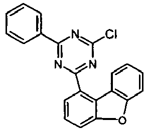 | 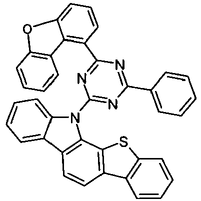 | 75% |
| f31 | 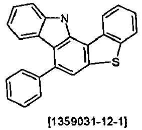 | 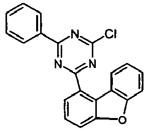 | 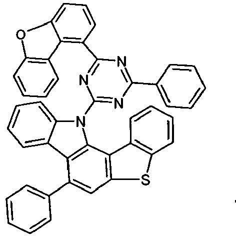 | 82% |
| f32 | 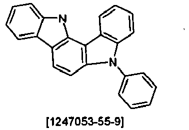 | 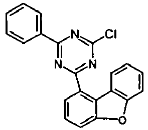 | 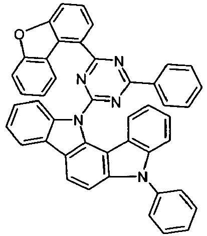 | 85% |
| f33 | 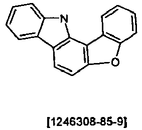 | 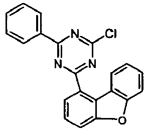 | 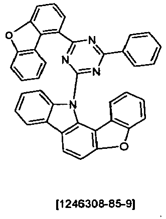 | 75% |
| f34 | 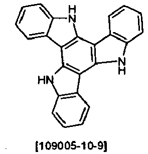 | 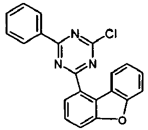 | 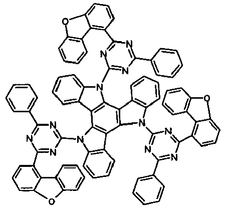 | 78% |
| f35 | 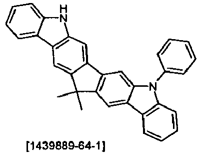 | 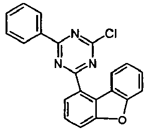 | 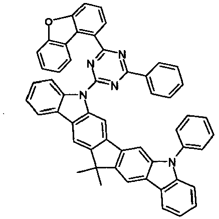 | 79% |
| f36 | 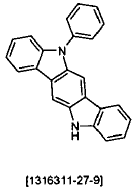 | 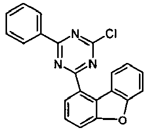 | 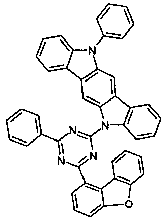 | 62% |
| f37 | 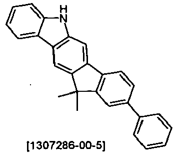 | 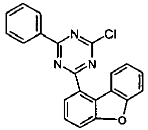 | 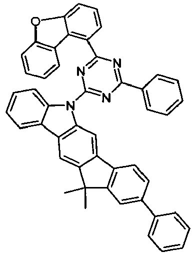 | 76% |
| f38 | 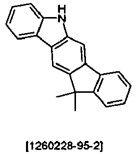 | 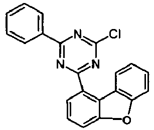 | 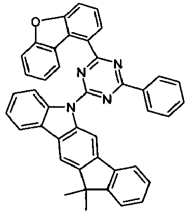 | 83% |
| f39 | 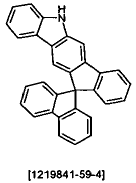 | 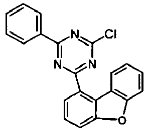 | 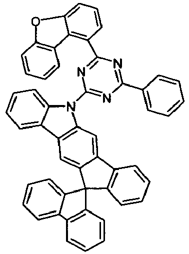 | 78% |
| f40 | 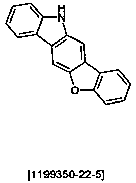 | 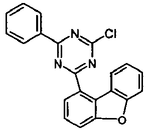 | 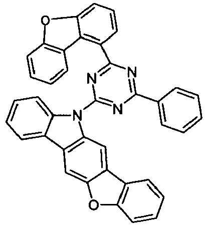 | 81% |
| f41 | 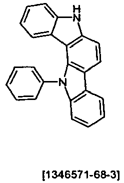 | 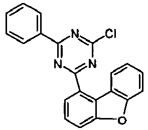 | 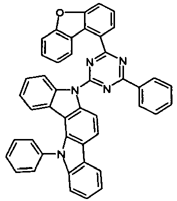 | 82% |
| f42 | 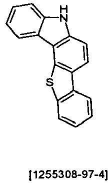 | 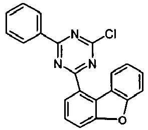 | 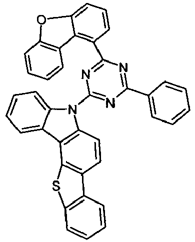 | 78% |
| f43 | 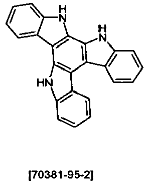 | 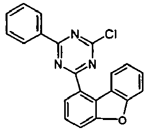 | 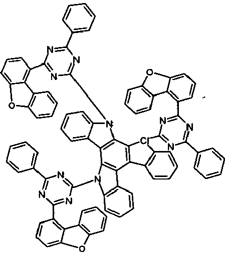 | 82% |
| f44 | 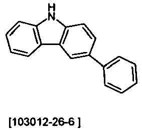 | 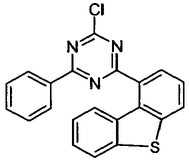 | 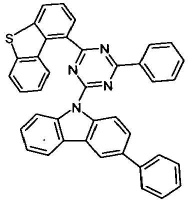 | 87% |
| f45 | 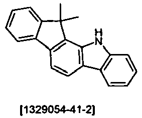 | 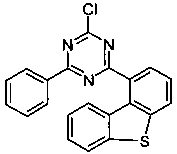 | 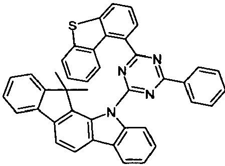 | 73% |
| f46 | 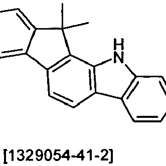 | 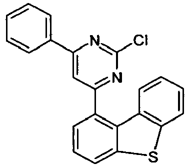 | 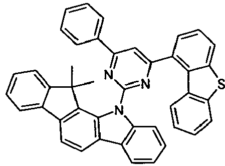 | 75% |
| f47 | 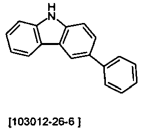 | 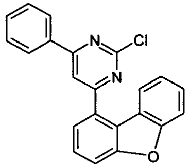 | 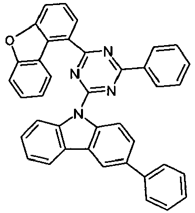 | 77% |
| f48 | 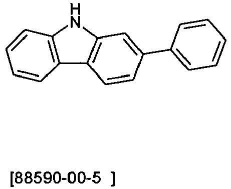 | 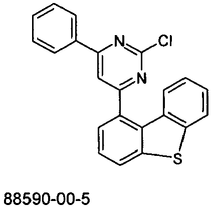 | 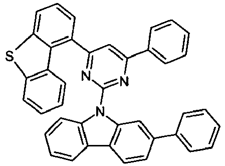 | 79% |
| f49 | 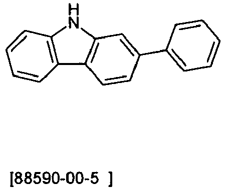 | 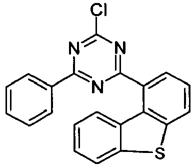 | 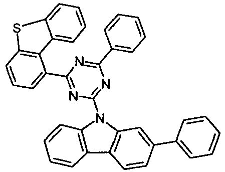 | 82% |
| f50 | 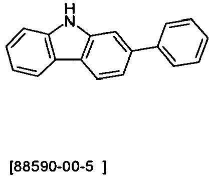 | 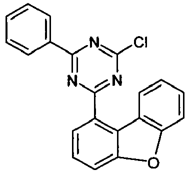 | 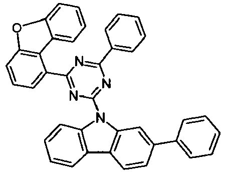 | 82% |

### g) 10-[3-(4-Dibenzofuran-1-yl-6-phenyl-[1,3,5]triazin-2-yl)-phenyl]-12,12-dimethyl-10,12-dihydro-10-aza-indeno[2,1-b]fluoren

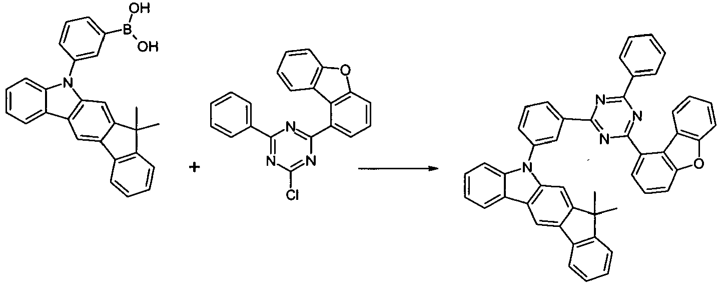

56 g (140 mmol) B-[3-[7,7-dimethylindeno[2,1-b]carbazol-5[7H]-yl)phenyl] boronsäure, 49 g (140 mmol) 2-Chlor-4-dibenzofuran-1-yl-6-phenyl-[1,3,5]triazin und 78.9 ml (158 mmol) Na₂CO₃ (2 M-Lösung in Wasser) werden in 120 mL Toluol, 120 mL Ethanol und 100 mL Wasser suspendiert. Zu dieser Suspension werden 2.6 g (2.2 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und zur Trockene eingeengt. Der Rückstand wird aus Toluol umkristallisiert. Der Rückstand wird mit Toluol heiß extrahiert, aus Chlorbenzol umkristallisiert (HPLC-Reinheit >99.9%) und im Vakuum sublimiert. Die Ausbeute beträgt 82 g (120 mmol), entsprechend 87% der Theorie.

Analog dazu werden die folgenden Verbindungen hergestellt:

| | **Edukt** 1 | Edukt 2 | **Produkt** | **Ausbeute** |
|---|---|---|---|---|
| g1 | 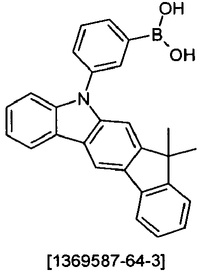 | 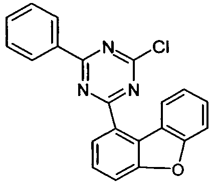 | 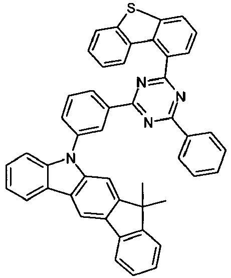 | 86% |
| g2 | 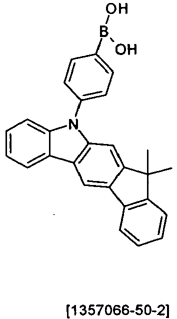 | 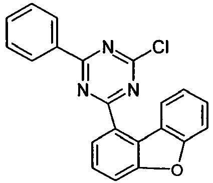 | 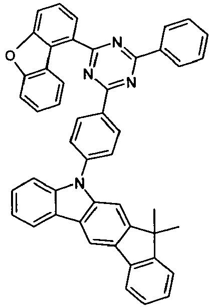 | 82% |
| g3 | 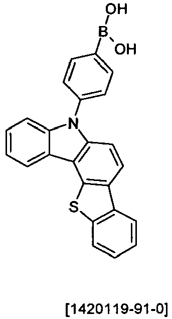 | 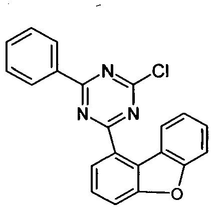 | 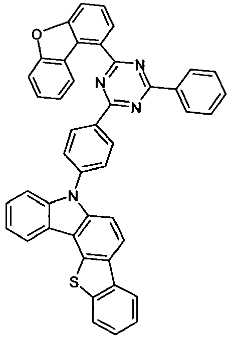 | 80% |
| g4 | 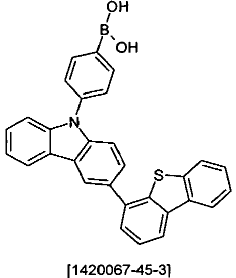 | 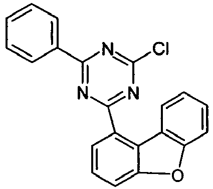 | 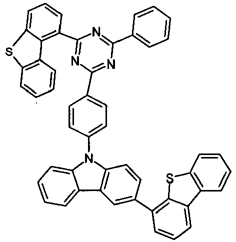 | 79% |
| g5 | 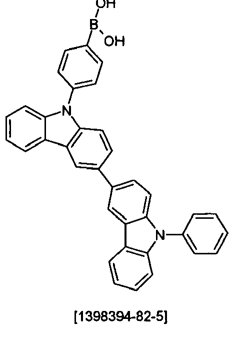 | 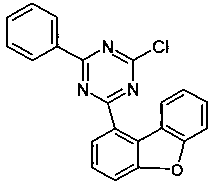 | 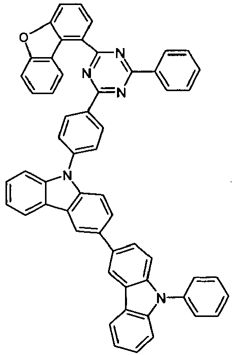 | 80% |
| g6 | 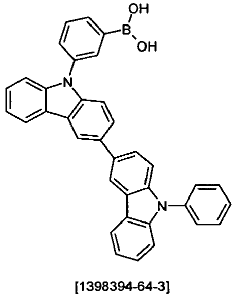 | 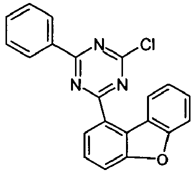 | 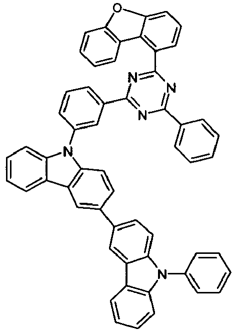 | 89% |
| g7 | 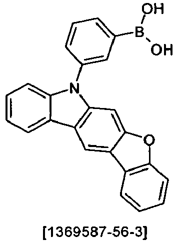 | 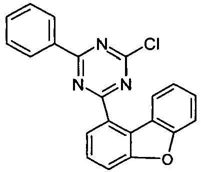 | 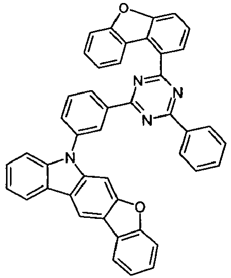 | 85% |
| g8 | 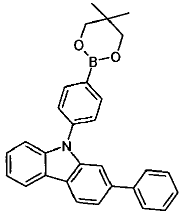 | 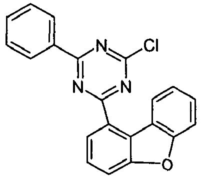 | 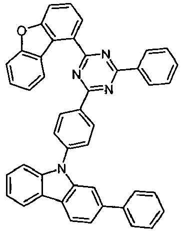 | 80% |

### h) Biphenyl-4-yl-(9,9-dimethyl-9H-fluoren-2-yl)-amin

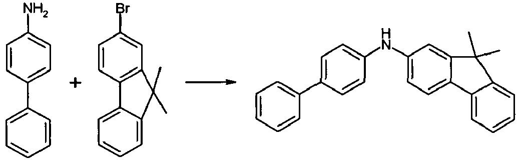

24.0 g (142 mmol, 1.2 eq) 4-Aminobiphenyl (CAS 92-67-1) und 32.0 g (117 mmol, 1.0 eq) 2-Brom-9,9'-dimethylfluoren (CAS 28320-31-2) werden in 950 ml Toluol vorgelegt und 30 Minuten mit Argon gesättigt. Anschließend werden 1.0 g (1.8 mmol, 0.02 eq.) 1,1'-Bis(diphenylphosphino)-ferrocen (CAS 12150-46-8), 350 mg (1.6 mmol, 0.01 eq.) Palladium(II)-acetat (CAS 3375-31-3) und 29 g (300 mmol, 2.6 eq.) Natrium-*tert*-butylat (CAS 865-48-5) zugegeben und über Nacht unter Rückfluss erhitzt. Nach beendeter Reaktion wird der Ansatz mit 300 ml Toluol verdünnt und mit Wasser extrahiert. Die organische Phase wird über Natriumsulfat getrocknet und das Lösungsmittel am Rotationsverdampfer entfernt. Das braune Öl wird mit 50 ml Ethylacetat versetzt und in eine Mischung aus Heptan/Essigester 20:1 gegeben. Der entstandene Feststoff wird abgesaugt und mit Heptan gewaschen. Nach Trocknung werden 29 g (80 mmol, 69%) des Produkts mit einer HPLC-Reinheit von 99.1% erhalten.

Analog können folgende Verbindungen erhalten werden:

| | **Edukt 1** | **Edukt 2** | **Produkt** | **Ausbeute** |
|---|---|---|---|---|
| h1 | 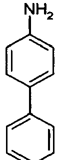 | 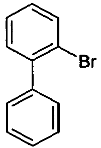 | 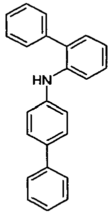 | 71% |
| | 92-67-1 | 2052-07-5 | | |
| h2 | 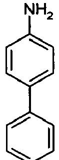 | 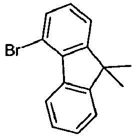 | 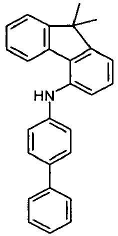 | 61% |
| | 92-67-1 | 942615-32-9 | | |
| h3 | 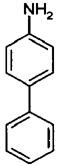 | 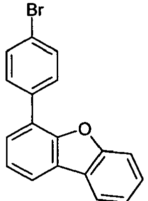 | 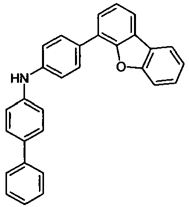 | 78% |
| | 92-67-1 | 955959-84-9 | | |
| h4 | 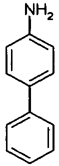 | 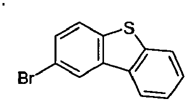 | 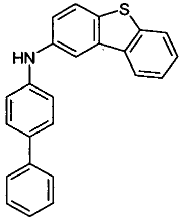 | 82% |
| | 92-67-1 | 22439-61-8 | | |
| h5 | 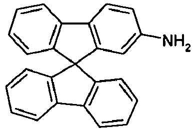 | 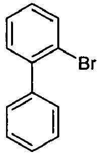 | 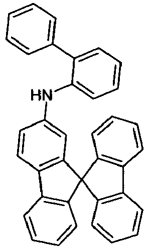 | 62% |
| | 118951-68-1 | 2052-07-5 | | |
| h6 | 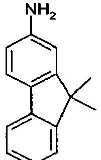 | 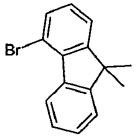 | 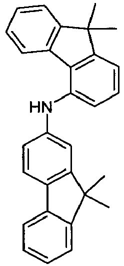 | 47% |
| | 108714-73-4 | 942615-32-9 | | |
| h7 | 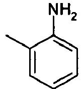 | 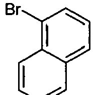 | 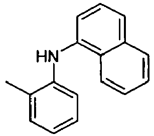 | 92% |
| | 95-53-4 | 90-11-9 | | |
| h8 | 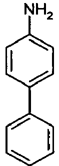 | 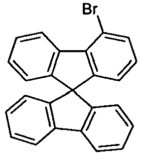 | 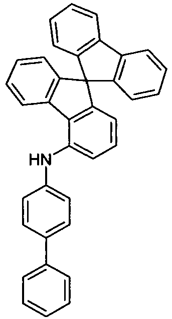 | 75% |
| | 92-67-1 | 171408-76-7 | | |
| h9 | 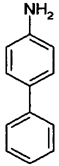 | 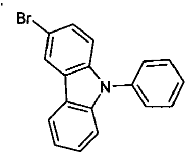 | 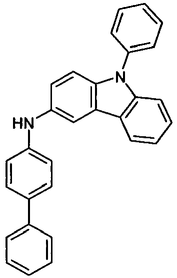 | 84% |
| | 92-67-1 | 1153-85-1 | | |
| h10 | 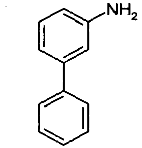 | 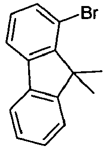 | 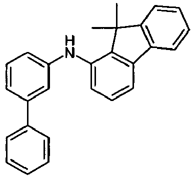 | 62% |
| | 90-41-5 | 1225053-54-2 | | |

### j) Bis-biphenyl-4-yl-(4-dibenzofuran-1-yl-6-phenyl-[1,3,5]triazin-2-yl)-amin

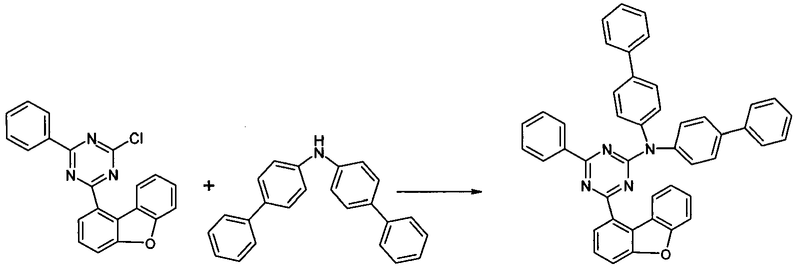

Eine entgaste Lösung von 49 g (140 mmol) 2-Chlor-4-dibenzofuran-1-yl-6-phenyl-[1,3,5]triazin und 43 g (140 mmol) Bis-biphenyl-4-yl-amin in 600 mL Toluol wird 1 h mit N₂ gesättigt. Danach wird die Lösung zuerst mit 2.09 mL (8.6 mmol) P(*t*Bu)₃, dann mit 1.38 g (6.1 mmol) Palladium(II)-acetat versetzt, und anschließend werden 17.7 g (185 mmol) NaOtBu im festen Zustand zugegeben. Die Reaktionsmischung wird 1 h unter Rückfluss erhitzt. Nach dem Abkühlen auf Raumtemperatur werden vorsichtig 500 mL Wasser zugesetzt. Die wässrige Phase wird mit 3 x 50 mL Toluol gewaschen, über MgSO₄ getrocknet und das Lösungsmittel im Vakuum entfernt. Danach wird das Rohprodukt über Kieselgel mit Heptan / Essigsäureester (20/1) chromatographisch gereinigt. Der Rückstand wird aus Toluol umkristallisiert und abschließend im Hochvakuum (p = 5 x 10⁻⁶ mbar) sublimiert. Die Ausbeute beträgt 60 g (94 mmol), entsprechend 69 % der Theorie.

Analog können folgende Verbindungen erhalten werden:

| | **Edukt 1** | **Edukt 2** | **Produkt** | **Ausbeute** |
|---|---|---|---|---|
| j1 | 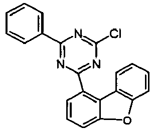 | 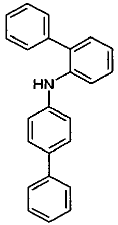 | 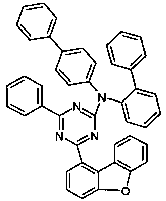 | 67% |
| j2 | 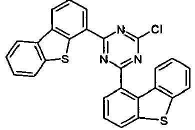 | 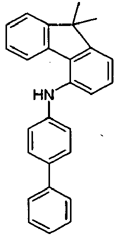 | 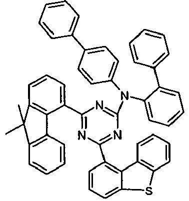 | 64% |
| j3 | 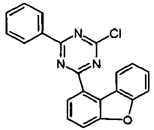 | 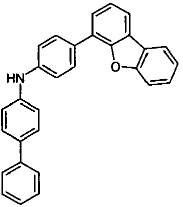 | 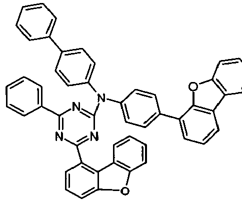 | 61% |
| j4 | 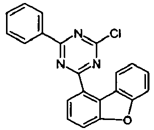 | 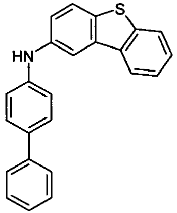 | 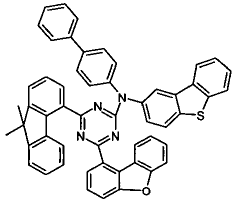 | 60% |
| j5 | 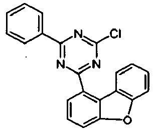 | 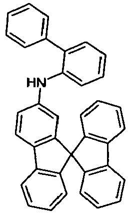 | 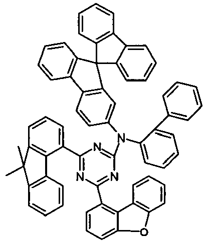 | 64% |
| j6 | 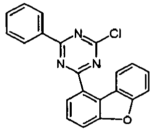 | 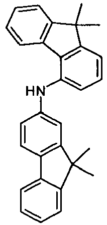 | 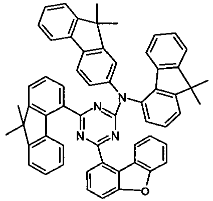 | 58% |
| j7 | 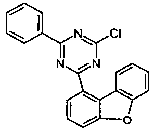 | 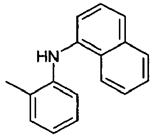 | 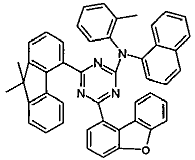 | 63% |
| j8 | 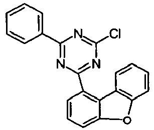 | 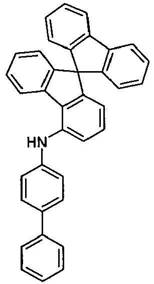 | 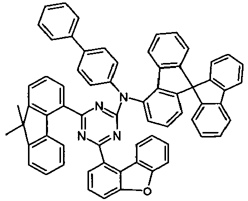 | 67% |
| j9 | 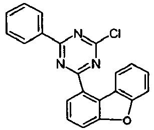 | 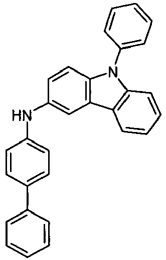 | 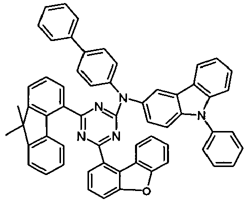 | 67% |
| j10 | 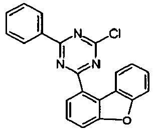 | 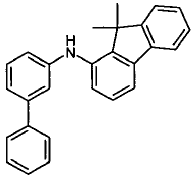 | 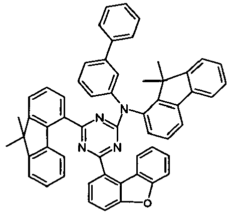 | 65% |

Analog zu Vorschrift (g) können folgende Verbindungen erhalten werden:

| | | | | |
|---|---|---|---|---|
| j11 | 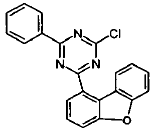 | 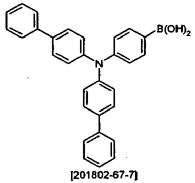 | 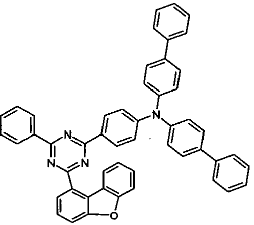 | 72% |
| j12 | 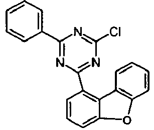 | 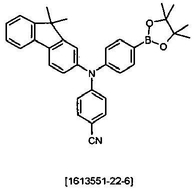 | 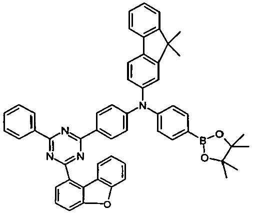 | 76% |
| j13 | 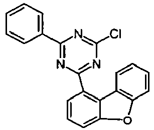 | 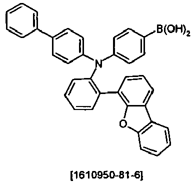 | 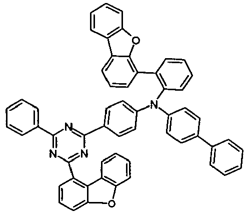 | 69% |
| j14 | 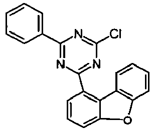 | 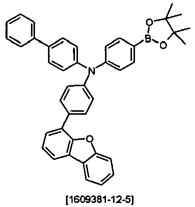 | 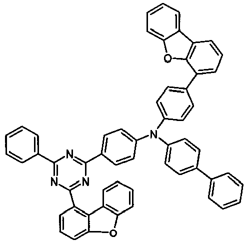 | 75% |
| j15 | 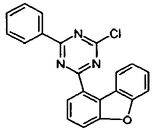 | 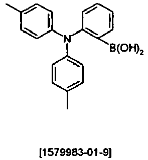 | 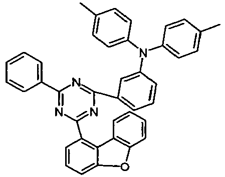 | 77% |
| j16 | 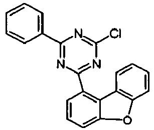 | 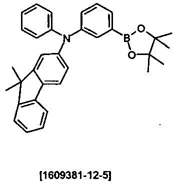 | 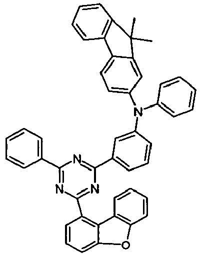 | 79% |

### k) 10-[3-(4-Dibenzofuran-1-yl-6-phenyl-[1,3,5]triazin-2-yl)-phenyl]-12,12-dimethyl-10,12-dihydro-10-aza-indeno[2,1-b]fluoren

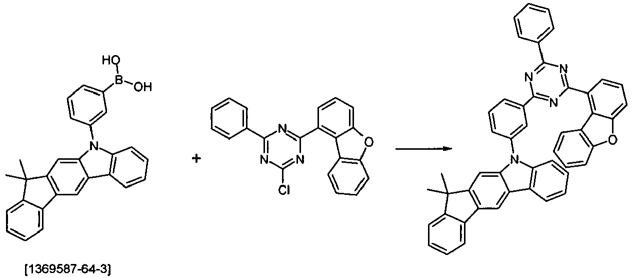

15.5 g (43.3 mmol) 2-Chlor-4-dibenzofuran-1-yl-6-phenyl-[1,3,5]triazin und 19.3 g (48 mmol) 3-(12,12-Dimethyl-12H-10-aza-indeno[2,1-b]fluoren-10-yl)-boronsäure werden in 80 mL Toluol gelöst und entgast. Es wird mit 281 mL einer entgasten 2M K₂CO₃-Lösung in Wasser und mit 2.5 g (2.2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert. Die Reinheit beträgt 99.9%. Ausbeute: 25.4 g (37 mmol), 78 % der Theorie.

Analog können folgende Verbindungen erhalten werden:

| | **Edukt 1** | **Edukt 2** | **Produkt** | **Ausbeute** |
|---|---|---|---|---|
| k1 | 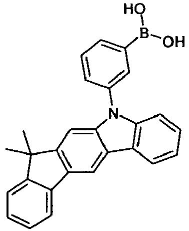 | 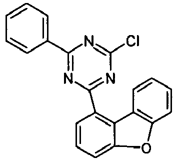 | 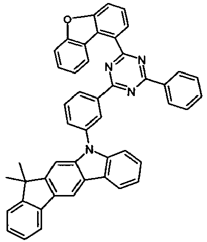 | 71% |
| | [1369587-64-3] | | | |
| k2 | 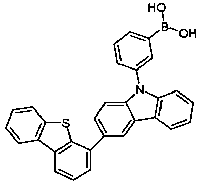 | 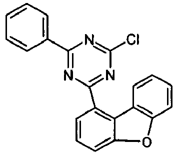 | 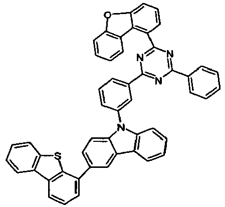 | 78% |
| | [1420067-45-3] | | | |
| k3 | 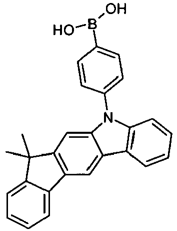 | 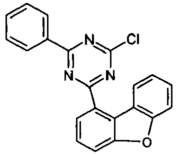 | 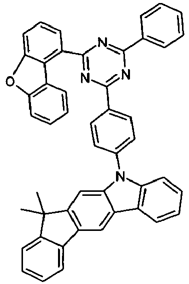 | 75% |
| | [1357066-50-2] | | | |
| k4 | 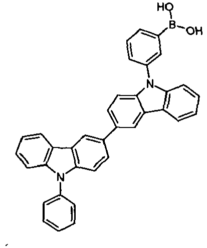 [1398394-64-3] | 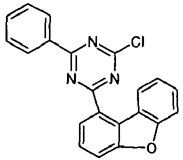 | 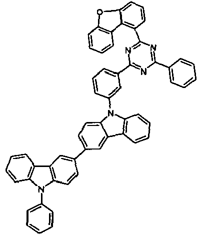 | 82% |
| k5 | 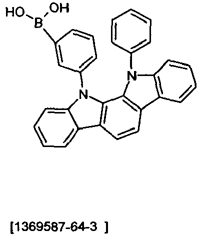 [1369587-64-3] | 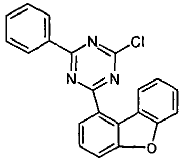 | 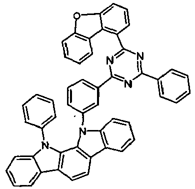 | 63% |
| k6 | 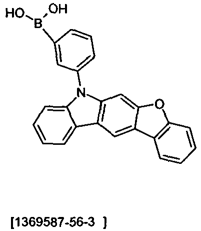 [1369587-56-3] | 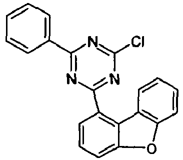 | 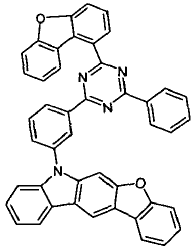 | 87% |
| k7 | 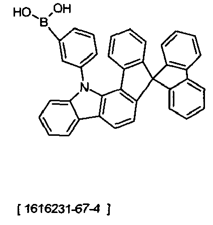 [1616231-67-4] | 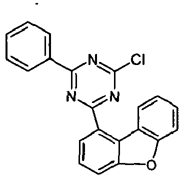 | 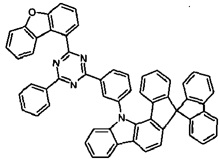 | 69% |
| k8 | 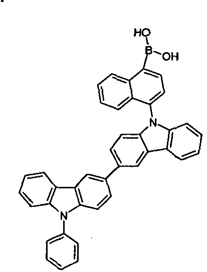 [1398395-43-1] | 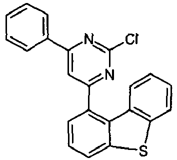 | 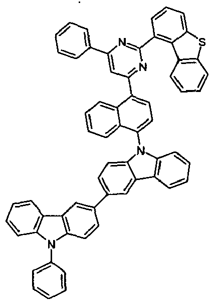 | 85% |

### Herstellung der OLEDs

In den folgenden Beispielen V1 bis E21 (siehe Tabellen 1 und 2) werden die Daten verschiedener OLEDs vorgestellt.

**Vorbehandlung für die Beispiele V1-E21:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen)poly(styrolsulfonat), bezogen als CLEVIOS™ P VP A1 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 70% in Tabelle 2 bedeutet, dass die in Spalte LD angegebene Lebensdauer der Zeit entspricht, nach der die Anfangsleuchtdichte von 4000 cd/m² auf 2800 cd/m² absinkt. Analog bedeutet L0;j0 = 20mA/cm², L1 = 80%, dass die Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiele V1-V4 sind Vergleichsbeispiele gemäß dem Stand der Technik, die Beispiele E1-E21 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben bei Einsatz als Matrixmaterialien in phosphoreszierenden OLEDs wesentliche Verbesserungen gegenüber dem Stand der Technik bezüglich der Lebensdauer der Bauteile. Durch Einsatz der erfindungsgemäßen Verbindungen EG1 bis EG5 in Kombination mit dem grün emittierenden Dotanden TEG1 lässt sich eine Steigerung der Lebensdauer um ca. 20% bis 40% gegenüber dem Stand der Technik beobachten (Vergleich der Beispiele V1 mit E1, E2 und V2 mit E3 sowie V3 mit E4 und V4 mit E5).

**Tabelle 1: Aufbau der OLEDs**

| Bsp | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | SdT1:TEG1 (90%:10%) 30nm | ST2 10nm | ST2:LiQ (50%:50%) 30nm | --- |
| V2 | SpA1 70nm | HATCN 5nm | SpMA1 90nm | SdT2:TEG1 (90%:10%) 30nm | ST2 10nm | ST2:LiQ (50%:50%) 30nm | --- |
| V3 | SpA1 70nm | HATCN 5nm | SpMA1 90nm | SdT3:TEG1 (90%:10%) 30nm | ST2 10nm | ST2:LiQ (50%:50%) 30nm | --- |
| V4 | SpA1 70nm | HATCN 5nm | SpMA1 90nm | SdT4:TEG1 (90%:10%) 30nm | ST2 10nm | ST2:LiQ (50%:50%) 30nm | --- |
| E1 | SpA1 70nm | HATCN 5nm | SpMA1 90nm | EG1:TEG1 (90%:10%) 30nm | ST2 10nm | ST2:LiQ (50%:50%) 30nm | --- |
| E2 | SpA1 70nm | HATCN 5nm | SpMA1 90nm | EG2:TEG1 (90%:10%) 30nm | ST2 10nm | ST2:LiQ (50%:50%) 30nm | --- |
| E3 | SpA1 70nm | HATCN 5nm | SpMA1 90nm | EG3:TEG1 (90%:10%) 30nm | ST2 10nm | ST2:LiQ (50%:50%) 30nm | --- |
| E4 | SpA1 70nm | HATCN 5nm | SpMA1 90nm | EG4:TEG1 (90%:10%) 30nm | ST2 10nm | ST2:LiQ (50%:50%) 30nm | --- |
| E5 | SpA1 70nm | HATCN 5nm | SpMA1 90nm | EG5:TEG1 (90%:10%) 30nm | ST2 10nm | ST2:LiQ (50%:50%) 30nm | --- |
| E6 | SpA1 70nm | HATCN 5nm | SpMA1 90nm | EG6:TEG1 (90%:10%) 30nm | ST2 10nm | ST2:LiQ (50%:50%) 30nm | --- |
| E7 | SpA1 70nm | HATCN 5nm | SpMA1 90nm | EG7:TEG1 (95%:5%) 30nm | ST2 10nm | ST2:LiQ (50%:50%) 30nm | --- |
| E8 | SpA1 70nm | HATCN 5nm | SpMA1 90nm | EG8:TEG1 (90%:10%) 30nm | ST2 10nm | ST2:LiQ (50%:50%) 30nm | --- |
| E9 | SpA1 70nm | HATCN 5nm | SpMA1 90nm | EG9:TEG1 (90%:10%) 30nm | ST2 10nm | ST2:LiQ (50%:50%) 30nm | --- |
| E10 | SpA1 70nm | HATCN 5nm | SpMA1 90nm | IC1:TEG1 (90%:10%) 30nm | --- | ST2:EG10 (50%:50%) 40nm | LiQ 3nm |
| E11 | SpA1 70nm | HATCN 5nm | SpMA1 90nm | EG11:TEG1 (90%:10%) 30nm | ST2 10nm | ST2:LiQ (50%:50%) 30nm | --- |
| E12 | SpA1 70nm | HATCN 5nm | SpMA1 90nm | IC1:TEG1 (90%:10%) 30nm | EG12 10nm | ST2:LiQ (50%:50%) 30nm | --- |
| E13 | SpA1 70nm | HATCN 5nm | SpMA1 90nm | EG13:TEG1 (90%:10%) 30nm | ST2 10nm | ST2:LiQ (50%:50%) 30nm | --- |
| E14 | SpA1 70nm | HATCN 5nm | SpMA1 90nm | EG14:TEG1 (90%:10%) 30nm | ST2 10nm | ST2:LiQ (50%:50%) 30nm | --- |
| E15 | SpA1 70nm | HATCN 5nm | SpMA1 90nm | EG15:TEG1 (90%:10%) 30nm | ST2 10nm | ST2:LiQ (50%:50%) 30nm | --- |
| E16 | SpA1 70nm | HATCN 5nm | SpMA1 90nm | EG16:IC4:TEG1 (45%:45%:10%) 30nm | IC1 10nm | ST2:LiQ (50%:50%) 30nm | --- |
| E17 | SpA1 70nm | HATCN 5nm | SpMA1 90nm | EG17:IC4:TEG1 (45%:45%:10%) 30nm | IC1 10nm | ST2:LiQ (50%:50%) 30nm | --- |
| E18 | SpA1 70nm | HATCN 5nm | SpMA1 90nm | EG18:IC4:TEG1 (45%:45%:10%) 30nm | IC1 10nm | ST2:LiQ (50%:50%) 30nm | --- |
| E19 | SpA1 70nm | HATCN 5nm | SpMA1 90nm | EG19:IC4:TEG1 (45%:45%:10%) 30nm | IC1 10nm | ST2:LiQ (50%:50%) 30nm | --- |
| E20 | SpA1 90nm | HATCN 5nm | SpMA1 130nm | EG20:TER3 (92%:8%) 30nm | --- | ST2:LiQ (50%:50%) 40nm | --- |
| E21 | SpA1 90nm | HATCN 5nm | SpMA1 130nm | EG21:TER3 (92%:8%) 30nm | --- | ST2:LiQ (50%:50%) 40nm | --- |

**Tabelle 2: Daten der OLEDs**

| Bsp. | U1000 (V) | SE1000 (cd/A) | LE1000 (lm/W) | EQE 1000 | CIE x/y bei 1000 cd/m² | L₀; j₀ | L1 % | LD (h) |
|---|---|---|---|---|---|---|---|---|
| V1 | 3.2 | 60 | 59 | 16.3% | 0.33/0.63 | 20 mA/cm² | 80 | 130 |
| V2 | 3.5 | 59 | 53 | 16.2% | 0.32/0.64 | 20 mA/cm² | 80 | 135 |
| V3 | 3.6 | 63 | 55 | 16.9% | 0.31/0.65 | 20 mA/cm² | 80 | 145 |
| V4 | 3.3 | 54 | 51 | 15.2% | 0.33/0.62 | 20 mA/cm² | 80 | 120 |
| E1 | 3.3 | 60 | 57 | 16.0% | 0.34/0.63 | 20 mA/cm² | 80 | 175 |
| E2 | 3.2 | 60 | 59 | 16.1% | 0.32/0.63 | 20 mA/cm² | 80 | 160 |
| E3 | 3.4 | 61 | 56 | 16.4% | 0.35/0.62 | 20 mA/cm² | 80 | 170 |
| E4 | 3.5 | 61 | 55 | 16.8% | 0.31/0.64 | 20 mA/cm² | 80 | 175 |
| E5 | 3.3 | 53 | 50 | 14.9% | 0.32/0.63 | 20 mA/cm² | 80 | 150 |
| E6 | 3.2 | 63 | 62 | 16.9% | 0.32/0.64 | 20 mA/cm² | 80 | 170 |
| E7 | 3.4 | 54 | 50 | 14.6% | 0.32/0.64 | 20 mA/cm² | 80 | 120 |
| E8 | 3.5 | 60 | 54 | 16.5% | 0.32/0.64 | 20 mA/cm² | 80 | 155 |
| E9 | 3.5 | 53 | 48 | 14.4% | 0.31/0.64 | 20 mA/cm² | 80 | 140 |
| E10 | 3.1 | 67 | 68 | 18.1% | 0.33/0.63 | 20 mA/cm² | 80 | 145 |
| E11 | 3.2 | 62 | 61 | 16.8% | 0.33/0.63 | 20 mA/cm² | 80 | 165 |
| E12 | 3.3 | 67 | 64 | 17.9% | 0.33/0.63 | 20 mA/cm² | 80 | 140 |
| E13 | 3.4 | 53 | 49 | 14.9% | 0.32/0.63 | 20 mA/cm² | 80 | 130 |
| E14 | 3.3 | 57 | 54 | 15.8% | 0.34/0.62 | 20 mA/cm² | 80 | 190 |
| E15 | 3.4 | 52 | 48 | 14.7% | 0.32/0.63 | 20 mA/cm² | 80 | 155 |
| E16 | 3.5 | 62 | 56 | 16.8% | 0.32/0.63 | 20 mA/cm² | 80 | 85 |
| E17 | 3.6 | 62 | 54 | 16.7% | 0.33/0.63 | 20 mA/cm² | 80 | 100 |
| E18 | 3.5 | 61 | 55 | 16.5% | 0.34/0.63 | 20 mA/cm² | 80 | 120 |
| E19 | 3.7 | 62 | 53 | 16.9% | 0.31/0.64 | 20 mA/cm² | 80 | 110 |
| E20 | 4.4 | 13 | 9 | 11.9% | 0.67/0.33 | 4000 cd/m² | 80 | 340 |
| E21 | 4.6 | 13 | 9 | 12.3% | 0.67/0.33 | 4000 cd/m² | 80 | 290 |

**Tabelle 3: Strukturformeln der Materialien für die OLEDs**

| | |
|---|---|
| 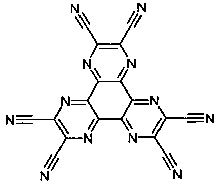 | 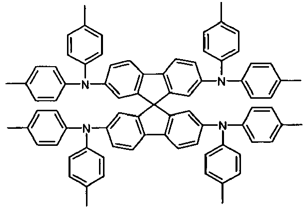 |
| HATCN | SpA1 |
| 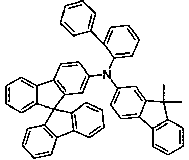 | 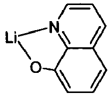 |
| SpMA1 | LiQ |
| 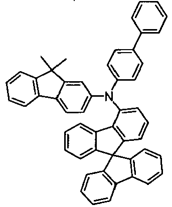 | 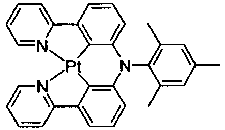 |
| SpMA2 | TER1 |
| 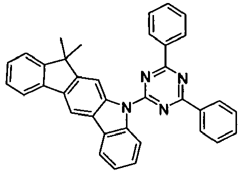 | 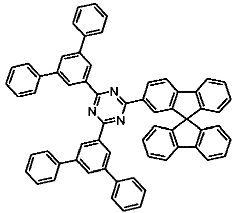 |
| IC1 | ST2 |
| 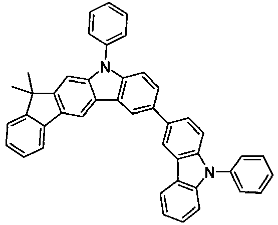 | 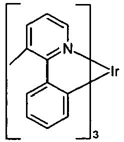 |
| IC3 | TEG1 |
| 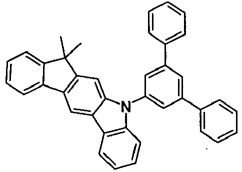 | |
| IC4 | |
| 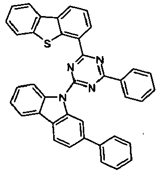 | 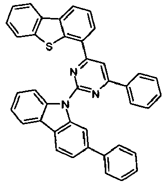 |
| SdT1 | SdT2 |
| 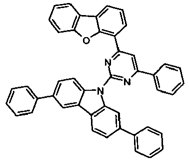 | 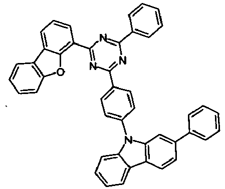 |
| SdT3 | SdT4 |
| 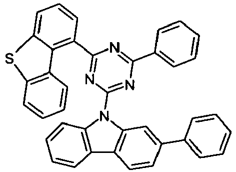 | 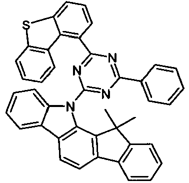 |
| EG1 | EG2 |
| 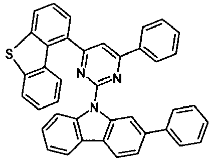 | 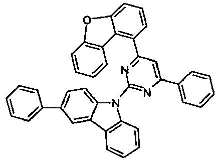 |
| EG3 | EG4 |
| 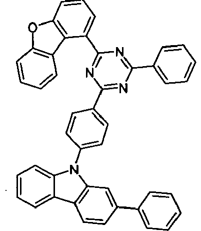 | 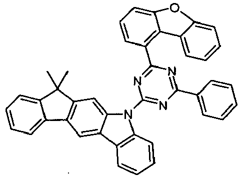 |
| EG5 | EG6 |
| 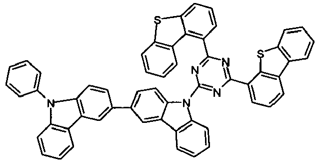 | 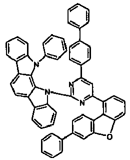 |
| EG7 | EG8 |
| 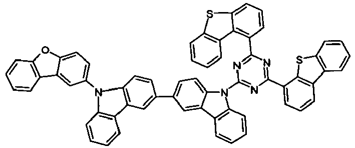 | 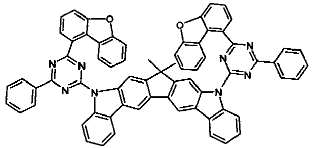 |
| EG9 | EG10 |
| 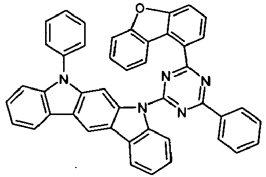 | 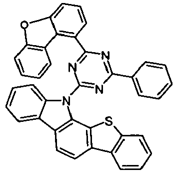 |
| EG11 | EG12 |
| 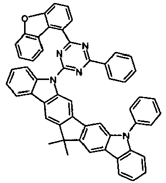 | 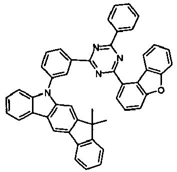 |
| EG13 | EG14 |
| 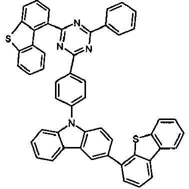 | 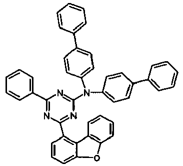 |
| EG15 | EG16 |
| 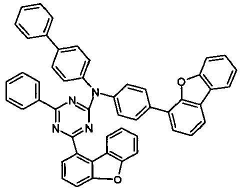 | 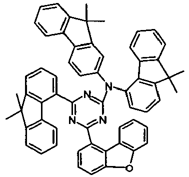 |
| EG17 | EG18 |
| 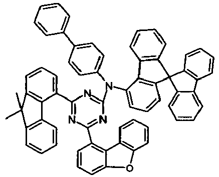 | 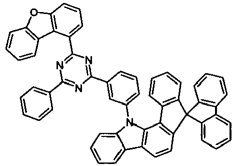 |
| EG19 | EG20 |
| 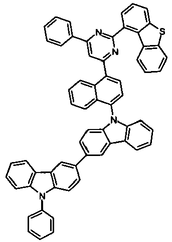 | |
| EG21 | |

## Patentansprüche

1. Verbindung gemäß Formel (1), 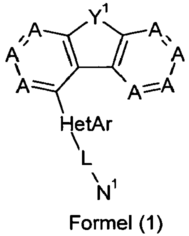 wobei für die verwendeten Symbole gilt:
A ist bei jedem Auftreten gleich oder verschieden CR¹ oder N, wobei maximal zwei Gruppen A pro Cyclus für N stehen;
Y¹ ist O oder S;
L ist bei jedem Auftreten gleich oder verschieden eine Einfachbindung oder ein aromatisches oder heteroaromatisches Ringsystem mit 5 bis 30 aromatischen Ringatomen, das durch einen oder mehrere Reste R¹ substituiert sein kann;
HetAr ist eine Gruppe der Formel (2) oder (3), 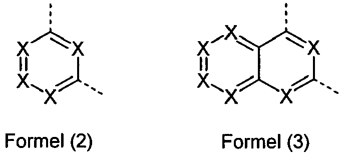 wobei die gestrichelten Bindungen die Verknüpfung dieser Gruppe mit dem Dibenzofuran- bzw. Dibenzothiophenderivat und mit L darstellen;
X ist bei jedem Auftreten gleich oder verschieden CR² oder N mit der Maßgabe, dass mindestens ein Symbol X für N steht;
N¹ ist eine Gruppe der folgenden Formel (4), (5) oder (6), 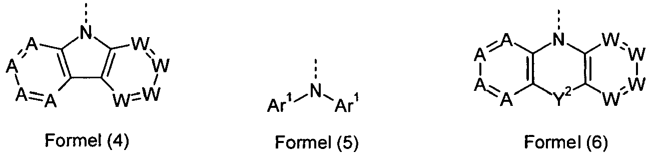 wobei die gestrichelte Bindung die Verknüpfung dieser Gruppe mit L darstellt;
Ar¹ ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann;
W ist bei jedem Auftreten gleich oder verschieden CR¹ oder N, wobei maximal zwei Gruppen W für N stehen, oder zwei benachbarte Gruppen W stehen zusammen für eine Gruppe der folgenden Formel (7) oder (8), wobei die Gruppe der Formel (4) bzw. (6) maximal eine Gruppe der Formel (7) oder (8) aufweist, und die verbleibenden Gruppen W stehen gleich oder verschieden bei jedem Auftreten für CR¹ oder N, 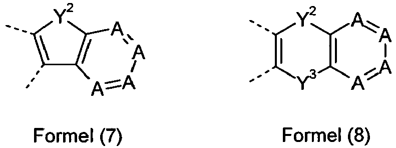 wobei die gestrichelten Bindungen die Verknüpfung dieser Gruppe andeuten und A die oben genannten Bedeutungen aufweist;
Y², Y³ ist gleich oder verschieden bei jedem Auftreten O, NR⁴, S, C(R⁴)₂, Si(R⁴)₂, BR⁴ oder C=O, wobei der Rest R⁴, der an N gebunden ist, ungleich H ist;
R¹, R², R³, R⁴ ist bei jedem Auftreten gleich oder verschieden ausgewählt aus der Gruppe bestehend aus H, D, F, Cl, Br, I, CN, NO₂, N(Ar²)₂, N(R⁵)₂, C(=O)Ar², C(=O)R⁵, P(=O)(Ar²)₂, P(Ar²)₂, B(Ar²)₂, Si(Ar²)₃, Si(R⁵)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, Si(R⁵)₂, C=O, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei benachbarte Substituenten R¹ bzw. zwei benachbarte Substituenten R³ bzw. zwei benachbarte Substituenten R⁴ miteinander ein aliphatisches, aromatisches oder heteroaromatisches Ringsystem bilden, das mit einem oder mehreren Resten R⁵ substituiert sein kann;
Ar² ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R⁵ substituiert sein kann; dabei können zwei Reste Ar², welche an dasselbe N-Atom, P-Atom oder B-Atom binden, auch durch eine Einfachbindung oder eine Brücke, ausgewählt aus N(R⁵), C(R⁵)₂, O oder S, miteinander verbrückt sein;
R⁵ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein aliphatisches Ringsystem bilden;
mit der Maßgabe, dass Y¹ für O steht, wenn die Gruppe N¹ für eine Gruppe der Formel (4) steht, die keine Gruppe der Formel (7) oder (8) enthält.

2. Verbindung nach Anspruch 1 gemäß Formel (1a), 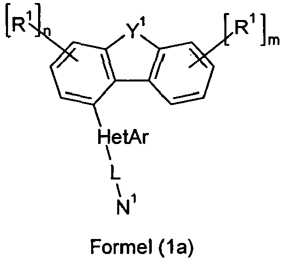 wobei die verwendeten Symbole die in Anspruch 1 genannten Bedeutungen aufweisen, n für 0, 1, 2 oder 3 und m für 0, 1, 2, 3 oder 4 steht.

3. Verbindung nach Anspruch 1 oder 2 gemäß einer der Formeln (1b) bis (1i), 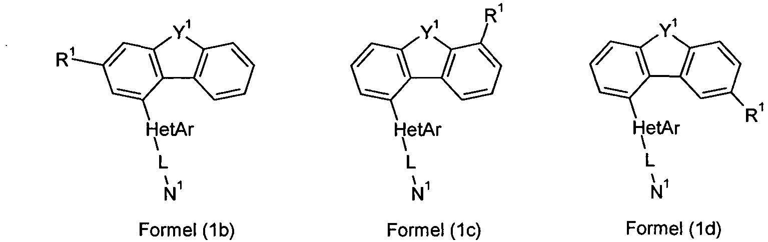 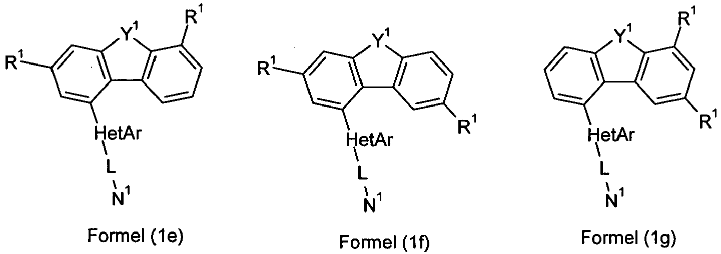 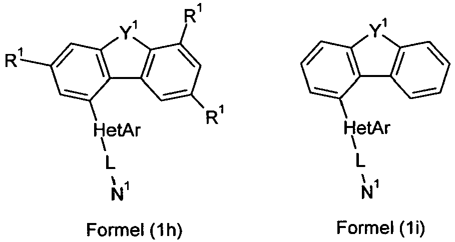 wobei die verwendeten Symbole die in Anspruch 1 genannten Bedeutungen aufweisen.

4. Verbindung nach einem oder mehreren der Ansprüche 1 bis 3, **dadurch gekennzeichnet, dass** die Gruppe der Formeln (2) und (3) ausgewählt ist aus den Gruppen der Formeln (2-1) bis (2-7) und (3-1), 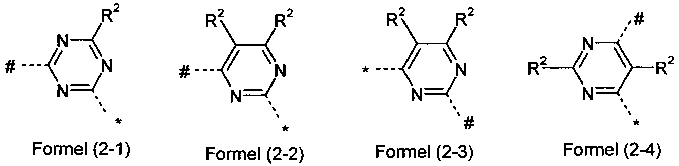 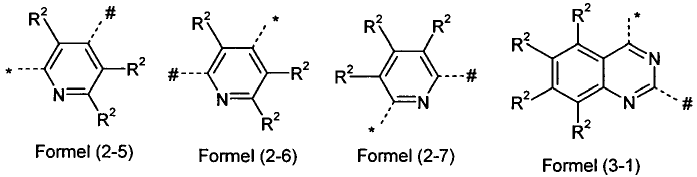 wobei die gestrichelte Bindung und * die Verknüpfung dieser Gruppen mit dem Dibenzofuran- bzw. Dibenzothiophenderivat in Formel (1) darstellt, die gestrichelte Bindung und # die Verknüpfung dieser Gruppen mit L bzw. für L gleich Einfachbindung mit N¹ darstellt und R² die in Anspruch 1 genannten Bedeutungen aufweist.

5. Verbindung nach einem oder mehreren der Ansprüche 1 bis 4, **dadurch gekennzeichnet, dass** die Gruppe der Formeln (2) und (3) ausgewählt ist aus den Gruppen der Formeln (2-1a) bis (3-1a), 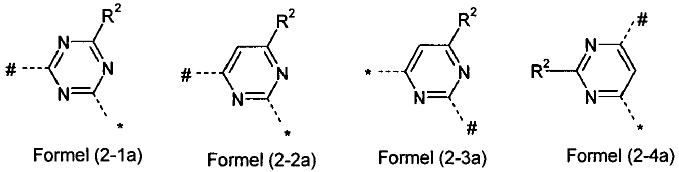 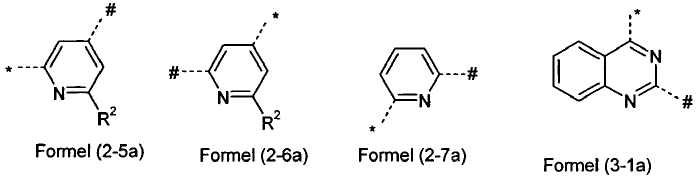 wobei die gestrichelte Bindung und * die Verknüpfung dieser Gruppen mit dem Dibenzofuran- bzw. Dibenzothiophenderivat in Formel (1) darstellt, die gestrichelte Bindung und # die Verknüpfung dieser Gruppen mit L bzw. für L gleich Einfachbindung mit N¹ darstellt und R² für H oder ein aromatisches oder heteroaromatisches Ringsystem mit 6 bis 24 aromatischen Ringatomen, das durch einen oder mehrere Reste R⁵ substituiert sein kann, steht.

6. Verbindung nach einem oder mehreren der Ansprüche 1 bis 5, **dadurch gekennzeichnet, dass** die Gruppen der Formeln (4) und (6) ausgewählt sind aus den Gruppen der Formeln (4-1), (4-2) und (6-1), 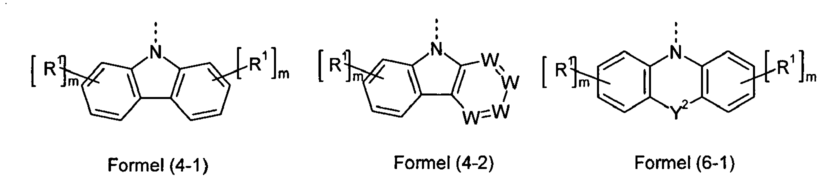 wobei R¹ und m die in Anspruch 1 und 2 genannten Bedeutungen aufweisen und weiterhin gilt:
zwei benachbarte Gruppen W stehen zusammen für eine Gruppe der Formel (7a) oder (8a) und die anderen beiden Gruppen W stehen für CR¹, 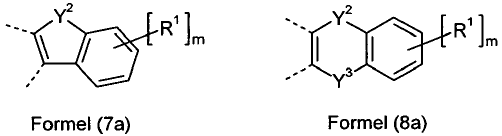 wobei Y², Y³, R¹ und m die in Anspruch 1 und 2 genannten Bedeutungen aufweisen.

7. Verbindung nach Anspruch 6, **dadurch gekennzeichnet, dass** die Gruppe der Formel (4-1) ausgewählt ist aus den Gruppen der Formeln (4-1a) bis (4-1f) und dass die Gruppe der Formel (4-2) ausgewählt ist aus den Gruppen der Formeln (4-2a) bis (4-2f), 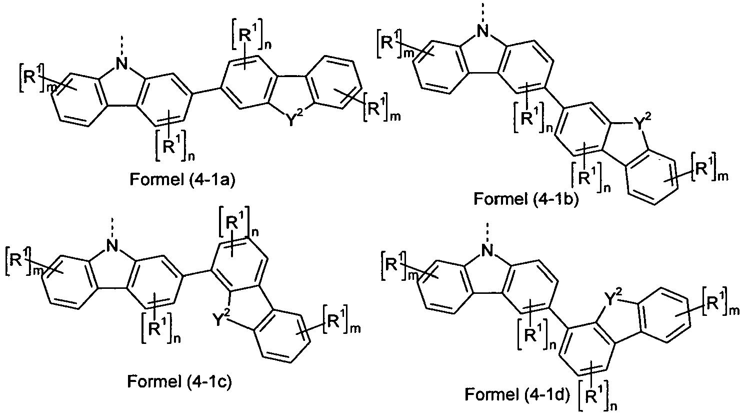 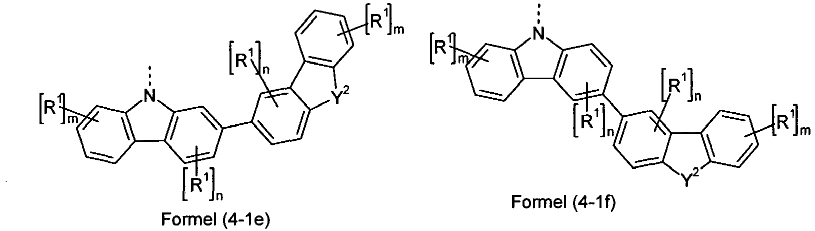 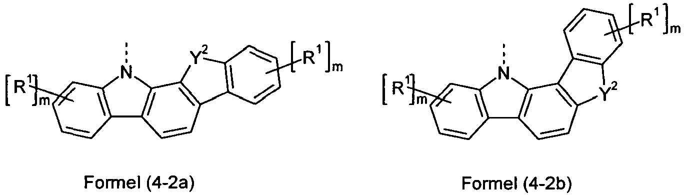 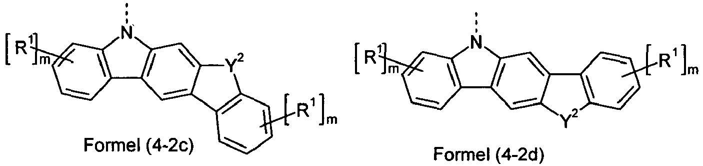 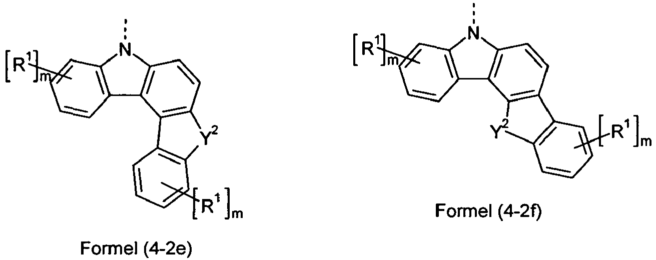 wobei die verwendeten Symbole und Indizes die in Anspruch 1 und 2 genannten Bedeutungen aufweisen.

8. Verbindung nach einem oder mehreren der Ansprüche 1 bis 5, **dadurch gekennzeichnet, dass** in der Gruppe der Formel (5) Ar¹ gleich oder verschieden bei jedem Auftreten für ein aromatisches oder heteroaromatisches Ringsystem mit 6 bis 24 aromatischen Ringatomen, das jeweils durch einen oder mehrere Reste R³ substituiert sein kann, steht.

9. Verbindung nach einem oder mehreren der Ansprüche 1 bis 8, **dadurch gekennzeichnet, dass** R¹ ausgewählt ist aus der Gruppe bestehend aus H, D, F, CN, N(Ar²)₂, C(=O)Ar², P(=O)(Ar²)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁵ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁵ substituiert sein kann.

10. Verbindung nach einem oder mehreren der Ansprüche 1 bis 9, für die gilt:
Y¹ ist O oder S;
L ist bei jedem Auftreten gleich oder verschieden eine Einfachbindung oder ein aromatisches oder heteroaromatisches Ringsystem mit 5 bis 24 aromatischen Ringatomen, das durch einen oder mehrere Reste R¹ substituiert sein kann;
HetAr ist eine Gruppe gemäß einer der Formeln (2-1) bis (2-7) oder (3-1) gemäß Anspruch 4;
N¹ ist eine Gruppe der Formel (4-1), (4-2), (5) oder (6-1), 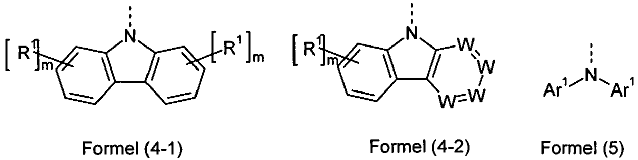 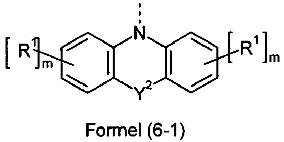 zwei benachbarte Gruppen W stehen zusammen für eine Gruppe der Formel (7a) oder (8a) und die anderen beiden Gruppen W stehen für CR¹, 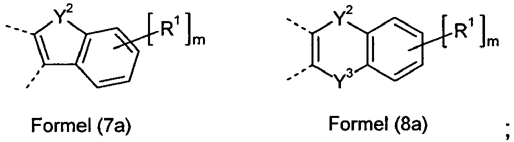
Ar¹ ist bei jedem Auftreten gleich oder verschieden ein aromatisches oder heteroaromatisches Ringsystem mit 6 bis 24 aromatischen Ringatomen, bevorzugt mit 6 bis 18 aromatischen Ringatomen, welches durch einen oder mehrere Reste R³ substituiert sein kann;
Y², Y³ ist gleich oder verschieden bei jedem Auftreten O, S, NR⁴ oder C(R⁴)₂, wobei der Rest R⁴, der an N gebunden ist, ungleich H ist;
R¹ ist bei jedem Auftreten gleich oder verschieden gewählt aus der Gruppe bestehend aus H, D, F, CN, N(Ar²)₂, C(=O)Ar², P(=O)(Ar²)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁵ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁵ substituiert sein kann;
R² ist bei jedem Auftreten gleich oder verschieden H oder ein aromatisches oder heteroaromatisches Ringsystem mit 6 bis 24 aromatischen Ringatomen, insbesondere mit 6 bis 18 aromatischen Ringatomen, das jeweils durch einen oder mehrere Reste R⁵ substituiert sein kann;
R³ ist bei jedem Auftreten gleich oder verschieden H, eine Alkylgruppe mit 1 bis 4 C-Atomen oder ein aromatisches oder heteroaromatisches Ringsystem mit 5 bis 14 aromatischen Ringatomen;
R⁴ ist für Y² bzw. Y³ = NR⁴ ein aromatisches oder heteroaromatisches Ringsystem mit 5 bis 24 aromatischen Ringatomen, das jeweils mit einem oder mehreren Resten R⁵ substituiert sein kann; und ist für Y² bzw. Y³ = C(R⁴)₂ bei jedem Auftreten gleich oder verschieden eine geradkettige Alkylgruppe mit 1 bis 10 C-Atomen oder eine verzweigte oder cyclische Alkylgruppe mit 3 bis 10 C-Atomen oder eine Alkenylgruppe mit 2 bis 10 C-Atomen stehen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, oder ein aromatisches oder heteroaromatisches Ringsystem mit 5 bis 24 aromatischen Ring-atomen, das jeweils mit einem oder mehreren Resten R⁵ substituiert sein kann; dabei können optional die beiden Substituenten R⁴ ein monocyclisches oder polycyclisches, aliphatisches, aromatisches oder heteroaromatisches Ringsystem bilden, das mit einem oder mehreren Resten R⁵ substituiert sein kann;
R⁵ ist bei jedem Auftreten gleich oder verschieden ausgewählt aus der Gruppe bestehend aus H, D, F, CN, einem aliphatischen Kohlenwasserstoffrest mit 1 bis 10 C-Atomen oder einem aromatischen oder heteroaromatischen Ringsystem mit 5 bis 30 aromatischen Ringatomen, das durch ein oder mehrere Alkylgruppen mit jeweils 1 bis 4 Kohlenstoffatomen substituiert sein kann;
die weiteren Symbole und Indizes haben die in den Ansprüchen 1 und 2 genannten Bedeutungen.

11. Verfahren zur Herstellung einer Verbindung nach einem oder mehreren der Ansprüche 1 bis 10, ausgehend von einem gegebenenfalls substituierten 1-Halogen-Dibenzofuran bzw. 1-Halogen-Dibenzothiophen, **gekennzeichnet durch** die folgenden Schritte:
a) optional Umsetzung der Halogengruppe zu einer Boronsäure bzw. einem Boronsäurederivat;
b) Einführung der Gruppe HetAr durch eine Kupplungsreaktion;
c) Einführung der Gruppe N¹ oder der Gruppe -L-N¹ durch eine Kupplungsreaktion.

12. Formulierung, enthaltend mindestens eine Verbindung nach einem oder mehreren der Ansprüche 1 bis 10 und mindestens eine weitere Verbindung und/oder ein Lösemittel.

13. Verwendung einer Verbindung nach einem oder mehreren der Ansprüche 1 bis 10 und/oder einer Formulierung nach Anspruch 12 in einer elektronischen Vorrichtung.

14. Elektronische Vorrichtung, bevorzugt ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und Organic Plasmon Emitting Devices, enthaltend mindestens eine Verbindung nach einem oder mehreren der Ansprüche 1 bis 10 oder eine Formulierung nach Anspruch 12.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 10 als Matrixmaterial für fluoreszierende oder phosphoreszierende Emitter in einer emittierenden Schicht und/oder in einer Elektronentransportschicht und/oder in einer elektronenblockierenden bzw. exzitonenblockierenden Schicht und/oder in einer Lochtransportschicht eingesetzt wird.

## Claims

1. Compound of the formula (1), 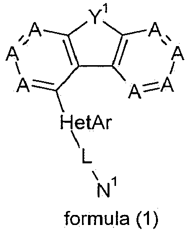 where the following applies to the symbols used:
A is on each occurrence, identically or differently, CR¹ or N, where a maximum of two groups A per ring stand for N;
Y¹ is O or S;
L is on each occurrence, identically or differently, a single bond or an aromatic or heteroaromatic ring system having 5 to 30 aromatic ring atoms, which may be substituted by one or more radicals R¹;
HetAr is a group of the formula (2) or (3), 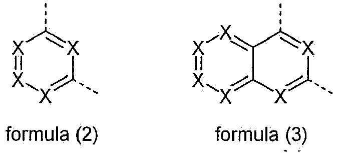 where the dashed bonds represent the linking of this group to the dibenzofuran or dibenzothiophene derivative and to L;
X is on each occurrence, identically or differently, CR² or N, with the proviso that at least one symbol X stands for N;
N¹ is a group of the following formula (4), (5) or (6), 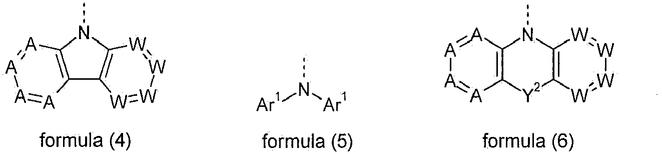 where the dashed bond represents the linking of this group to L;
Ar¹ is on each occurrence, identically or differently, an aromatic or heteroaromatic ring system having 5 to 30 aromatic ring atoms, which may be substituted by one or more radicals R³;
W is on each occurrence, identically or differently, CR¹ or N, where a maximum of two groups W stand for N, or two adjacent groups W together stand for a group of the following formula (7) or (8), where the group of the formula (4) or (6) contains a maximum of one group of the formula (7) or (8), and the remaining groups W stand, identically or differently on each occurrence, for CR¹ or N, 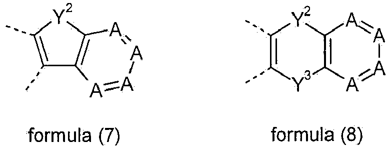 where the dashed bonds indicate the linking of this group, and A has the meanings given above;
Y², Y³ are, identically or differently on each occurrence, O, NR⁴, S, C(R⁴)₂, Si(R⁴)₂, BR⁴ or C=O, where the radical R⁴ which is bonded to N is not equal to H;
R¹, R², R³, R⁴ are selected on each occurrence, identically or differently, from the group consisting of H, D, F, CI, Br, I, CN, NO₂, N(Ar²)₂, N(R⁵)₂, C(=O)Ar², C(=O)R⁵, P(=O)(Ar²)₂, P(Ar²)₂, B(Ar²)₂, Si(Ar²)₃, Si(R⁵)₃, a straight-chain alkyl, alkoxy or thio-alkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals R⁵, where one or more non-adjacent CH₂ groups may be replaced by R⁵C=CR⁵, Si(R⁵)₂, C=O, C=NR⁵, P(=O)(R⁵), SO, SO₂, NR⁵, O, S or CONR⁵ and where one or more H atoms may be replaced by D, F, CI, Br, I, CN or NO₂, an aromatic or heteroaromatic ring system having 5 to 40 aromatic ring atoms, which may in each case be substituted by one or more radicals R⁵, an aryloxy or heteroaryloxy group having 5 to 40 aromatic ring atoms, which may be substituted by one or more radicals R⁵, or an aralkyl or heteroaralkyl group having 5 to 40 aromatic ring atoms, which may be substituted by one or more radicals R⁵; two adjacent substituents R¹ or two adjacent substituents R² or two adjacent substituents R³ here may optionally form an aliphatic, aromatic or heteroaromatic ring system with one another, which may be substituted by one or more radicals R⁵;
Ar² is on each occurrence, identically or differently, an aromatic or heteroaromatic ring system having 5 to 30 aromatic ring atoms, which may be substituted by one or more non-aromatic radicals R⁵; two radicals Ar² which are bonded to the same N atom, P atom or B atom here may also be bridged to one another by a single bond or a bridge selected from N(R⁵), C(R⁵)₂, O or S;
R⁵ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, CI, Br, I or CN and which may be substituted by one or more alkyl groups, each having 1 to 4 carbon atoms; two or more adjacent substituents R⁵ here may form an aliphatic ring system with one another; with the proviso that Y¹ stands for O if the group N¹ stands for a group of the formula (4) which does not contain a group of the formula (7) or (8).

2. Compound according to Claim 1 of the formula (1a), 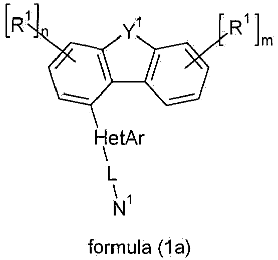 where the symbols used have the meanings given in Claim 1, n stands for 0, 1, 2 or 3 and m stands for 0, 1, 2, 3 or 4.

3. Compound according to Claim 1 or 2 of one of the formulae (1b) to (1i), 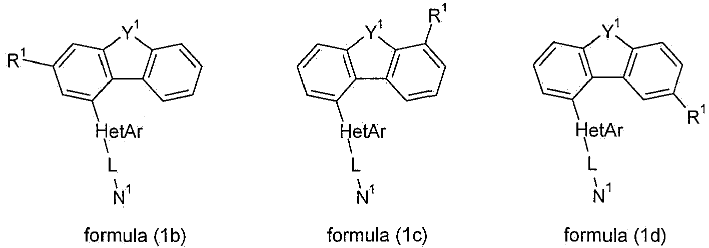 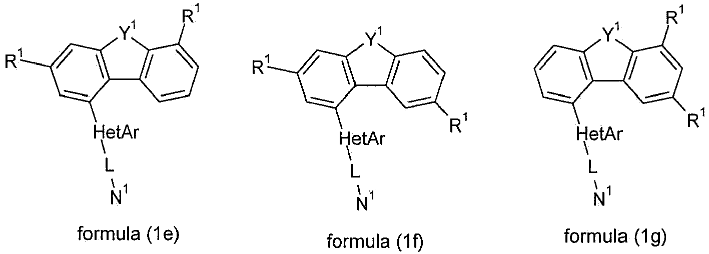 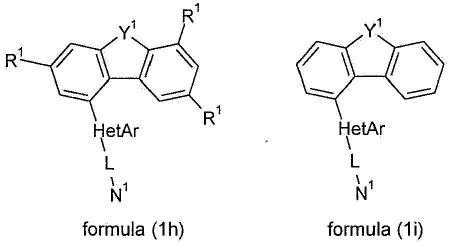 where the symbols used have the meanings given in Claim 1.

4. Compound according to one or more of Claims 1 to 3, **characterised in that** the groups of the formulae (2) and (3) are selected from the groups of the formulae (2-1) to (2-7) and (3-1), 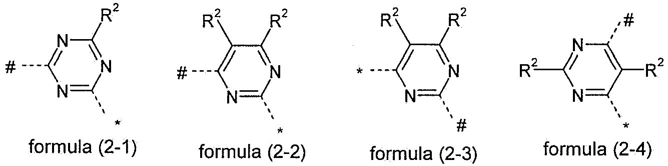 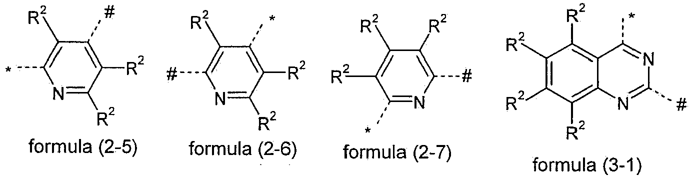 where the dashed bond and * represents the linking of these groups to the dibenzofuran or dibenzothiophene derivative in formula (1), the dashed bond and # represents the linking of these groups to L or, for L equal to a single bond, to N¹, and R² has the meanings given in Claim 1.

5. Compound according to one or more of Claims 1 to 4, **characterised in that** the groups of the formulae (2) and (3) are selected from the groups of the formulae (2-1a) to (3-1a), 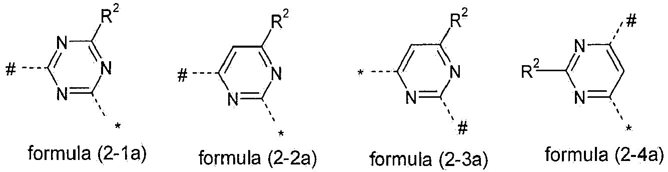 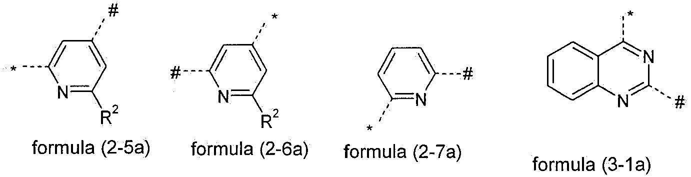 where the dashed bond and * represents the linking of these groups to the dibenzofuran or dibenzothiophene derivative in formula (1), the dashed bond and # represents the linking of these groups to L or, for L equal to a single bond, to N¹, and R² stands for H or an aromatic or heteroaromatic ring system having 6 to 24 aromatic ring atoms, which may be substituted by one or more radicals R⁵.

6. Compound according to one or more of Claims 1 to 5, **characterised in that** the groups of the formulae (4) and (6) are selected from the groups of the formulae (4-1), (4-2) and (6-1), 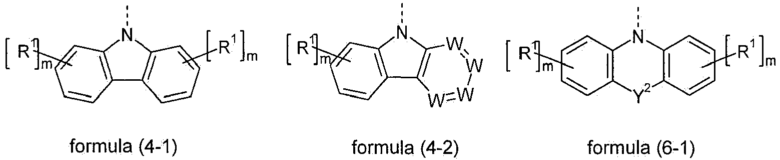 where R¹ and m have the meanings given in Claims 1 and 2, and furthermore:
two adjacent groups W together stand for a group of the formula (7a) or (8a) and the other two groups W stand for CR¹, 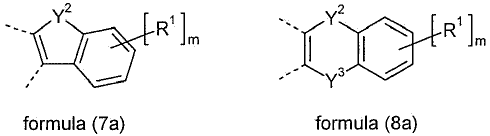 where Y², Y³, R¹ and m have the meanings given in Claims 1 and 2.

7. Compound according to Claim 6, **characterised in that** the group of the formula (4-1) is selected from the groups of the formulae (4-1a) to (4-1f) and **in that** the group of the formula (4-2) is selected from the groups of the formulae (4-2a) to (4-2f), 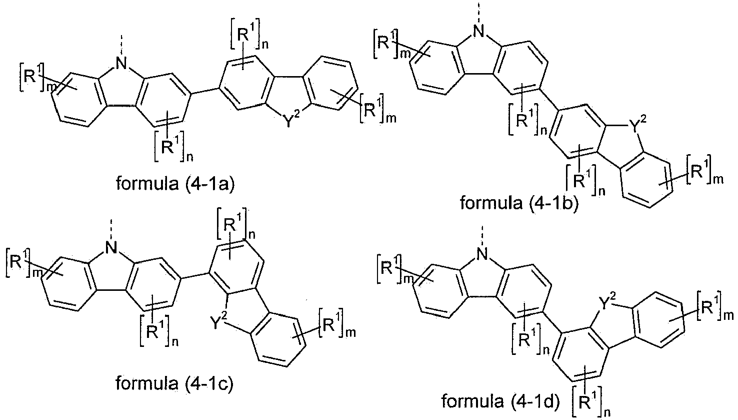 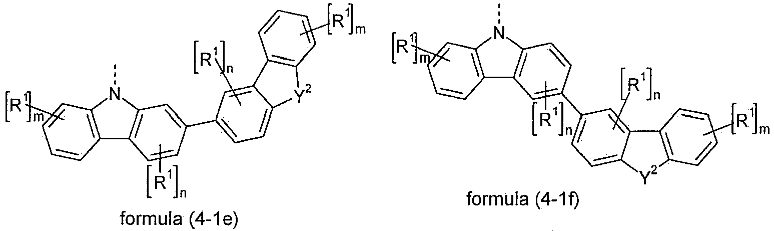 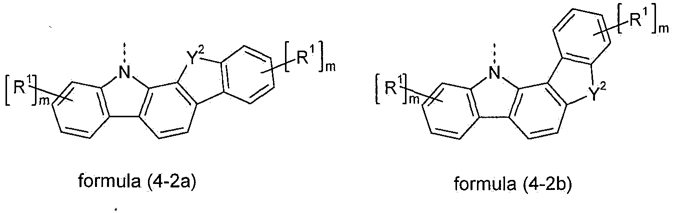 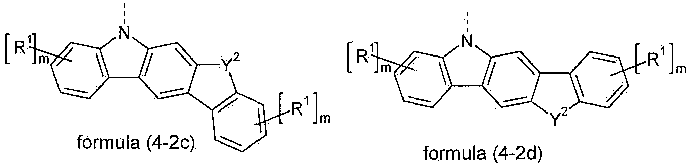 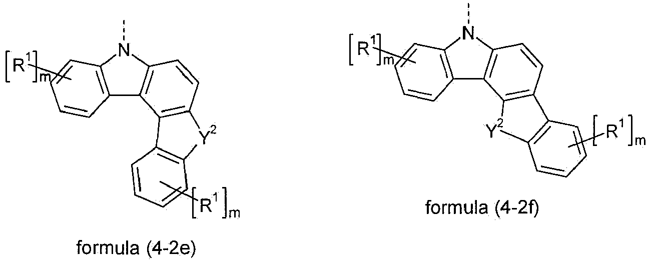 where the symbols and indices used have the meanings given in Claims 1 and 2.

8. Compound according to one or more of Claims 1 to 5, **characterised in that** Ar¹ in the group of the formula (5) stands, identically or differently on each occurrence, for an aromatic or heteroaromatic ring system having 6 to 24 aromatic ring atoms, which may in each case be substituted by one or more radicals R³.

9. Compound according to one or more of Claims 1 to 8, **characterised in that** R¹ is selected from the group consisting of H, D, F, CN, N(Ar²)₂, C(=O)Ar², P(=O)(Ar²)₂, a straight-chain alkyl or alkoxy group having 1 to 10 C atoms or a branched or cyclic alkyl or alkoxy group having 3 to 10 C atoms or an alkenyl group having 2 to 10 C atoms, which may in each case be substituted by one or more radicals R⁵, where one or more non-adjacent CH₂ groups may be replaced by O and where one or more H atoms may be replaced by D or F, an aromatic or heteroaromatic ring system having 5 to 24 aromatic ring atoms, which may in each case be substituted by one or more radicals R⁵, or an aralkyl or heteroaralkyl group having 5 to 25 aromatic ring atoms, which may be substituted by one or more radicals R¹; two substituents R¹ which are bonded to adjacent carbon atoms here may optionally form a monocyclic or polycyclic, aliphatic ring system, which may be substituted by one or more radicals R⁵.

10. Compound according to one or more of Claims 1 to 9 for which:
Y¹ is O or S;
L is on each occurrence, identically or differently, a single bond or an aromatic or heteroaromatic ring system having 5 to 24 aromatic ring atoms, which may be substituted by one or more radicals R¹;
HetAr is a group of one of the formulae (2-1) to (2-7) or (3-1) according to Claim 4;
N¹ is a group of the formula (4-1), (4-2), (5) or (6-1), 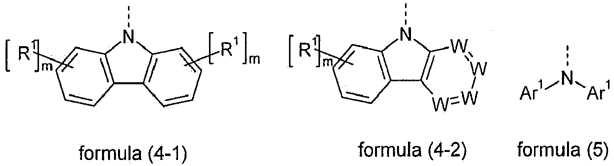 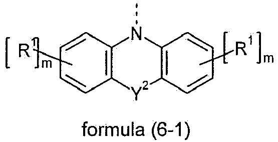 two adjacent groups W together stand for a group of the formula (7a) or (8a) and the other two groups W stand for CR¹, 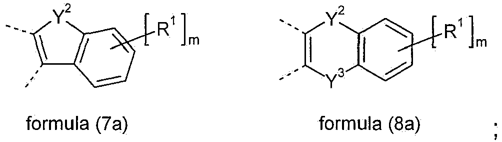
Ar¹ is on each occurrence, identically or differently, an aromatic or heteroaromatic ring system having 6 to 24 aromatic ring atoms, preferably having 6 to 18 aromatic ring atoms, which may be substituted by one or more radicals R³;
Y², Y³ are, identically or differently on each occurrence, O, S, NR⁴ or C(R⁴)₂, where the radical R⁴ which is bonded to N is not equal to H;
R¹ is selected on each occurrence, identically or differently, from the group consisting of H, D, F, CN, N(Ar²)₂, C(=O)Ar², P(=O)(Ar²)₂, a straight-chain alkyl or alkoxy group having 1 to 10 C atoms or a branched or cyclic alkyl or alkoxy group having 3 to 10 C atoms or an alkenyl group having 2 to 10 C atoms, which may in each case be substituted by one or more radicals R⁵, where one or more non-adjacent CH₂ groups may be replaced by O and where one or more H atoms may be replaced by D or F, an aromatic or hetero-aromatic ring system having 5 to 24 aromatic ring atoms, which may in each case be substituted by one or more radicals R⁵, or an aralkyl or heteroaralkyl group having 5 to 25 aromatic ring atoms, which may be substituted by one or more radicals R¹; two substituents R¹ which are bonded to adjacent carbon atoms here may optionally form a monocyclic or polycyclic, aliphatic ring system, which may be substituted by one or more radicals R⁵;
R² is on each occurrence, identically or differently, H or an aromatic or heteroaromatic ring system having 6 to 24 aromatic ring atoms, in particular having 6 to 18 aromatic ring atoms, which may in each case be substituted by one or more radicals R⁵;
R³ is on each occurrence, identically or differently, H, an alkyl group having 1 to 4 C atoms or an aromatic or heteroaromatic ring system having 5 to 14 aromatic ring atoms;
R⁴ is, for Y² or Y³ = NR⁴, an aromatic or heteroaromatic ring system having 5 to 24 aromatic ring atoms, which may in each case be substituted by one or more radicals R⁵; and is, for Y² or Y³ = C(R⁴)₂, on each occurrence, identically or differently, a straight-chain alkyl group having 1 to 10 C atoms or a branched or cyclic alkyl group having 3 to 10 C atoms or an alkenyl group having 2 to 10 C atoms, which may in each case be substituted by one or more radicals R⁵, where one or more non-adjacent CH₂ groups may be replaced by O and where one or more H atoms may be replaced by D or F, or an aromatic or heteroaromatic ring system having 5 to 24 aromatic ring atoms, which may in each case be substituted by one or more radicals R⁵; the two substituents R⁴ here may optionally form a monocyclic or polycyclic, aliphatic, aromatic or heteroaromatic ring system, which may be substituted by one or more radicals R⁵;
R⁵ is selected on each occurrence, identically or differently, from the group consisting of H, D, F, CN, an aliphatic hydrocarbon radical having 1 to 10 C atoms or an aromatic or heteroaromatic ring system having 5 to 30 aromatic ring atoms, which may be substituted by one or more alkyl groups, each having 1 to 4 carbon atoms;
the other symbols and indices have the meanings given in Claims 1 and 2.

11. Process for the preparation of a compound according to one or more of Claims 1 to 10, starting from an optionally substituted 1-halo-dibenzofuran or 1-halodibenzothiophene, **characterised by** the following steps:
a) optionally conversion of the halogen group into a boronic acid or a boronic acid derivative;
b) introduction of the group HetAr by a coupling reaction;
c) introduction of the group N¹ or the group -L-N¹ by a coupling reaction.

12. Formulation comprising at least one compound according to one or more of Claims 1 to 10 and at least one further compound and/or a solvent.

13. Use of a compound according to one or more of Claims 1 to 10 and/or a formulation according to Claim 12 in an electronic device.

14. Electronic device, preferably selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and organic plasmon emitting devices, comprising at least one compound according to one or more of Claims 1 to 10 or a formulation according to Claim 12.

15. Electronic device according to Claim 14, which is an organic electroluminescent device, **characterised in that** the compound according to one or more of Claims 1 to 10 is employed as matrix material for fluorescent or phosphorescent emitters in an emitting layer and/or in an electron-transport layer and/or in an electron-blocking or exciton-blocking layer and/or in a hole-transport layer.

## Revendications

1. Composé de la formule (1) : 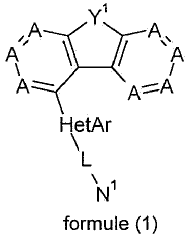 dans laquelle ce qui suit s'applique aux symboles qui sont utilisés :
A est pour chaque occurrence, de manière identique ou différente, CR¹ ou N, où un maximum de deux groupes A par cycle représentent N ;
Y¹ est O ou S ;
L est pour chaque occurrence, de manière identique ou différente, une liaison simple ou un système de cycle aromatique ou hétéroaromatique qui comporte de 5 à 30 atomes de cycle aromatique, lequel peut être substitué par un radical ou par plusieurs radicaux R¹ ;
HetAr est un groupe de la formule (2) ou (3) : 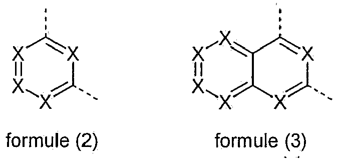 dans lesquelles les liens en pointillés représentent la liaison de ce groupe sur le dérivé de dibenzofurane ou de dibenzothiophène et sur L ;
X est pour chaque occurrence, de manière identique ou différente, CR² ou N, étant entendu qu'au moins un symbole X représente N;
N¹ est un groupe de la formule (4), (5) ou (6) qui suit : 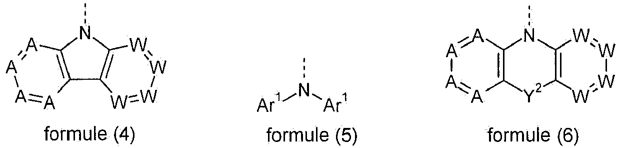 dans lesquelles le lien en pointillés représente la liaison de ce groupe sur L ;
Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R³ ;
W est pour chaque occurrence, de manière identique ou différente, CR¹ ou N, où un maximum de deux groupes W représentent N, ou deux groupes W adjacents représentent en association un groupe de la formule (7) ou (8) qui suit, où le groupe de la formule (4) ou (6) contient un maximum d'un seul groupe de la formule (7) ou (8), et les groupes W restants représentent, de manière identique ou différente pour chaque occurrence, CR¹ ou N ; 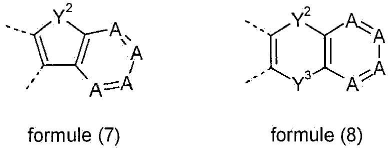 dans lesquelles les liens en pointillés indiquent la liaison de ce groupe, et A présente la signification qui a été donnée ci-avant ;
Y², Y³ sont, de manière identique ou différente pour chaque occurrence, O, NR⁴, S, C(R⁴)₂, Si(R⁴)₂, BR⁴ ou C=O, où le radical R⁴ qui est lié à N n'est pas égal à H ;
R¹, R², R³, R⁴ sont sélectionnés pour chaque occurrence, de manière identique ou différente, parmi le groupe qui est constitué par H, D, F, Cl, Br, I, CN, NO₂, N(Ar²)₂, N(R⁵)₂, C(=O)Ar², C(=O)R⁵, P(=O)(Ar²)₂, P(Ar²)₂, B(Ar²)₂, Si(Ar²)₃, Si(R⁵)₃, un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle qui comporte de 2 à 20 atomes de C, lequel peut dans chaque cas être substitué par un radical ou par plusieurs radicaux R⁵, où un ou plusieurs groupe(s) CH₂ non adjacents peut/ peuvent être remplacé(s) par R⁵C=CR⁵, Si(R⁵)₂, C=O, C=NR⁵, P(=O)(R⁵), SO, SO₂, NR⁵, O, S ou CONR⁵ et où un ou plusieurs atome(s) de H peut/peuvent être remplacé(s) par D, F, Cl, Br, I, CN ou NO₂, un système de cycle aromatique ou hétéroaromatique qui comporte de 5 à 40 atomes de cycle aromatique, lequel peut dans chaque cas être substitué par un radical ou par plusieurs radicaux R⁵, un groupe aryloxy ou hétéroaryloxy qui comporte de 5 à 40 atomes de cycle aromatique, lequel peut être substitué par un radical ou par plusieurs radicaux R⁵, où un groupe aralkyle ou hétéroaralkyle qui comporte de 5 à 40 atomes de cycle aromatique, lequel peut être substitué par un radical ou par plusieurs radicaux R⁵ ; deux substituants R¹ adjacents ou deux substituants R² adjacents ou deux substituants R³ adjacents peuvent ici en option former un système de cycle aliphatique, aromatique ou hétéroaromatique l'un avec l'autre, lequel peut être substitué par un radical ou par plusieurs radicaux R⁵ ;
Ar² est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R⁵ non aromatiques ; deux radicaux Ar² qui sont liés au même atome de N, au même atome de P ou au même atome de B peuvent ici également être pontés l'un à l'autre au moyen d'une liaison simple ou d'un pont qui est sélectionné parmi N(R⁵), C(R⁵)₂, O ou S ;
R⁵ est sélectionné pour chaque occurrence, de manière identique ou différente, parmi le groupe qui est constitué par H, D, F, CN, un radical hydrocarbone aliphatique qui comporte de 1 à 20 atome(s) de C ou un système de cycle aromatique ou hétéroaromatique qui comporte de 5 à 30 atomes de cycle aromatique, dans lequel un ou plusieurs atome(s) de H peut/peuvent être remplacé(s) par D, F, Cl, Br, I ou CN et lequel peut être substitué par un ou plusieurs groupe(s) alkyle, chacun comportant de 1 à 4 atome(s) de carbone ; deux substituants R⁵ adjacents ou plus peuvent ici former un système de cycle aliphatique l'un avec l'autre ou les uns avec les autres ;
étant entendu que Y¹ représente O si le groupe N¹ représente un groupe de la formule (4) qui ne contient pas un groupe de la formule (7) ou (8).

2. Composé selon la revendication 1 de la formule (1a) : 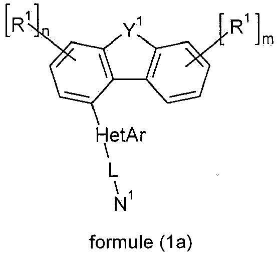 dans laquelle les symboles qui sont utilisés présentent les significations qui ont été données selon la revendication 1, n représente 0, 1, 2 ou 3 et m représente 0, 1, 2, 3 ou 4.

3. Composé selon la revendication 1 ou 2 de l'une des formules (1b) à (1i) : 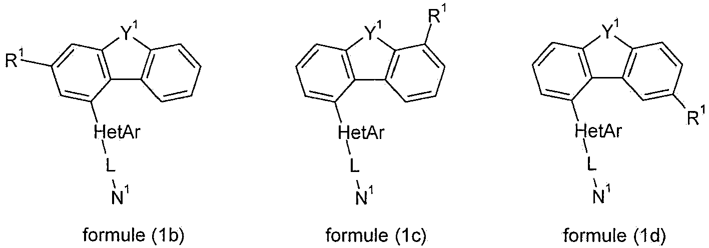 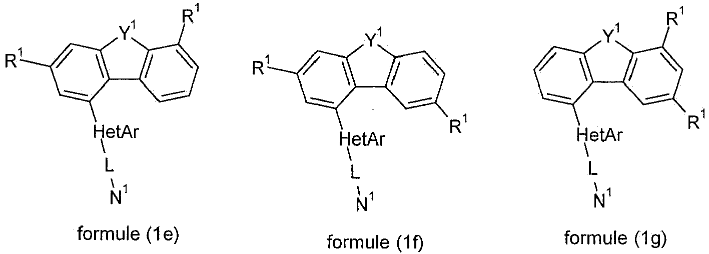 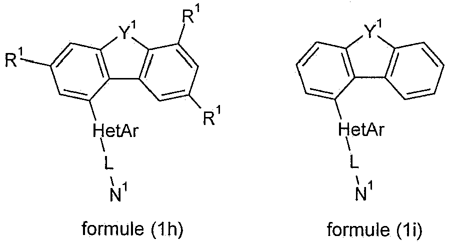 dans lesquelles les symboles qui sont utilisés présentent les significations qui ont été données selon la revendication 1.

4. Composé selon une ou plusieurs des revendications 1 à 3, **caractérisé en ce que** les groupes des formules (2) et (3) sont sélectionnés parmi les groupes des formules (2-1) à (2-7) et (3-1) : 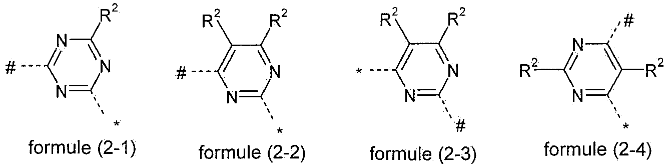 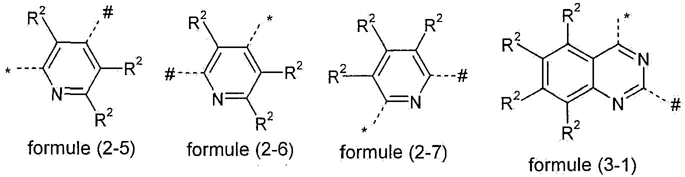 dans lesquelles le lien en pointillés et * représente la liaison de ces groupes sur le dérivé de dibenzofurane ou de dibenzothiophène dans la formule (1), le lien en pointillés et # représente la liaison de ces groupes sur L ou, pour L égal à une liaison simple, sur N¹, et R² présente les significations qui ont été données selon la revendication 1.

5. Composé selon une ou plusieurs des revendications 1 à 4, **caractérisé en ce que** les groupes des formules (2) et (3) sont sélectionnés parmi les groupes des formules (2-1a) à (3-1a) : 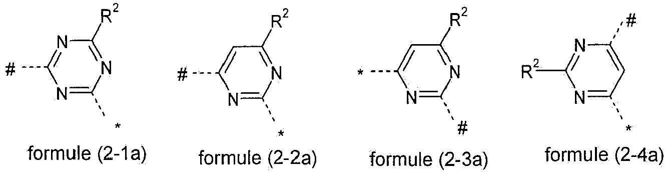 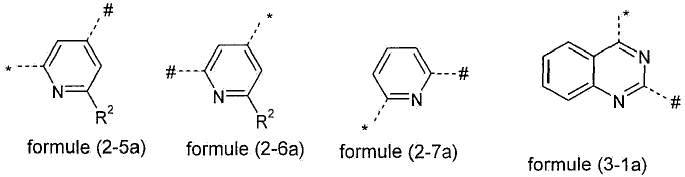 dans lesquelles le lien en pointillés et * représente la liaison de ces groupes sur le dérivé de dibenzofurane ou de dibenzothiophène selon la formule (1), le lien en pointillés et # représente la liaison de ces groupes sur L ou, pour L égal à une liaison simple, sur N¹, et R² représente H ou un système de cycle aromatique ou hétéroaromatique qui comporte de 6 à 24 atomes de cycle aromatique, lequel peut être substitué par un radical ou par plusieurs radicaux R⁵.

6. Composé selon une ou plusieurs des revendications 1 à 5, **caractérisé en ce que** les groupes des formules (4) et (6) sont sélectionnés parmi les groupes des formules (4-1), (4-2) et (6-1) : 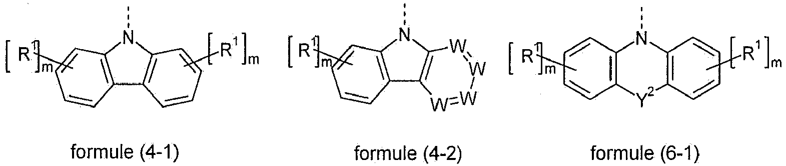 dans lesquelles R¹ et m présentent les significations qui ont été données selon les revendications 1 et 2, et en outre :
deux groupes W adjacents représentent en association un groupe de la formule (7a) ou (8a) et les deux autres groupes W représentent CR¹ ; 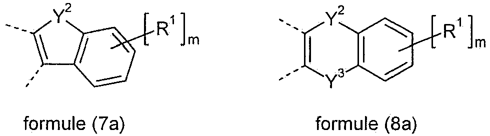 dans lesquelles Y², Y³, R¹ et m présentent les significations qui ont été données selon les revendications 1 et 2.

7. Composé selon la revendication 6, **caractérisé en ce que** le groupe de la formule (4-1) est sélectionné parmi les groupes des formules (4-1a) à (4-1f) et **en ce que** le groupe de la formule (4-2) est sélectionné parmi les groupes des formules (4-2a) à (4-2f) : 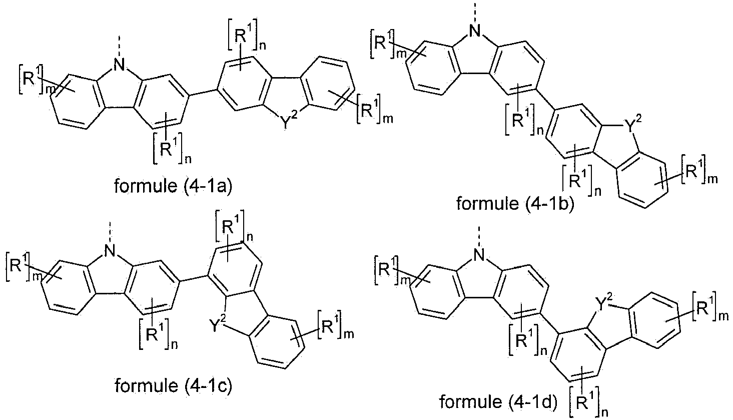 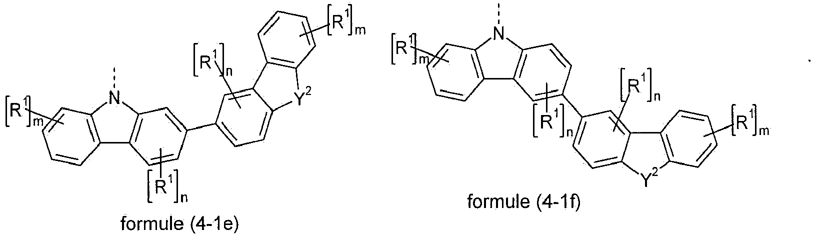 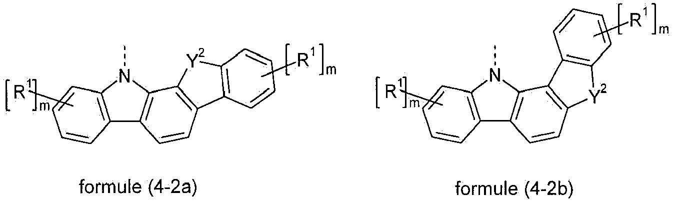 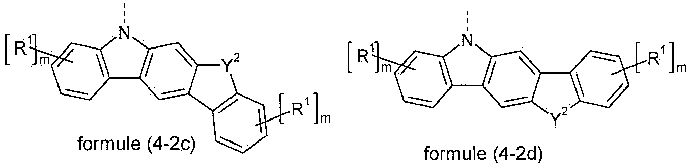 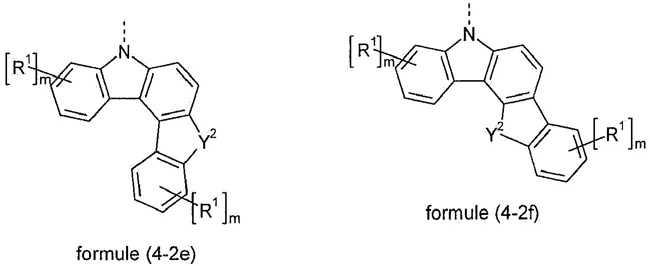 dans lesquelles les symboles et les indices qui sont utilisés présentent les significations qui ont été données selon les revendications 1 et 2.

8. Composé selon une ou plusieurs des revendications 1 à 5, **caractérisé en ce que** Ar¹ dans le groupe de la formule (5) représente, de manière identique ou différente pour chaque occurrence, un système de cycle aromatique ou hétéroaromatique qui comporte de 6 à 24 atomes de cycle aromatique, lequel peut dans chaque cas être substitué par un radical ou par plusieurs radicaux R³.

9. Composé selon une ou plusieurs des revendications 1 à 8, **caractérisé en ce que** R¹ est sélectionné parmi le groupe qui est constitué par H, D, F, CN, N(Ar²)₂, C(=O)Ar², P(=O)(Ar²)₂, un groupe alkyle ou alcoxy en chaîne droite qui comporte de 1 à 10 atome(s) de C ou un groupe alkyle ou alcoxy ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R⁵, où un ou plusieurs groupe(s) CH₂ non adjacents peut/ peuvent être remplacé(s) par O et où un ou plusieurs atome(s) de H peut/peuvent être remplacé(s) par D ou F, un système de cycle aromatique ou hétéroaromatique qui comporte de 5 à 24 atomes de cycle aromatique, lequel peut dans chaque cas être substitué par un radical ou par plusieurs radicaux R⁵, ou un groupe aralkyle ou hétéroaralkyle qui comporte de 5 à 25 atomes de cycle aromatique, lequel peut être substitué par un radical ou par plusieurs radicaux R¹ ; deux substituants R¹ qui sont liés à des atomes de carbone adjacents peuvent ici en option former un système de cycle aliphatique mono- ou polycyclique, lequel peut être substitué par un radical ou par plusieurs radicaux R⁵.

10. Composé selon une ou plusieurs des revendications 1 à 9 pour lequel :
Y¹ est O ou S ;
L est pour chaque occurrence, de manière identique ou différente, une liaison simple ou un système de cycle aromatique ou hétéroaromatique qui comporte de 5 à 24 atomes de cycle aromatique, lequel peut être substitué par un radical ou par plusieurs radicaux R¹ ;
HetAr est un groupe de l'une des formules (2-1) à (2-7) ou (3-1) selon la revendication 4 ;
N¹ est un groupe de la formule (4-1), (4-2), (5) ou (6-1) : 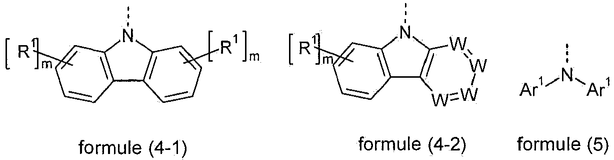 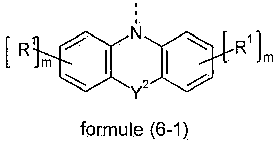 deux groupes W adjacents représentent en association un groupe de la formule (7a) ou (8a) et les deux autres groupes W représentent CR¹ ; 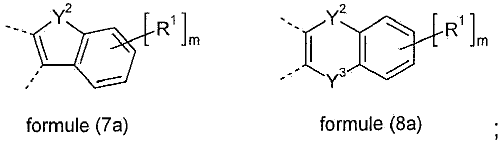
Ar¹ est pour chaque occurrence, de manière identique ou différente, un système de cycle aromatique ou hétéroaromatique qui comporte de 6 à 24 atomes de cycle aromatique, de préférence qui comporte de 6 à 18 atomes de cycle aromatique, lequel peut être substitué par un radical ou par plusieurs radicaux R³ ;
Y², Y³ sont, de manière identique ou différente pour chaque occurrence, O, S, NR⁴ ou C(R⁴)₂, où le radical R⁴ qui est lié à N n'est pas égal à H ;
R¹ est sélectionné pour chaque occurrence, de manière identique ou différente, parmi le groupe qui est constitué par H, D, F, CN, N(Ar²)₂, C(=O)Ar², P(=O)(Ar²)₂, un groupe alkyle ou alcoxy en chaîne droite qui comporte de 1 à 10 atome(s) de C ou un groupe alkyle ou alcoxy ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R⁵, où un ou plusieurs groupe(s) CH₂ non adjacents peut/peuvent être remplacé(s) par O et où un ou plusieurs atome(s) de H peut/peuvent être remplacé(s) par D ou F, un système de cycle aromatique ou hétéroaromatique qui comporte de 5 à 24 atomes de cycle aromatique, lequel peut dans chaque cas être substitué par un radical ou par plusieurs radicaux R⁵, où un groupe aralkyle ou hétéroaralkyle qui comporte de 5 à 25 atomes de cycle aromatique, lequel peut être substitué par un radical ou par plusieurs radicaux R¹ ; deux substituants R¹ qui sont liés à des atomes de carbone adjacents peuvent ici, en option, former un système de cycle aliphatique mono- ou polycyclique, lequel peut être substitué par un radical ou par plusieurs radicaux R⁵ ;
R² est pour chaque occurrence, de manière identique ou différente, H ou un système de cycle aromatique ou hétéroaromatique qui comporte de 6 à 24 atomes de cycle aromatique, en particulier qui comporte de 6 à 18 atomes de cycle aromatique, lequel peut, dans chaque cas, être substitué par un radical ou par plusieurs radicaux R⁵ ;
R³ est pour chaque occurrence, de manière identique ou différente, H, un groupe alkyle qui comporte de 1 à 4 atome(s) de C ou un système de cycle aromatique ou hétéroaromatique qui comporte de 5 à 14 atomes de cycle aromatique ;
R⁴ est, pour Y² ou Y³ = NR⁴, un système de cycle aromatique ou hétéroaromatique qui comporte de 5 à 24 atomes de cycle aromatique, lequel peut dans chaque cas être substitué par un radical ou par plusieurs radicaux R⁵ ; et est, pour Y² ou Y³ = C(R⁴)₂, pour chaque occurrence, de manière identique ou différente, un groupe alkyle en chaîne droite qui comporte de 1 à 10 atome(s) de C ou un groupe alkyle ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R⁵, où un ou plusieurs groupe(s) CH₂ non adjacents peut/peuvent être remplacé(s) par O et où un ou plusieurs atome(s) de H peut/ peuvent être remplacé(s) par D ou F, ou un système de cycle aromatique ou hétéroaromatique qui comporte de 5 à 24 atomes de cycle aromatique, lequel peut dans chaque cas être substitué par un radical ou par plusieurs radicaux R⁵; les deux substituants R⁴ peuvent ici en option former un système de cycle aliphatique, aromatique ou hétéroaromatique mono- ou polycyclique, lequel peut être substitué par un radical ou par plusieurs radicaux R⁵ ;
R⁵ est sélectionné pour chaque occurrence, de manière identique ou différente, parmi le groupe qui est constitué par H, D, F, CN, un radical hydrocarbone aliphatique qui comporte de 1 à 10 atome(s) de C ou un système de cycle aromatique ou hétéroaromatique qui comporte de 5 à 30 atomes de cycle aromatique, lequel peut être substitué par un ou par plusieurs groupe(s) alkyle, chacun comportant de 1 à 4 atome(s) de carbone ;
les autres symboles et les autres indices présentent les significations qui ont été données selon les revendications 1 et 2.

11. Procédé pour la préparation d'un composé selon une ou plusieurs des revendications 1 à 10, en partant d'un 1-halodibenzofurane ou d'un 1-halodibenzothiophène en option substitué, **caractérisé par** les étapes qui suivent :
a) en option, la conversion du groupe halogène selon un acide boronique ou un dérivé d'acide boronique ;
b) l'introduction du groupe HetAr au moyen d'une réaction de couplage ; et
c) l'introduction du groupe N¹ ou du groupe -L-N¹ au moyen d'une réaction de couplage.

12. Formulation comprenant au moins un composé selon une ou plusieurs des revendications 1 à 10 et au moins un autre composé et/ou un solvant.

13. Utilisation d'un composé selon une ou plusieurs des revendications 1 à 10 et/ou d'une formulation selon la revendication 12 dans un dispositif électronique.

14. Dispositif électronique, de préférence sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant(s) organiques, les détecteurs optiques organiques, les photorécepteurs organiques, les dispositifs à extinction de champ organiques, les cellules électrochimiques à émission de lumière, les diodes laser organiques et les dispositifs à émission de plasmons organiques, comprenant au moins un composé selon une ou plusieurs des revendications 1 à 10 ou une formulation selon la revendication 12.

15. Dispositif électronique selon la revendication 14, lequel est un dispositif électroluminescent organique, **caractérisé en ce que** le composé selon une ou plusieurs des revendications 1 à 10 est utilisé en tant que matériau de matrice pour des émetteurs fluorescents ou phosphorescents dans une couche d'émission et/ou dans une couche de transport d'électrons et/ou dans une couche de blocage d'électrons ou de blocage d'excitons et/ou dans une couche de transport de trous.
